# EUROPÄISCHE PATENTANMELDUNG

(11) **EP 3 366 778 A1**
(43) Veröffentlichungstag der Anmeldung: **29.08.2018**
(21) Anmeldenummer: 17158439.4
(22) Anmeldetag: 28.02.2017
(51) Int. Cl.: C12N 15/82, A01H 1/08, C12N 9/18, C07K 14/415

(54) **HAPLOIDISIERUNG IN SORGHUM**

(71) Anmelder: KWS SAAT SE, 37574 Einbeck (DE)
(72) Erfinder: KLOIBER-MAITZ, Monika, 37574 Einbeck (DE); WIECKHORST, Silke, 37574 Einbeck (DE); BOLDUAN, Christof, 37574 Einbeck (DE); OUZUNOVA, Milena, 37077 Göttingen (DE)

(57) **Zusammenfassung**

Es werden Sorghumhirsepflanzen zur Verfügung gestellt, die durch Modifikationen im Genom, die eine pollenspezifisch exprimierte Patatin-Phospholipase betreffen, in der Lage sind Haploidie zu induzieren, wodurch haploide Nachkommen erzeugt und in kurzer Zeit durch Chromosomenverdopplung Inzuchtlinien, d.h. reinerbige Vater- und Mutterlinien, für die Hybridzüchtung hergestellt werden können. Zusätzlich werden Verfahren zur Herstellung von transgenen sowie nicht-transgenen pflanzliche Haploiden-Induktoren und die Verbesserung der Induktionsleistung von Pflanzen bereitgestellt.

SEQUENCE LISTING

<110>  KWS SAAT SE

<120>  Haploidisierung in Sorghum

<130>  KWS0259EP

<160>  45

<170>  PatentIn version 3.5

<210>  1
<211>  2031
<212>  DNA
<213>  Sorghum bicolor

<400>  1
acgcagtgcc tatatacaca agccaccatc tgtcgttgcc tcctcctcct cagctcatca      60

ctagtcacac gtacacccgc aatcgattga aaatttatcg cgttcgtcca aacaatacca     120

gagctcgacg atcatcttaa ttgttggatc gatctgaata atctttcttc attcgtggcc     180

tcgacgagta agtaggtagc tcatttgatc gatcaaccgc gagtgtgtgc ttgatcgaag     240

gcggcaatgg cgacctacta ctcttcgcgg cgtccatgca acgcctgcag cacgaaggcg     300

atggccggga gcgtggtcgg cgagcccgtc gtgctggggc agagggtgac ggtgctgacg     360

gtggacggcg gcggcatccg tggtctcatc cccggaacca tccttgcctt cctcgaggcc     420

cggctgcagg agctggacgg gccggaggtt aggctcgcgg actacttcga ctacatcgcc     480

gggacgagca ccggcgggct catcaccgcc atgctcaccg cgcccggcaa ggacaggcgg     540

cctctctacg ctgccaagga catcaaccaa ttctacatgg agaattgccc tcgcatcttc     600

cctcaaaagt gagtccgatc atcccggccg gccgccattg ttggcctcgc attattgatc     660

gatcatgcat gcatgcatgc atgtgatgtg taggagcagc aggcttgcgg ccgccatgtc     720

cgcgctgagg aagccaaggt acaacggcaa gtgcctccgt aacctgatca tgagcatgct     780

cggcgagacg agggtgagcg acacgctcac caacgtcatc atccctacct tcgacgtcag     840

gctgctgcag cccatcatct tctccaccta cgacgtacgt acgccgccgg ccgccgtcgt     900

catgaataat caatcagctg tacattattg cacatggctg catgcctact gaacatgttt     960

aatttgcagg ccaagagcat gcctctgaag aacgcgctgc tctccgacgt gtgcatcggc    1020

acgtccgccg cgccgaccta cctcccggcg cactacttcc agaccaagga cgccggcagt    1080

ggcaaggaac gcgagtacaa cctcatcgac ggcggtgtcg ccgccaacaa tccggtaatt    1140

aattaactga aaacgaacga acgcaattcc atatccattg tccagaagat cgatgctaat    1200

tgttgcttag gcacgtcgtt gccgccggcc gattgcattg cacttgcaca tgcgtagacg    1260

atggttgcga tgacgcagat caccaagaag atgcttgcca gcaaggagaa ggccgaggag    1320

ctgtacccag tgaagccgtg gaactgccgc aagttcctgg tgctgtccat cgggacgggg    1380

tcgacgtcgg agcagggcct gtacacggcg cggcagtgct cgcggtgggg catctgccgg 1440

tggatccgga acaacggcat ggcccccatc atcgacatct tcatggcggc gagctcggac 1500

ctggtggaca tccacgtcgc cgcgatgttc cagtcgctcc acagcgacgg cgactacctg 1560

cgcatccagg acaactcgct gcacggcgcc gcggccaccg tggacgcggc gacgccggag 1620

aacatgcgga cgctcgtcgg gatcggggag cggatgctgg cgcagcgggt gtccagggtc 1680

aacgtggaga cagggaggta cgaaccggtg cctggggaag gaagcaacgc tgatgcgctc 1740

gctgggatcg caaggcagct ctcggaggag aggaggacaa ggctcgcgcg ccgcacctcc 1800

gccatcgtca gctccggtgg tgcctctaga cgtacgtgtg cctcaaaggt ctccaatgtc 1860

taagagaagt gaatttgttg cctgattaaa atcttaatta attaattctg tgggctgtgg 1920

ctcaaataaa actatatcat taaaaaaccc gccatgaact agttctagga aaatacgcat 1980

ggttttagaa agaaaaacat aaactaattc taacaaaatg tacacacctc t 2031

<210> 2
<211> 1314
<212> DNA
<213> Artificial Sequence

<220>
<223> cDNA of Sorghum bicolor Phospholipase

<400> 2
atggcgacct actactcttc gcggcgtcca tgcaacgcct gcagcacgaa ggcgatggcc 60

gggagcgtgg tcggcgagcc cgtcgtgctg ggcagagggt tgacggtgct gacggtggac 120

ggcggcggca tccgtggtct catccccgga accatccttg ccttcctcga ggcccggctg 180

caggagctgg acgggccgga ggttaggctc gcggactact tcgactacat cgccgggacg 240

agcaccggcg ggctcatcac cgccatgctc accgcgcccg gcaaggacag gcggcctctc 300

tacgctgcca aggacatcaa ccaattctac atggagaatt gccctcgcat cttccctcaa 360

aagagcagca ggcttgcggc cgccatgtcc gcgctgagga agccaaggta caacggcaag 420

tgcctccgta acctgatcat gagcatgctc ggcgagacga gggtgagcga cacgctcacc 480

aacgtcatca tccctacctt cgacgtcagg ctgctgcagc ccatcatctt ctccacctac 540

gacgccaaga gcatgcctct gaagaacgcg ctgctctccg acgtgtgcat cggcacgtcc 600

gccgcgccga cctacctccc ggcgcactac ttccagacca aggacgccgg cagtggcaag 660

gaacgcgagt acaacctcat cgacggcggt gtcgccgcca acaatccgac gatggttgcg 720

atgacgcaga tcaccaagaa gatgcttgcc agcaaggaga aggccgagga gctgtaccca 780

gtgaagccgt ggaactgccg caagttcctg gtgctgtcca tcgggacggg gtcgacgtcg 840

gagcagggcc tgtacacggc gcggcagtgc tcgcggtggg gcatctgccg gtggatccgg 900

aacaacggca tggcccccat catcgacatc ttcatggcgg cgagctcgga cctggtggac 960

atccacgtcg ccgcgatgtt ccagtcgctc cacagcgacg gcgactacct gcgcatccag 1020

gacaactcgc tgcacggcgc cgcggccacc gtggacgcgg cgacgccgga gaacatgcgg 1080

acgctcgtcg ggatcgggga gcggatgctg gcgcagcggg tgtccagggt caacgtggag 1140

acagggaggt acgaaccggt gcctggggaa ggaagcaacg ctgatgcgct cgctgggatc 1200

gcaaggcagc tctcggagga gaggaggaca aggctcgcgc gccgcacctc cgccatcgtc 1260

agctccggtg gtgcctctag acgtacgtgt gcctcaaagg tctccaatgt ctaa 1314

<210> 3
<211> 437
<212> PRT
<213> Sorghum bicolor

<400> 3

Met Ala Thr Tyr Tyr Ser Ser Arg Arg Pro Cys Asn Ala Cys Ser Thr
1               5                   10                  15

Lys Ala Met Ala Gly Ser Val Val Gly Glu Pro Val Val Leu Gly Gln
            20                  25                  30

Arg Val Thr Val Leu Thr Val Asp Gly Gly Gly Ile Arg Gly Leu Ile
        35                  40                  45

Pro Gly Thr Ile Leu Ala Phe Leu Glu Ala Arg Leu Gln Glu Leu Asp
    50                  55                  60

Gly Pro Glu Val Arg Leu Ala Asp Tyr Phe Asp Tyr Ile Ala Gly Thr
65                  70                  75                  80

Ser Thr Gly Gly Leu Ile Thr Ala Met Leu Thr Ala Pro Gly Lys Asp
                85                  90                  95

Arg Arg Pro Leu Tyr Ala Ala Lys Asp Ile Asn Gln Phe Tyr Met Glu
            100                 105                 110

Asn Cys Pro Arg Ile Phe Pro Gln Lys Ser Ser Arg Leu Ala Ala Ala
        115                 120                 125

Met Ser Ala Leu Arg Lys Pro Arg Tyr Asn Gly Lys Cys Leu Arg Asn
    130                 135                 140

Leu Ile Met Ser Met Leu Gly Glu Thr Arg Val Ser Asp Thr Leu Thr
145                 150                 155                 160

Asn Val Ile Ile Pro Thr Phe Asp Val Arg Leu Leu Gln Pro Ile Ile

                165                 170                 175

Phe Ser Thr Tyr Asp Ala Lys Ser Met Pro Leu Lys Asn Ala Leu Leu
            180                 185                 190

Ser Asp Val Cys Ile Gly Thr Ser Ala Ala Pro Thr Tyr Leu Pro Ala
        195                 200                 205

His Tyr Phe Gln Thr Lys Asp Ala Gly Ser Gly Lys Glu Arg Glu Tyr
    210                 215                 220

Asn Leu Ile Asp Gly Gly Val Ala Ala Asn Asn Pro Thr Met Val Ala
225                 230                 235                 240

Met Thr Gln Ile Thr Lys Lys Met Leu Ala Ser Lys Glu Lys Ala Glu
                245                 250                 255

Glu Leu Tyr Pro Val Lys Pro Trp Asn Cys Arg Lys Phe Leu Val Leu
            260                 265                 270

Ser Ile Gly Thr Gly Ser Thr Ser Glu Gln Gly Leu Tyr Thr Ala Arg
        275                 280                 285

Gln Cys Ser Arg Trp Gly Ile Cys Arg Trp Ile Arg Asn Asn Gly Met
    290                 295                 300

Ala Pro Ile Ile Asp Ile Phe Met Ala Ala Ser Ser Asp Leu Val Asp
305                 310                 315                 320

Ile His Val Ala Ala Met Phe Gln Ser Leu His Ser Asp Gly Asp Tyr
                325                 330                 335

Leu Arg Ile Gln Asp Asn Ser Leu His Gly Ala Ala Ala Thr Val Asp
            340                 345                 350

Ala Ala Thr Pro Glu Asn Met Arg Thr Leu Val Gly Ile Gly Glu Arg
        355                 360                 365

Met Leu Ala Gln Arg Val Ser Arg Val Asn Val Glu Thr Gly Arg Tyr
    370                 375                 380

Glu Pro Val Pro Gly Glu Gly Ser Asn Ala Asp Ala Leu Ala Gly Ile
385                 390                 395                 400

Ala Arg Gln Leu Ser Glu Glu Arg Arg Thr Arg Leu Ala Arg Arg Thr
                405                 410                 415

Ser Ala Ile Val Ser Ser Gly Gly Ala Ser Arg Arg Thr Cys Ala Ser
            420                 425                 430

Lys Val Ser Asn Val
        435

<210>  4
<211>  2031
<212>  DNA
<213>  Artificial Sequence

<220>
<223>  genomic DNA of mutated Sorghum bicolor phospholipase R59Q

<400>  4
acgcagtgcc tatatacaca agccaccatc tgtcgttgcc tcctcctcct cagctcatca      60

ctagtcacac gtacacccgc aatcgattga aaatttatcg cgttcgtcca aacaatacca     120

gagctcgacg atcatcttaa ttgttggatc gatctgaata atctttcttc attcgtggcc     180

tcgacgagta agtaggtagc tcatttgatc gatcaaccgc gagtgtgtgc ttgatcgaag     240

gcggcaatgg cgacctacta ctcttcgcgg cgtccatgca acgcctgcag cacgaaggcg     300

atggccggga gcgtggtcgg cgagcccgtc gtgctggggc agagggtgac ggtgctgacg     360

gtggacggcg gcggcatccg tggtctcatc cccggaacca tccttgcctt cctcgaggcc     420

cagctgcagg agctggacgg gccggaggtt aggctcgcgg actacttcga ctacatcgcc     480

gggacgagca ccggcgggct catcaccgcc atgctcaccg cgcccggcaa ggacaggcgg     540

cctctctacg ctgccaagga catcaaccaa ttctacatgg agaattgccc tcgcatcttc     600

cctcaaaagt gagtccgatc atcccggccg gccgccattg ttggcctcgc attattgatc     660

gatcatgcat gcatgcatgc atgtgatgtg taggagcagc aggcttgcgg ccgccatgtc     720

cgcgctgagg aagccaaggt acaacggcaa gtgcctccgt aacctgatca tgagcatgct     780

cggcgagacg agggtgagcg acacgctcac caacgtcatc atccctacct tcgacgtcag     840

gctgctgcag cccatcatct tctccaccta cgacgtacgt acgccgccgg ccgccgtcgt     900

catgaataat caatcagctg tacattattg cacatggctg catgcctact gaacatgttt     960

aatttgcagg ccaagagcat gcctctgaag aacgcgctgc tctccgacgt gtgcatcggc    1020

acgtccgccg cgccgaccta cctcccggcg cactacttcc agaccaagga cgccggcagt    1080

ggcaaggaac gcgagtacaa cctcatcgac ggcggtgtcg ccgccaacaa tccggtaatt    1140

aattaactga aaacgaacga acgcaattcc atatccattg tccagaagat cgatgctaat    1200

tgttgcttag gcacgtcgtt gccgccggcc gattgcattg cacttgcaca tgcgtagacg    1260

atggttgcga tgacgcagat caccaagaag atgcttgcca gcaaggagaa ggccgaggag    1320

ctgtacccag tgaagccgtg gaactgccgc aagttcctgg tgctgtccat cgggacgggg    1380

tcgacgtcgg agcagggcct gtacacggcg cggcagtgct cgcggtgggg catctgccgg 1440

tggatccgga acaacggcat ggcccccatc atcgacatct tcatggcggc gagctcggac 1500

ctggtggaca tccacgtcgc cgcgatgttc cagtcgctcc acagcgacgg cgactacctg 1560

cgcatccagg acaactcgct gcacggcgcc gcggccaccg tggacgcggc gacgccggag 1620

aacatgcgga cgctcgtcgg gatcggggag cggatgctgg cgcagcgggt gtccagggtc 1680

aacgtggaga cagggaggta cgaaccggtg cctggggaag gaagcaacgc tgatgcgctc 1740

gctgggatcg caaggcagct ctcggaggag aggaggacaa ggctcgcgcg ccgcacctcc 1800

gccatcgtca gctccggtgg tgcctctaga cgtacgtgtg cctcaaaggt ctccaatgtc 1860

taagagaagt gaatttgttg cctgattaaa atcttaatta attaattctg tgggctgtgg 1920

ctcaaataaa actatatcat taaaaaaccc gccatgaact agttctagga aaatacgcat 1980

ggttttagaa agaaaaacat aaactaattc taacaaaatg tacacacctc t 2031

<210> 5
<211> 1314
<212> DNA
<213> Artificial Sequence

<220>
<223> cDNA of Sorghum bicolor phospholipase mutant R59Q

<400> 5
atggcgacct actactcttc gcggcgtcca tgcaacgcct gcagcacgaa ggcgatggcc 60

gggagcgtgg tcggcgagcc cgtcgtgctg ggcagagggg tgacggtgct gacggtggac 120

ggcggcggca tccgtggtct catccccgga accatccttg ccttcctcga ggcccagctg 180

caggagctgg acgggccgga ggttaggctc gcggactact tcgactacat cgccgggacg 240

agcaccggcg ggctcatcac cgccatgctc accgcgcccg gcaaggacag gcggcctctc 300

tacgctgcca aggacatcaa ccaattctac atggagaatt gccctcgcat cttccctcaa 360

aagagcagca ggcttgcggc cgccatgtcc gcgctgagga agccaaggta caacggcaag 420

tgcctccgta acctgatcat gagcatgctc ggcgagacga gggtgagcga cacgctcacc 480

aacgtcatca tccctacctt cgacgtcagg ctgctgcagc ccatcatctt ctccacctac 540

gacgccaaga gcatgcctct gaagaacgcg ctgctctccg acgtgtgcat cggcacgtcc 600

gccgcgccga cctacctccc ggcgcactac ttccagacca aggacgccgg cagtggcaag 660

gaacgcgagt acaacctcat cgacggcggt gtcgccgcca acaatccgac gatggttgcg 720

atgacgcaga tcaccaagaa gatgcttgcc agcaaggaga aggccgagga gctgtaccca 780

gtgaagccgt ggaactgccg caagttcctg gtgctgtcca tcgggacggg gtcgacgtcg 840

gagcagggcc tgtacacggc gcggcagtgc tcgcggtggg gcatctgccg gtggatccgg 900

aacaacggca tggcccccat catcgacatc ttcatggcgg cgagctcgga cctggtggac 960

atccacgtcg ccgcgatgtt ccagtcgctc cacagcgacg gcgactacct gcgcatccag 1020

gacaactcgc tgcacggcgc cgcggccacc gtggacgcgg cgacgccgga gaacatgcgg 1080

acgctcgtcg ggatcgggga gcggatgctg gcgcagcggg tgtccagggt caacgtggag 1140

acagggaggt acgaaccggt gcctggggaa ggaagcaacg ctgatgcgct cgctgggatc 1200

gcaaggcagc tctcggagga gaggaggaca aggctcgcgc gccgcacctc cgccatcgtc 1260

agctccggtg gtgcctctag acgtacgtgt gcctcaaagg tctccaatgt ctaa 1314

<210> 6
<211> 437
<212> PRT
<213> Artificial Sequence

<220>
<223> amino acid sequence of mutated Sorghum bicolor phospholipase R59Q

<400> 6

Met Ala Thr Tyr Tyr Ser Ser Arg Arg Pro Cys Asn Ala Cys Ser Thr
1               5                   10                  15

Lys Ala Met Ala Gly Ser Val Val Gly Glu Pro Val Val Leu Gly Gln
            20                  25                  30

Arg Val Thr Val Leu Thr Val Asp Gly Gly Gly Ile Arg Gly Leu Ile
        35                  40                  45

Pro Gly Thr Ile Leu Ala Phe Leu Glu Ala Gln Leu Gln Glu Leu Asp
    50                  55                  60

Gly Pro Glu Val Arg Leu Ala Asp Tyr Phe Asp Tyr Ile Ala Gly Thr
65                  70                  75                  80

Ser Thr Gly Gly Leu Ile Thr Ala Met Leu Thr Ala Pro Gly Lys Asp
                85                  90                  95

Arg Arg Pro Leu Tyr Ala Ala Lys Asp Ile Asn Gln Phe Tyr Met Glu
            100                 105                 110

Asn Cys Pro Arg Ile Phe Pro Gln Lys Ser Ser Arg Leu Ala Ala Ala
        115                 120                 125

Met Ser Ala Leu Arg Lys Pro Arg Tyr Asn Gly Lys Cys Leu Arg Asn
    130                 135                 140

Leu Ile Met Ser Met Leu Gly Glu Thr Arg Val Ser Asp Thr Leu Thr
145                 150                 155                 160

Asn Val Ile Ile Pro Thr Phe Asp Val Arg Leu Leu Gln Pro Ile Ile
                165                 170                 175

Phe Ser Thr Tyr Asp Ala Lys Ser Met Pro Leu Lys Asn Ala Leu Leu
            180                 185                 190

Ser Asp Val Cys Ile Gly Thr Ser Ala Ala Pro Thr Tyr Leu Pro Ala
        195                 200                 205

His Tyr Phe Gln Thr Lys Asp Ala Gly Ser Gly Lys Glu Arg Glu Tyr
    210                 215                 220

Asn Leu Ile Asp Gly Gly Val Ala Ala Asn Asn Pro Thr Met Val Ala
225                 230                 235                 240

Met Thr Gln Ile Thr Lys Lys Met Leu Ala Ser Lys Glu Lys Ala Glu
                245                 250                 255

Glu Leu Tyr Pro Val Lys Pro Trp Asn Cys Arg Lys Phe Leu Val Leu
            260                 265                 270

Ser Ile Gly Thr Gly Ser Thr Ser Glu Gln Gly Leu Tyr Thr Ala Arg
        275                 280                 285

Gln Cys Ser Arg Trp Gly Ile Cys Arg Trp Ile Arg Asn Asn Gly Met
    290                 295                 300

Ala Pro Ile Ile Asp Ile Phe Met Ala Ala Ser Ser Asp Leu Val Asp
305                 310                 315                 320

Ile His Val Ala Ala Met Phe Gln Ser Leu His Ser Asp Gly Asp Tyr
                325                 330                 335

Leu Arg Ile Gln Asp Asn Ser Leu His Gly Ala Ala Ala Thr Val Asp
            340                 345                 350

Ala Ala Thr Pro Glu Asn Met Arg Thr Leu Val Gly Ile Gly Glu Arg
        355                 360                 365

Met Leu Ala Gln Arg Val Ser Arg Val Asn Val Glu Thr Gly Arg Tyr
    370                 375                 380

Glu Pro Val Pro Gly Glu Gly Ser Asn Ala Asp Ala Leu Ala Gly Ile
385                 390                 395                 400

Ala Arg Gln Leu Ser Glu Glu Arg Arg Thr Arg Leu Ala Arg Arg Thr
                405                 410                 415

Ser Ala Ile Val Ser Ser Gly Gly Ala Ser Arg Arg Thr Cys Ala Ser
            420                 425                 430

Lys Val Ser Asn Val
        435

<210>  7
<211>  2031
<212>  DNA
<213>  Artificial Sequence

<220>
<223>  genomic DNA of mutated Sorghum bicolor phospholipase V162I

<400>  7
acgcagtgcc tatatacaca agccaccatc tgtcgttgcc tcctcctcct cagctcatca      60

ctagtcacac gtacacccgc aatcgattga aaatttatcg cgttcgtcca aacaatacca     120

gagctcgacg atcatcttaa ttgttggatc gatctgaata atctttcttc attcgtggcc     180

tcgacgagta agtaggtagc tcatttgatc gatcaaccgc gagtgtgtgc ttgatcgaag     240

gcggcaatgg cgacctacta ctcttcgcgg cgtccatgca acgcctgcag cacgaaggcg     300

atggccggga gcgtggtcgg cgagcccgtc gtgctggggc agagggtgac ggtgctgacg     360

gtggacggcg gcggcatccg tggtctcatc cccggaacca tccttgcctt cctcgaggcc     420

cggctgcagg agctggacgg gccggaggtt aggctcgcgg actacttcga ctacatcgcc     480

gggacgagca ccggcgggct catcaccgcc atgctcaccg cgcccggcaa ggacaggcgg     540

cctctctacg ctgccaagga catcaaccaa ttctacatgg agaattgccc tcgcatcttc     600

cctcaaaagt gagtccgatc atcccggccg gccgccattg ttggcctcgc attattgatc     660

gatcatgcat gcatgcatgc atgtgatgtg taggagcagc aggcttgcgg ccgccatgtc     720

cgcgctgagg aagccaaggt acaacggcaa gtgcctccgt aacctgatca tgagcatgct     780

cggcgagacg agggtgagcg acacgctcac caacatcatc atccctacct tcgacgtcag     840

gctgctgcag cccatcatct tctccaccta cgacgtacgt acgccgccgg ccgccgtcgt     900

catgaataat caatcagctg tacattattg cacatggctg catgcctact gaacatgttt     960

aatttgcagg ccaagagcat gcctctgaag aacgcgctgc tctccgacgt gtgcatcggc    1020

acgtccgccg cgccgaccta cctcccggcg cactacttcc agaccaagga cgccggcagt    1080

ggcaaggaac gcgagtacaa cctcatcgac ggcggtgtcg ccgccaacaa tccggtaatt    1140

aattaactga aaacgaacga acgcaattcc atatccattg tccagaagat cgatgctaat    1200

tgttgcttag gcacgtcgtt gccgccggcc gattgcattg cacttgcaca tgcgtagacg    1260

atggttgcga tgacgcagat caccaagaag atgcttgcca gcaaggagaa ggccgaggag    1320

ctgtacccag tgaagccgtg gaactgccgc aagttcctgg tgctgtccat cgggacgggg 1380

tcgacgtcgg agcagggcct gtacacggcg cggcagtgct cgcggtgggg catctgccgg 1440

tggatccgga acaacggcat ggcccccatc atcgacatct tcatggcggc gagctcggac 1500

ctggtggaca tccacgtcgc cgcgatgttc cagtcgctcc acagcgacgg cgactacctg 1560

cgcatccagg acaactcgct gcacggcgcc gcggccaccg tggacgcggc gacgccggag 1620

aacatgcgga cgctcgtcgg gatcggggag cggatgctgg cgcagcgggt gtccagggtc 1680

aacgtggaga cagggaggta cgaaccggtg cctggggaag gaagcaacgc tgatgcgctc 1740

gctgggatcg caaggcagct ctcggaggag aggaggacaa ggctcgcgcg ccgcacctcc 1800

gccatcgtca gctccggtgg tgcctctaga cgtacgtgtg cctcaaaggt ctccaatgtc 1860

taagagaagt gaatttgttg cctgattaaa atcttaatta attaattctg tgggctgtgg 1920

ctcaaataaa actatatcat taaaaaaccc gccatgaact agttctagga aaatacgcat 1980

ggttttagaa agaaaaacat aaactaattc taacaaaatg tacacacctc t 2031

<210> 8
<211> 1314
<212> DNA
<213> Artificial Sequence

<220>
<223> cDNA Sorghum bicolor Phospholipase mutant V162I

<400> 8
atggcgacct actactcttc gcggcgtcca tgcaacgcct gcagcacgaa ggcgatggcc 60

gggagcgtgg tcggcgagcc cgtcgtgctg ggcagagggg tgacggtgct gacggtggac 120

ggcggcggca tccgtggtct catccccgga accatccttg ccttcctcga ggcccggctg 180

caggagctgg acgggccgga ggttaggctc gcggactact tcgactacat cgccgggacg 240

agcaccggcg ggctcatcac cgccatgctc accgcgcccg gcaaggacag gcggcctctc 300

tacgctgcca aggacatcaa ccaattctac atggagaatt gccctcgcat cttccctcaa 360

aagagcagca ggcttgcggc cgccatgtcc gcgctgagga agccaaggta caacggcaag 420

tgcctccgta acctgatcat gagcatgctc ggcgagacga gggtgagcga cacgctcacc 480

aacatcatca tccctacctt cgacgtcagg ctgctgcagc ccatcatctt ctccacctac 540

gacgccaaga gcatgcctct gaagaacgcg ctgctctccg acgtgtgcat cggcacgtcc 600

gccgcgccga cctacctccc ggcgcactac ttccagacca aggacgccgg cagtggcaag 660

gaacgcgagt acaacctcat cgacggcggt gtcgccgcca acaatccgac gatggttgcg 720

atgacgcaga tcaccaagaa gatgcttgcc agcaaggaga aggccgagga gctgtaccca 780

gtgaagccgt ggaactgccg caagttcctg gtgctgtcca tcgggacggg gtcgacgtcg 840

gagcagggcc tgtacacggc gcggcagtgc tcgcggtggg gcatctgccg gtggatccgg 900

aacaacggca tggcccccat catcgacatc ttcatggcgg cgagctcgga cctggtggac 960

atccacgtcg ccgcgatgtt ccagtcgctc cacagcgacg gcgactacct gcgcatccag 1020

gacaactcgc tgcacggcgc cgcggccacc gtggacgcgg cgacgccgga gaacatgcgg 1080

acgctcgtcg ggatcgggga gcggatgctg gcgcagcggg tgtccagggt caacgtggag 1140

acagggaggt acgaaccggt gcctggggaa ggaagcaacg ctgatgcgct cgctgggatc 1200

gcaaggcagc tctcggagga gaggaggaca aggctcgcgc gccgcacctc cgccatcgtc 1260

agctccggtg gtgcctctag acgtacgtgt gcctcaaagg tctccaatgt ctaa 1314

<210> 9
<211> 437
<212> PRT
<213> Artificial Sequence

<220>
<223> amino acid sequence of mutated Sorghum bicolor phospholipase
 V162I

<400> 9

Met Ala Thr Tyr Tyr Ser Ser Arg Arg Pro Cys Asn Ala Cys Ser Thr
1               5                   10                  15

Lys Ala Met Ala Gly Ser Val Val Gly Glu Pro Val Val Leu Gly Gln
            20                  25                  30

Arg Val Thr Val Leu Thr Val Asp Gly Gly Gly Ile Arg Gly Leu Ile
        35                  40                  45

Pro Gly Thr Ile Leu Ala Phe Leu Glu Ala Arg Leu Gln Glu Leu Asp
    50                  55                  60

Gly Pro Glu Val Arg Leu Ala Asp Tyr Phe Asp Tyr Ile Ala Gly Thr
65                  70                  75                  80

Ser Thr Gly Gly Leu Ile Thr Ala Met Leu Thr Ala Pro Gly Lys Asp
                85                  90                  95

Arg Arg Pro Leu Tyr Ala Ala Lys Asp Ile Asn Gln Phe Tyr Met Glu
            100                 105                 110

Asn Cys Pro Arg Ile Phe Pro Gln Lys Ser Ser Arg Leu Ala Ala Ala
        115                 120                 125

Met Ser Ala Leu Arg Lys Pro Arg Tyr Asn Gly Lys Cys Leu Arg Asn
    130                 135                 140

Leu Ile Met Ser Met Leu Gly Glu Thr Arg Val Ser Asp Thr Leu Thr
145                 150                 155                 160

Asn Ile Ile Ile Pro Thr Phe Asp Val Arg Leu Leu Gln Pro Ile Ile
                165                 170                 175

Phe Ser Thr Tyr Asp Ala Lys Ser Met Pro Leu Lys Asn Ala Leu Leu
            180                 185                 190

Ser Asp Val Cys Ile Gly Thr Ser Ala Ala Pro Thr Tyr Leu Pro Ala
        195                 200                 205

His Tyr Phe Gln Thr Lys Asp Ala Gly Ser Gly Lys Glu Arg Glu Tyr
    210                 215                 220

Asn Leu Ile Asp Gly Gly Val Ala Ala Asn Asn Pro Thr Met Val Ala
225                 230                 235                 240

Met Thr Gln Ile Thr Lys Lys Met Leu Ala Ser Lys Glu Lys Ala Glu
                245                 250                 255

Glu Leu Tyr Pro Val Lys Pro Trp Asn Cys Arg Lys Phe Leu Val Leu
            260                 265                 270

Ser Ile Gly Thr Gly Ser Thr Ser Glu Gln Gly Leu Tyr Thr Ala Arg
        275                 280                 285

Gln Cys Ser Arg Trp Gly Ile Cys Arg Trp Ile Arg Asn Asn Gly Met
    290                 295                 300

Ala Pro Ile Ile Asp Ile Phe Met Ala Ala Ser Ser Asp Leu Val Asp
305                 310                 315                 320

Ile His Val Ala Ala Met Phe Gln Ser Leu His Ser Asp Gly Asp Tyr
                325                 330                 335

Leu Arg Ile Gln Asp Asn Ser Leu His Gly Ala Ala Ala Thr Val Asp
            340                 345                 350

Ala Ala Thr Pro Glu Asn Met Arg Thr Leu Val Gly Ile Gly Glu Arg
        355                 360                 365

Met Leu Ala Gln Arg Val Ser Arg Val Asn Val Glu Thr Gly Arg Tyr
    370                 375                 380

Glu Pro Val Pro Gly Glu Gly Ser Asn Ala Asp Ala Leu Ala Gly Ile
385                 390                 395                 400

Ala Arg Gln Leu Ser Glu Glu Arg Arg Thr Arg Leu Ala Arg Arg Thr
                405                 410                 415

Ser Ala Ile Val Ser Ser Gly Gly Ala Ser Arg Arg Thr Cys Ala Ser
            420                 425                 430

Lys Val Ser Asn Val
        435

<210>  10
<211>  2031
<212>  DNA
<213>  Artificial Sequence

<220>
<223>  genomic DNA of mutated Sorghum bicolor phospholipase S291L

<400>  10
acgcagtgcc tatatacaca agccaccatc tgtcgttgcc tcctcctcct cagctcatca      60

ctagtcacac gtacacccgc aatcgattga aaatttatcg cgttcgtcca aacaatacca     120

gagctcgacg atcatcttaa ttgttggatc gatctgaata atctttcttc attcgtggcc     180

tcgacgagta agtaggtagc tcatttgatc gatcaaccgc gagtgtgtgc ttgatcgaag     240

gcggcaatgg cgacctacta ctcttcgcgg cgtccatgca acgcctgcag cacgaaggcg     300

atggccggga gcgtggtcgg cgagcccgtc gtgctggggc agagggtgac ggtgctgacg     360

gtggacggcg gcggcatccg tggtctcatc cccggaacca tccttgcctt cctcgaggcc     420

cggctgcagg agctggacgg gccggaggtt aggctcgcgg actacttcga ctacatcgcc     480

gggacgagca ccggcgggct catcaccgcc atgctcaccg cgcccggcaa ggacaggcgg     540

cctctctacg ctgccaagga catcaaccaa ttctacatgg agaattgccc tcgcatcttc     600

cctcaaaagt gagtccgatc atcccggccg gccgccattg ttggcctcgc attattgatc     660

gatcatgcat gcatgcatgc atgtgatgtg taggagcagc aggcttgcgg ccgccatgtc     720

cgcgctgagg aagccaaggt acaacggcaa gtgcctccgt aacctgatca tgagcatgct     780

cggcgagacg agggtgagcg acacgctcac caacgtcatc atccctacct tcgacgtcag     840

gctgctgcag cccatcatct tctccaccta cgacgtacgt acgccgccgg ccgccgtcgt     900

catgaataat caatcagctg tacattattg cacatggctg catgcctact gaacatgttt     960

aatttgcagg ccaagagcat gcctctgaag aacgcgctgc tctccgacgt gtgcatcggc    1020

acgtccgccg cgccgaccta cctcccggcg cactacttcc agaccaagga cgccggcagt    1080

ggcaaggaac gcgagtacaa cctcatcgac ggcggtgtcg ccgccaacaa tccggtaatt    1140

aattaactga aaacgaacga acgcaattcc atatccattg tccagaagat cgatgctaat    1200

tgttgcttag gcacgtcgtt gccgccggcc gattgcattg cacttgcaca tgcgtagacg    1260

atggttgcga tgacgcagat caccaagaag atgcttgcca gcaaggagaa ggccgaggag 1320

ctgtacccag tgaagccgtg gaactgccgc aagttcctgg tgctgtccat cgggacgggg 1380

tcgacgtcgg agcagggcct gtacacggcg cggcagtgct tgcggtgggg catctgccgg 1440

tggatccgga acaacggcat ggcccccatc atcgacatct tcatggcggc gagctcggac 1500

ctggtggaca tccacgtcgc cgcgatgttc cagtcgctcc acagcgacgg cgactacctg 1560

cgcatccagg acaactcgct gcacggcgcc gcggccaccg tggacgcggc gacgccggag 1620

aacatgcgga cgctcgtcgg gatcggggag cggatgctgg cgcagcgggt gtccagggtc 1680

aacgtggaga cagggaggta cgaaccggtg cctggggaag gaagcaacgc tgatgcgctc 1740

gctgggatcg caaggcagct ctcggaggag aggaggacaa ggctcgcgcg ccgcacctcc 1800

gccatcgtca gctccggtgg tgcctctaga cgtacgtgtg cctcaaaggt ctccaatgtc 1860

taagagaagt gaatttgttg cctgattaaa atcttaatta attaattctg tgggctgtgg 1920

ctcaaataaa actatatcat taaaaaaccc gccatgaact agttctagga aaatacgcat 1980

ggttttagaa agaaaaacat aaactaattc taacaaaatg tacacacctc t 2031

<210> 11
<211> 1314
<212> DNA
<213> Artificial Sequence

<220>
<223> cDNA of Sorghum bicolor phospholipase mutant S291L

<400> 11
atggcgacct actactcttc gcggcgtcca tgcaacgcct gcagcacgaa ggcgatggcc 60

gggagcgtgg tcggcgagcc cgtcgtgctg gggcagaggg tgacggtgct gacggtggac 120

ggcggcggca tccgtggtct catccccgga accatccttg ccttcctcga ggcccggctg 180

caggagctgg acgggccgga ggttaggctc gcggactact tcgactacat cgccgggacg 240

agcaccggcg ggctcatcac cgccatgctc accgcgcccg gcaaggacag gcggcctctc 300

tacgctgcca aggacatcaa ccaattctac atggagaatt gccctcgcat cttccctcaa 360

aagagcagca ggcttgcggc cgccatgtcc gcgctgagga agccaaggta caacggcaag 420

tgcctccgta acctgatcat gagcatgctc ggcgagacga gggtgagcga cacgctcacc 480

aacgtcatca tccctacctt cgacgtcagg ctgctgcagc ccatcatctt ctccacctac 540

gacgccaaga gcatgcctct gaagaacgcg ctgctctccg acgtgtgcat cggcacgtcc 600

gccgcgccga cctacctccc ggcgcactac ttccagacca aggacgccgg cagtggcaag 660

gaacgcgagt acaacctcat cgacggcggt gtcgccgcca acaatccgac gatggttgcg 720

atgacgcaga tcaccaagaa gatgcttgcc agcaaggaga aggccgagga gctgtaccca 780

gtgaagccgt ggaactgccg caagttcctg gtgctgtcca tcgggacggg gtcgacgtcg 840

gagcagggcc tgtacacggc gcggcagtgc ttgcggtggg gcatctgccg gtggatccgg 900

aacaacggca tggcccccat catcgacatc ttcatggcgg cgagctcgga cctggtggac 960

atccacgtcg ccgcgatgtt ccagtcgctc cacagcgacg gcgactacct gcgcatccag 1020

gacaactcgc tgcacggcgc cgcggccacc gtggacgcgg cgacgccgga gaacatgcgg 1080

acgctcgtcg ggatcgggga gcggatgctg gcgcagcggg tgtccagggt caacgtggag 1140

acagggaggt acgaaccggt gcctggggaa ggaagcaacg ctgatgcgct cgctgggatc 1200

gcaaggcagc tctcggagga gaggaggaca aggctcgcgc gccgcacctc cgccatcgtc 1260

agctccggtg gtgcctctag acgtacgtgt gcctcaaagg tctccaatgt ctaa 1314

<210> 12
<211> 437
<212> PRT
<213> Artificial Sequence

<220>
<223> amino acid sequence of mutated Sorghum bicolor phospholipase
 S291L

<400> 12

Met Ala Thr Tyr Tyr Ser Ser Arg Arg Pro Cys Asn Ala Cys Ser Thr
1               5                   10                  15

Lys Ala Met Ala Gly Ser Val Val Gly Glu Pro Val Val Leu Gly Gln
            20                  25                  30

Arg Val Thr Val Leu Thr Val Asp Gly Gly Gly Ile Arg Gly Leu Ile
        35                  40                  45

Pro Gly Thr Ile Leu Ala Phe Leu Glu Ala Arg Leu Gln Glu Leu Asp
    50                  55                  60

Gly Pro Glu Val Arg Leu Ala Asp Tyr Phe Asp Tyr Ile Ala Gly Thr
65                  70                  75                  80

Ser Thr Gly Gly Leu Ile Thr Ala Met Leu Thr Ala Pro Gly Lys Asp
                85                  90                  95

Arg Arg Pro Leu Tyr Ala Ala Lys Asp Ile Asn Gln Phe Tyr Met Glu
            100                 105                 110

Asn Cys Pro Arg Ile Phe Pro Gln Lys Ser Ser Arg Leu Ala Ala Ala
        115                 120                 125

Met Ser Ala Leu Arg Lys Pro Arg Tyr Asn Gly Lys Cys Leu Arg Asn

        130                 135                 140

Leu Ile Met Ser Met Leu Gly Glu Thr Arg Val Ser Asp Thr Leu Thr
145                 150                 155                 160

Asn Val Ile Ile Pro Thr Phe Asp Val Arg Leu Leu Gln Pro Ile Ile
                165                 170                 175

Phe Ser Thr Tyr Asp Ala Lys Ser Met Pro Leu Lys Asn Ala Leu Leu
            180                 185                 190

Ser Asp Val Cys Ile Gly Thr Ser Ala Ala Pro Thr Tyr Leu Pro Ala
        195                 200                 205

His Tyr Phe Gln Thr Lys Asp Ala Gly Ser Gly Lys Glu Arg Glu Tyr
    210                 215                 220

Asn Leu Ile Asp Gly Gly Val Ala Ala Asn Asn Pro Thr Met Val Ala
225                 230                 235                 240

Met Thr Gln Ile Thr Lys Lys Met Leu Ala Ser Lys Glu Lys Ala Glu
                245                 250                 255

Glu Leu Tyr Pro Val Lys Pro Trp Asn Cys Arg Lys Phe Leu Val Leu
            260                 265                 270

Ser Ile Gly Thr Gly Ser Thr Ser Glu Gln Gly Leu Tyr Thr Ala Arg
        275                 280                 285

Gln Cys Leu Arg Trp Gly Ile Cys Arg Trp Ile Arg Asn Asn Gly Met
    290                 295                 300

Ala Pro Ile Ile Asp Ile Phe Met Ala Ala Ser Ser Asp Leu Val Asp
305                 310                 315                 320

Ile His Val Ala Ala Met Phe Gln Ser Leu His Ser Asp Gly Asp Tyr
                325                 330                 335

Leu Arg Ile Gln Asp Asn Ser Leu His Gly Ala Ala Ala Thr Val Asp
            340                 345                 350

Ala Ala Thr Pro Glu Asn Met Arg Thr Leu Val Gly Ile Gly Glu Arg
        355                 360                 365

Met Leu Ala Gln Arg Val Ser Arg Val Asn Val Glu Thr Gly Arg Tyr
    370                 375                 380

Glu Pro Val Pro Gly Glu Gly Ser Asn Ala Asp Ala Leu Ala Gly Ile
385                 390                 395                 400

Ala Arg Gln Leu Ser Glu Glu Arg Arg Thr Arg Leu Ala Arg Arg Thr
                405                 410                 415

Ser Ala Ile Val Ser Ser Gly Gly Ala Ser Arg Arg Thr Cys Ala Ser
            420                 425                 430

Lys Val Ser Asn Val
        435

<210>  13
<211>  2031
<212>  DNA
<213>  Artificial Sequence

<220>
<223>  genomic DNA of mutated Sorghum bicolor phospholipase Q372stop

<400>  13
acgcagtgcc tatatacaca agccaccatc tgtcgttgcc tcctcctcct cagctcatca      60

ctagtcacac gtacacccgc aatcgattga aaatttatcg cgttcgtcca aacaatacca     120

gagctcgacg atcatcttaa ttgttggatc gatctgaata atctttcttc attcgtggcc     180

tcgacgagta agtaggtagc tcatttgatc gatcaaccgc gagtgtgtgc ttgatcgaag     240

gcggcaatgg cgacctacta ctcttcgcgg cgtccatgca acgcctgcag cacgaaggcg     300

atggccggga gcgtggtcgg cgagcccgtc gtgctggggc agagggtgac ggtgctgacg     360

gtggacggcg gcggcatccg tggtctcatc cccggaacca tccttgcctt cctcgaggcc     420

cggctgcagg agctggacgg gccggaggtt aggctcgcgg actacttcga ctacatcgcc     480

gggacgagca ccggcgggct catcaccgcc atgctcaccg cgcccggcaa ggacaggcgg     540

cctctctacg ctgccaagga catcaaccaa ttctacatgg agaattgccc tcgcatcttc     600

cctcaaaagt gagtccgatc atcccggccg gccgccattg ttggcctcgc attattgatc     660

gatcatgcat gcatgcatgc atgtgatgtg taggagcagc aggcttgcgg ccgccatgtc     720

cgcgctgagg aagccaaggt acaacggcaa gtgcctccgt aacctgatca tgagcatgct     780

cggcgagacg agggtgagcg acacgctcac caacgtcatc atccctacct tcgacgtcag     840

gctgctgcag cccatcatct tctccaccta cgacgtacgt acgccgccgg ccgccgtcgt     900

catgaataat caatcagctg tacattattg cacatggctg catgcctact gaacatgttt     960

aatttgcagg ccaagagcat gcctctgaag aacgcgctgc tctccgacgt gtgcatcggc    1020

acgtccgccg cgccgaccta cctcccggcg cactacttcc agaccaagga cgccggcagt    1080

ggcaaggaac gcgagtacaa cctcatcgac ggcggtgtcg ccgccaacaa tccggtaatt    1140

aattaactga aaacgaacga acgcaattcc atatccattg tccagaagat cgatgctaat 1200

tgttgcttag gcacgtcgtt gccgccggcc gattgcattg cacttgcaca tgcgtagacg 1260

atggttgcga tgacgcagat caccaagaag atgcttgcca gcaaggagaa ggccgaggag 1320

ctgtacccag tgaagccgtg gaactgccgc aagttcctgg tgctgtccat cgggacgggg 1380

tcgacgtcgg agcagggcct gtacacggcg cggcagtgct cgcggtgggg catctgccgg 1440

tggatccgga acaacggcat ggcccccatc atcgacatct tcatggcggc gagctcggac 1500

ctggtggaca tccacgtcgc cgcgatgttc cagtcgctcc acagcgacgg cgactacctg 1560

cgcatccagg acaactcgct gcacggcgcc gcggccaccg tggacgcggc gacgccggag 1620

aacatgcgga cgctcgtcgg gatcggggag cggatgctgg cgtagcgggt gtccagggtc 1680

aacgtggaga cagggaggta cgaaccggtg cctggggaag gaagcaacgc tgatgcgctc 1740

gctgggatcg caaggcagct ctcggaggag aggaggacaa ggctcgcgcg ccgcacctcc 1800

gccatcgtca gctccggtgg tgcctctaga cgtacgtgtg cctcaaaggt ctccaatgtc 1860

taagagaagt gaatttgttg cctgattaaa atcttaatta attaattctg tgggctgtgg 1920

ctcaaataaa actatatcat taaaaaaccc gccatgaact agttctagga aaatacgcat 1980

ggttttagaa agaaaaacat aaactaattc taacaaaatg tacacacctc t 2031

<210> 14
<211> 1116
<212> DNA
<213> Artificial Sequence

<220>
<223> cDNA of Sorghum bicolor phospholipase mutant Q372stop

<400> 14
atggcgacct actactcttc gcggcgtcca tgcaacgcct gcagcacgaa ggcgatggcc 60

gggagcgtgg tcggcgagcc cgtcgtgctg gggcagaggg tgacggtgct gacggtggac 120

ggcggcggca tccgtggtct catccccgga accatccttg ccttcctcga ggcccggctg 180

caggagctgg acgggccgga ggttaggctc gcggactact tcgactacat cgccgggacg 240

agcaccggcg ggctcatcac cgccatgctc accgcgcccg gcaaggacag gcggcctctc 300

tacgctgcca aggacatcaa ccaattctac atggagaatt gccctcgcat cttccctcaa 360

aagagcagca ggcttgcggc cgccatgtcc gcgctgagga agccaaggta caacggcaag 420

tgcctccgta acctgatcat gagcatgctc ggcgagacga gggtgagcga cacgctcacc 480

aacgtcatca tccctacctt cgacgtcagg ctgctgcagc ccatcatctt ctccacctac 540

gacgccaaga gcatgcctct gaagaacgcg ctgctctccg acgtgtgcat cggcacgtcc 600

gccgcgccga cctacctccc ggcgcactac ttccagacca aggacgccgg cagtggcaag 660

gaacgcgagt acaacctcat cgacggcggt gtcgccgcca acaatccgac gatggttgcg 720

atgacgcaga tcaccaagaa gatgcttgcc agcaaggaga aggccgagga gctgtaccca     780

gtgaagccgt ggaactgccg caagttcctg gtgctgtcca tcgggacggg gtcgacgtcg     840

gagcagggcc tgtacacggc gcggcagtgc tcgcggtggg gcatctgccg gtggatccgg     900

aacaacggca tggcccccat catcgacatc ttcatggcgg cgagctcgga cctggtggac     960

atccacgtcg ccgcgatgtt ccagtcgctc cacagcgacg gcgactacct gcgcatccag    1020

gacaactcgc tgcacggcgc cgcggccacc gtggacgcgg cgacgccgga gaacatgcgg    1080

acgctcgtcg ggatcgggga gcggatgctg gcgtag                              1116

<210>  15
<211>  371
<212>  PRT
<213>  Artificial Sequence

<220>
<223>  amino acid sequence of mutated Sorghum bicolor phospholipase
       Q372stop

<400>  15

Met Ala Thr Tyr Tyr Ser Ser Arg Arg Pro Cys Asn Ala Cys Ser Thr
1               5                   10                  15

Lys Ala Met Ala Gly Ser Val Val Gly Glu Pro Val Val Leu Gly Gln
            20                  25                  30

Arg Val Thr Val Leu Thr Val Asp Gly Gly Gly Ile Arg Gly Leu Ile
        35                  40                  45

Pro Gly Thr Ile Leu Ala Phe Leu Glu Ala Arg Leu Gln Glu Leu Asp
    50                  55                  60

Gly Pro Glu Val Arg Leu Ala Asp Tyr Phe Asp Tyr Ile Ala Gly Thr
65                  70                  75                  80

Ser Thr Gly Gly Leu Ile Thr Ala Met Leu Thr Ala Pro Gly Lys Asp
                85                  90                  95

Arg Arg Pro Leu Tyr Ala Ala Lys Asp Ile Asn Gln Phe Tyr Met Glu
            100                 105                 110

Asn Cys Pro Arg Ile Phe Pro Gln Lys Ser Ser Arg Leu Ala Ala Ala
        115                 120                 125

Met Ser Ala Leu Arg Lys Pro Arg Tyr Asn Gly Lys Cys Leu Arg Asn
    130                 135                 140

Leu Ile Met Ser Met Leu Gly Glu Thr Arg Val Ser Asp Thr Leu Thr
145                 150                 155                 160

Asn Val Ile Ile Pro Thr Phe Asp Val Arg Leu Leu Gln Pro Ile Ile
                165                 170                 175

Phe Ser Thr Tyr Asp Ala Lys Ser Met Pro Leu Lys Asn Ala Leu Leu
            180                 185                 190

Ser Asp Val Cys Ile Gly Thr Ser Ala Ala Pro Thr Tyr Leu Pro Ala
        195                 200                 205

His Tyr Phe Gln Thr Lys Asp Ala Gly Ser Gly Lys Glu Arg Glu Tyr
    210                 215                 220

Asn Leu Ile Asp Gly Gly Val Ala Ala Asn Asn Pro Thr Met Val Ala
225                 230                 235                 240

Met Thr Gln Ile Thr Lys Lys Met Leu Ala Ser Lys Glu Lys Ala Glu
                245                 250                 255

Glu Leu Tyr Pro Val Lys Pro Trp Asn Cys Arg Lys Phe Leu Val Leu
            260                 265                 270

Ser Ile Gly Thr Gly Ser Thr Ser Glu Gln Gly Leu Tyr Thr Ala Arg
        275                 280                 285

Gln Cys Ser Arg Trp Gly Ile Cys Arg Trp Ile Arg Asn Asn Gly Met
    290                 295                 300

Ala Pro Ile Ile Asp Ile Phe Met Ala Ala Ser Ser Asp Leu Val Asp
305                 310                 315                 320

Ile His Val Ala Ala Met Phe Gln Ser Leu His Ser Asp Gly Asp Tyr
                325                 330                 335

Leu Arg Ile Gln Asp Asn Ser Leu His Gly Ala Ala Ala Thr Val Asp
            340                 345                 350

Ala Ala Thr Pro Glu Asn Met Arg Thr Leu Val Gly Ile Gly Glu Arg
        355                 360                 365

Met Leu Ala
    370

<210>  16
<211>  2979
<212>  DNA

<213>  Helianthus annuus

<400>  16
atgaataaca tcaatcttgt aatagtttcg cttgtaatcg cgattgtagc catccaaccc      60

cttgcgcaag agcaaaccga tgtaggtgag gcaaatttcg tcactgttct tagcatcgat     120

ggtgggggtg ttcgtggcat tgttcccgcc accttgcttg cttttcttga atccaaaatt     180

caggtactcg aacttaaaat gcacatgtgc atcatattac aagctgtaac ttattattga     240

aatgtgccgt ctcttcggat aggaaataga tgggccagat gcacgaattg cggattattt     300

tgatgtaata gccggaacaa gcacaggagg gctgatgaca actatgcttg cagctcctaa     360

tgagaaaaat cgtcccatgt tcgccgcaaa agacattacc aacttctact ttcaacattc     420

gcctaggatc ttccctaaaa tagggtaaac tctaactagt ttccggatct ataagatcat     480

cattaaatac aagtttcatt ttctttttcg aatcaaatac agacacacat ttgatgaggc     540

gcaaccttat ccttctcaaa acgaagcctg cgaaatgggg tattctccta caaagacttt     600

tgtaattcat gttctagtgg gtgtttggat gtgcgtttta aaactgatta ttatttacat     660

gtagtttctg aagaaaataa aacagttatt caaacacttt ttgttaataa ttctactaga     720

aaaaaaaaat ccttgtcagg aaattaatta aaaaaaagtt accatctatt aaagttcttt     780

cttactaatc aaaagttttt aaattttatt atcatgttat tataactaaa catacacatc     840

caaacactat ctcataccac atgattacac aagtctatta tttgaatatg ctaacttagt     900

attttcatat aataagtttt taaaacgcca catccaaaac ccttgattct tattttacat     960

tgtgtagcta aaacagtgtt tatacataaa aacaatcagt tatataaatc aaagcattat    1020

ttaactaaag taagctcggt tcaaactcga taagagaatt aatatatacg agtcgagttc    1080

ctgttgacca gatttcgcta gtgttaagtt tcgagttcaa aattgtatat gaacttgaac    1140

ctgtgtatca tcatacttga catttaaacc ataatgttgt tgaataaata aagtgatttt    1200

attttgtagt cggaccaaat tcatgaattc ggtagtaacc gtacttggtg aggccaccgg    1260

accaaagtat gatggtaaat atcttcgagc catggcaaag atgatgttaa aaaacctcac    1320

tattaaagat acgttgacga atgttgtcat acctgctttc gacattaggc ggcttcaacc    1380

tgttatcttc tcctctgctc aagtaattaa actcgttttt tatatttata gcagttctct    1440

atttaaaatt gattgtgtat cataaaatgg tttctgtttg atacgtttag ggaaaagagg    1500

tcgcgtggaa aaatgctttg ctagcagacg tatgcattag taccgcggcg gcaccaacat    1560

ttttcccgcc atactatttt gagactagag acgtcgatgg aaccaagcac acttttgatc     1620

taatcgatgg cggggtagct gcaaacaatc cggtagttac atttcaacaa tattgagttt    1680

gcattttatt tttaggacaa gtagtcacat tagggtgaag ggtgtgttca agctcatccc    1740

gaaggtggga gcggtgttcc cactcgtacc tcatggcgca ttttcttctt tgttgcagct    1800

ccaattttaa aaagccaccc cgccttttcc attccatagc gccacgtcaa ctgggaaatg 1860

gtgttcccac tggtattgga gatttggagg cgctacgcca cctctgtcat cccgaagcca 1920

caccctccac ccttaggagt gttatcggtt cagttttcgg tttatacagt ttaaaggttt 1980

ttttttggtt gaaaccaaaa accgaactga actgaacgga attcgggtag ttcacaactg 2040

aaccaaaaac tgaatccata ttcggttttc tgtttgaccg aataatcatt atttgttatt 2100

tgattgtgcg aggttaaata agattgagca aaaattgtaa ttaattggat ttggctaaat 2160

gacaatttaa ataaccgtta tttgttatcc gattcaaacc gaacacccaa ctcgatttgg 2220

tttaataccg aaccgacaac cggatacgta attcagttcg ctattaaaag gttcgggttt 2280

ggtagggttt aacccatttg aaatcgaatc ttcagagaaa gggttcgcta atgtcaccca 2340

cataaccaaa gaaatcttgt ttaaatgtta atggcagaca catttggcta tcacacatat 2400

aaccaaagaa gcggtgatgg ggaaatacag gttctctggc ccggaggttt tcgacggaag 2460

acggatgctt gtgctttcac tcggcactgg tacgcagacg tacaatgact tatatactgc 2520

acaaaaggct gcaaaatggg ggttgcttag ttggatcttt accaatggta ctgcgccaat 2580

cctccgcatt tttggtgatg ccatgtcaga tatggtcgac atccatgtgt caactatatt 2640

ccaatcgttg caagtcgaaa aaaactatct gcgtattcag gtataactaa gaacatataa 2700

atataatgtt gtataggtta catgtttagt aacaaggagt ttttttatgg gcaggaagat 2760

aacttgaaag gggaagcaac tgcaatggat atttcatcac ctgagaacat gagggcgcta 2820

gaggacattg gcaagaaatt gttgaagaaa ccgttgtcga gattggatgt ggagacaggc 2880

aagcttgaac cagttaaagg agaaggtacg aatgctgatg cattagcacg tttcgccact 2940

ttgctttgtg ccgaacgaaa gcgccgcaat ccagcttaa 2979

<210> 17
<211> 1305
<212> DNA
<213> Artificial Sequence

<220>
<223> cDNA of Helianthus annuus phospholipase

<400> 17
atgaataaca tcaatcttgt aatagtttcg cttgtaatcg cgattgtagc catccaaccc 60

cttgcgcaag agcaaaccga tgtaggtgag gcaaatttcg tcactgttct tagcatcgat 120

ggtgggggtg ttcgtggcat tgttcccgcc accttgcttg cttttcttga atccaaaatt 180

caggaaatag atgggccaga tgcacgaatt gcggattatt ttgatgtaat agccggaaca 240

agcacaggag ggctgatgac aactatgctt gcagctccta atgagaaaaa tcgtcccatg 300

ttcgccgcaa aagacattac caacttctac tttcaacatt cgcctaggat cttccctaaa 360

ataggacaca catttgatga ggcgcaacct tatccttctc aaaacgaagc ctgcgaaatg 420

ggtcggacca aattcatgaa ttcggtagta accgtacttg gtgaggccac cggaccaaag 480

tatgatggta aatatcttcg agccatggca aagatgatgt taaaaaacct cactattaaa 540

gatacgttga cgaatattgt catacctgct ttcgacatca ggcggcttca acctgttatc 600

ttctcctctg ctcaaggaaa agaggtcgcg tggaaaaatg ctttgctagc agacgtatgc 660

attagtaccg cggcggcacc aacgtttttc ccgccatact attttgagac tagagatgtc 720

gatggaacca agcacacttt tgatctaatc gatggcgggg tagctgcaaa caatccgaca 780

catttggcta tcacacatat aaccaaagaa gcggtgatgg ggaaatacag gttctctggc 840

ccggaggttt tcgacggcag acggatgctt gtgctttcac tcggcactgg tacgcagacg 900

tacaatgact tatacactgc acaaaaggct gcaaaatggg ggttgcttag ttggatcttt 960

accaatggta ctgcgccaat cctccgcatt tttggtgatg ccatgtcaga tatggtcgac 1020

atccatgtgt caactatatt ccaatcgttg caagtcgaaa aaaactatct gcgtattcag 1080

gaagataact tgaaagggga agcaactgca atggatattt catcacccga gaacatgagg 1140

gcgctagagg acattggcaa gaaattgttg aagaaaccgt tgtcgagatt ggatgtggag 1200

acaggcaagc ttgaaccagt taaaggagaa ggtacgaatg ctgatgcatt agcacgtttc 1260

gccactttgc tttgtgccga acgaaagcgc cgcaatccag cttaa 1305

<210> 18
<211> 434
<212> PRT
<213> Helianthus annuus

<400> 18

Met Asn Asn Ile Asn Leu Val Ile Val Ser Leu Val Ile Ala Ile Val
1               5                   10                  15

Ala Ile Gln Pro Leu Ala Gln Glu Gln Thr Asp Val Gly Glu Ala Asn
            20                  25                  30

Phe Val Thr Val Leu Ser Ile Asp Gly Gly Gly Val Arg Gly Ile Val
        35                  40                  45

Pro Ala Thr Leu Leu Ala Phe Leu Glu Ser Lys Ile Gln Glu Ile Asp
    50                  55                  60

Gly Pro Asp Ala Arg Ile Ala Asp Tyr Phe Asp Val Ile Ala Gly Thr
65                  70                  75                  80

Ser Thr Gly Gly Leu Met Thr Thr Met Leu Ala Ala Pro Asn Glu Lys
                85                  90                  95

Asn Arg Pro Met Phe Ala Ala Lys Asp Ile Thr Asn Phe Tyr Phe Gln
            100                 105                 110

His Ser Pro Arg Ile Phe Pro Lys Ile Gly His Thr Phe Asp Glu Ala
        115                 120                 125

Gln Pro Tyr Pro Ser Gln Asn Glu Ala Cys Glu Met Gly Arg Thr Lys
    130                 135                 140

Phe Met Asn Ser Val Val Thr Val Leu Gly Glu Ala Thr Gly Pro Lys
145                 150                 155                 160

Tyr Asp Gly Lys Tyr Leu Arg Ala Met Ala Lys Met Met Leu Lys Asn
                165                 170                 175

Leu Thr Ile Lys Asp Thr Leu Thr Asn Ile Val Ile Pro Ala Phe Asp
            180                 185                 190

Ile Arg Arg Leu Gln Pro Val Ile Phe Ser Ser Ala Gln Gly Lys Glu
        195                 200                 205

Val Ala Trp Lys Asn Ala Leu Leu Ala Asp Val Cys Ile Ser Thr Ala
    210                 215                 220

Ala Ala Pro Thr Phe Phe Pro Pro Tyr Tyr Phe Glu Thr Arg Asp Val
225                 230                 235                 240

Asp Gly Thr Lys His Thr Phe Asp Leu Ile Asp Gly Gly Val Ala Ala
                245                 250                 255

Asn Asn Pro Thr His Leu Ala Ile Thr His Ile Thr Lys Glu Ala Val
            260                 265                 270

Met Gly Lys Tyr Arg Phe Ser Gly Pro Glu Val Phe Asp Gly Arg Arg
        275                 280                 285

Met Leu Val Leu Ser Leu Gly Thr Gly Thr Gln Thr Tyr Asn Asp Leu
    290                 295                 300

Tyr Thr Ala Gln Lys Ala Ala Lys Trp Gly Leu Leu Ser Trp Ile Phe
305                 310                 315                 320

Thr Asn Gly Thr Ala Pro Ile Leu Arg Ile Phe Gly Asp Ala Met Ser
                325                 330                 335

Asp Met Val Asp Ile His Val Ser Thr Ile Phe Gln Ser Leu Gln Val
            340                 345                 350

Glu Lys Asn Tyr Leu Arg Ile Gln Glu Asp Asn Leu Lys Gly Glu Ala
        355                 360                 365

Thr Ala Met Asp Ile Ser Ser Pro Glu Asn Met Arg Ala Leu Glu Asp
    370                 375                 380

Ile Gly Lys Lys Leu Leu Lys Lys Pro Leu Ser Arg Leu Asp Val Glu
385                 390                 395                 400

Thr Gly Lys Leu Glu Pro Val Lys Gly Glu Gly Thr Asn Ala Asp Ala
                405                 410                 415

Leu Ala Arg Phe Ala Thr Leu Leu Cys Ala Glu Arg Lys Arg Arg Asn
            420                 425                 430

Pro Ala

<210>  19
<211>  1531
<212>  DNA
<213>  Hordeum vulgare

<400>  19
atggcaagct actggtgccg gcgaccgtgc gagtcgtgca gcacgagggc gatggcgggc      60

agcgtggtcg gccagccggt ggcgccgggg cagagggtga cggtgctgac catcgacggc     120

ggcggcatcc gcggcctcat cccgggcacc atcctcgcct tcctcgaggc caggctgcag     180

gagctggacg ggccggacgc gcgcctggcc gactacttcg actgcatcgc cggcaccagc     240

accggcgggc tcatcaccgc catgctcacc gcgcccggcc aggacggccg cccgctcttc     300

gccgccaagg acgtcaaccg cttctacctc gacaacgggc cctacatctt cccccaaagg     360

cgagagcacg agcgatctca tggccatgga tcatgccagc tgaaccggtg attgatgtgt     420

gaattgtgtt gagctgtgcc aggaggtgcg cgctcgccgc ggtgaccgcg tcgctgaggc     480

ggccgaggta cagcggcaag tacctgcacg gcaagatcag gagcatgctg ggcgagacga     540

ggctgtgcga cgcgctcacc gacgtcgtca tccccacctt cgacgtcaag cttctccagc     600

ccatcatctt ctccacatac gacgtatgct aatttacgcc attgatcaga tagatccatc     660

ctccttggat cagacagaca tggggctaaa atcgttgaag gatcatgcgt gcaggccagg     720

aacatgcccc tgaagaacgc gcggcttgcc gacatctgca tcggcacctc cgccgccccg     780

acctacctcc cggcgcacca cttccacacc caagacgaca acggtaagga gcgcgagtac     840

aacctcatcg acggcggcgt cgccgccaac aatccggtaa ccaaccaatc aagcgtccat     900

cagtcatcac atattcagat acgctgcccg agcacactga tgaactgagt tgtgagagat     960

gcagacgatg gtgaccatga cgcagatcac caagaagatg atggtcaagg acagggagga 1020

gctgtacccg gtgaagccgt cggactgcgg caagttcctg gtgctgtcca tcgggaccgg 1080

ctcgacgtcg gaccaggggc tgtacacggc caagcagtgc tcccagtggg gcatcatccg 1140

ctggctgcgc aacaagggca tggcgcccat catcgacatc ttcatggcgg ccagctccga 1200

cctcgtcgac atccacgccg ccgtgctctt ccagtcgctg cacagcgacg gtaactacct 1260

ccgcatccag gacaactcgc tccacggccc agccgcgacg gtggacgccg ccacgcccga 1320

gaacatggcg gagctcctca ggatcggcga gcggatgttg gcacagaggg tgtccagggt 1380

gaacgtcgag accgggaggt acgaggagat acggggcgcc gggagcaacg ccgacgcgct 1440

cgccggcttc gccaaacagc tctccgacga gaggaggaca aggctcggcc gccggcgcgt 1500

tggcgccggc cgtctgaaat ccagacgctg a 1531

<210> 20
<211> 1269
<212> DNA
<213> Artificial Sequence

<220>
<223> cDNA of Hordeum vulgare phospholipase

<400> 20
atggcaagct actggtgccg gcgaccgtgc gagtcgtgca gcacgagggc gatggcgggc 60

agcgtggtcg gccagccggt ggcgccgggg cagagggtga cggtgctgac catcgacggc 120

ggcggcatcc gcggcctcat cccgggcacc atcctcgcct tcctcgaggc caggctgcag 180

gagctggacg ggccggacgc gcgcctggcc gactacttcg actgcatcgc cggcaccagc 240

accggcgggc tcatcaccgc catgctcacc gcgcccggcc aggacggccg cccgctcttc 300

gccgccaagg acgtcaaccg cttctacctc gacaacgggc cctacatctt cccccaaagg 360

aggtgcgcgc tcgccgcggt gaccgcgtcg ctgaggcggc cgaggtacag cggcaagtac 420

ctgcacggca agatcaggag catgctgggc gagacgaggc tgtgcgacgc gctcaccgac 480

gtcgtcatcc ccaccttcga cgtcaagctt ctccagccca tcatcttctc cacatacgac 540

gccaggaaca tgcccctgaa gaacgcgcgg cttgccgaca tctgcatcgg cacctccgcc 600

gccccgacct acctcccggc gcaccacttc cacacccaag acgacaacgg taaggagcgc 660

gagtacaacc tcatcgacgg cggcgtcgcc gccaacaatc cgacgatggt gaccatgacg 720

cagatcacca agaagatgat ggtcaaggac agggaggagc tgtacccggt gaagccgtcg 780

gactgcggca agttcctggt gctgtccatc gggaccggct cgacgtcgga ccaggggctg 840

tacacggcca agcagtgctc ccagtggggc atcatccgct ggctgcgcaa caagggcatg 900

gcgcccatca tcgacatctt catggcggcc agctccgacc tcgtcgacat ccacgccgcc 960

gtgctcttcc agtcgctgca cagcgacggt aactacctcc gcatccagga caactcgctc 1020

cacggcccag ccgcgacggt ggacgccgcc acgcccgaga acatggcgga gctcctcagg 1080

atcggcgagc ggatgttggc acagagggtg tccagggtga acgtcgagac cgggaggtac 1140

gaggagatac ggggcgccgg gagcaacgcc gacgcgctcg ccggcttcgc caaacagctc 1200

tccgacgaga ggaggacaag gctcggccgc cggcgcgttg gcgccggccg tctgaaatcc 1260

agacgctga 1269

<210> 21
<211> 422
<212> PRT
<213> Hordeum vulgare

<400> 21

Met Ala Ser Tyr Trp Cys Arg Arg Pro Cys Glu Ser Cys Ser Thr Arg
1               5                   10                  15

Ala Met Ala Gly Ser Val Val Gly Gln Pro Val Ala Pro Gly Gln Arg
            20                  25                  30

Val Thr Val Leu Thr Ile Asp Gly Gly Gly Ile Arg Gly Leu Ile Pro
        35                  40                  45

Gly Thr Ile Leu Ala Phe Leu Glu Ala Arg Leu Gln Glu Leu Asp Gly
    50                  55                  60

Pro Asp Ala Arg Leu Ala Asp Tyr Phe Asp Cys Ile Ala Gly Thr Ser
65                  70                  75                  80

Thr Gly Gly Leu Ile Thr Ala Met Leu Thr Ala Pro Gly Gln Asp Gly
                85                  90                  95

Arg Pro Leu Phe Ala Ala Lys Asp Val Asn Arg Phe Tyr Leu Asp Asn
            100                 105                 110

Gly Pro Tyr Ile Phe Pro Gln Arg Arg Cys Ala Leu Ala Ala Val Thr
        115                 120                 125

Ala Ser Leu Arg Arg Pro Arg Tyr Ser Gly Lys Tyr Leu His Gly Lys
    130                 135                 140

Ile Arg Ser Met Leu Gly Glu Thr Arg Leu Cys Asp Ala Leu Thr Asp
145                 150                 155                 160

Val Val Ile Pro Thr Phe Asp Val Lys Leu Leu Gln Pro Ile Ile Phe
                165                 170                 175

Ser Thr Tyr Asp Ala Arg Asn Met Pro Leu Lys Asn Ala Arg Leu Ala
        180                 185                 190

Asp Ile Cys Ile Gly Thr Ser Ala Ala Pro Thr Tyr Leu Pro Ala His
    195                 200                 205

His Phe His Thr Gln Asp Asp Asn Gly Lys Glu Arg Glu Tyr Asn Leu
    210                 215                 220

Ile Asp Gly Gly Val Ala Ala Asn Asn Pro Thr Met Val Thr Met Thr
225                 230                 235                 240

Gln Ile Thr Lys Lys Met Met Val Lys Asp Arg Glu Glu Leu Tyr Pro
                245                 250                 255

Val Lys Pro Ser Asp Cys Gly Lys Phe Leu Val Leu Ser Ile Gly Thr
        260                 265                 270

Gly Ser Thr Ser Asp Gln Gly Leu Tyr Thr Ala Lys Gln Cys Ser Gln
    275                 280                 285

Trp Gly Ile Ile Arg Trp Leu Arg Asn Lys Gly Met Ala Pro Ile Ile
    290                 295                 300

Asp Ile Phe Met Ala Ala Ser Ser Asp Leu Val Asp Ile His Ala Ala
305                 310                 315                 320

Val Leu Phe Gln Ser Leu His Ser Asp Gly Asn Tyr Leu Arg Ile Gln
                325                 330                 335

Asp Asn Ser Leu His Gly Pro Ala Ala Thr Val Asp Ala Ala Thr Pro
        340                 345                 350

Glu Asn Met Ala Glu Leu Leu Arg Ile Gly Glu Arg Met Leu Ala Gln
    355                 360                 365

Arg Val Ser Arg Val Asn Val Glu Thr Gly Arg Tyr Glu Glu Ile Arg
    370                 375                 380

Gly Ala Gly Ser Asn Ala Asp Ala Leu Ala Gly Phe Ala Lys Gln Leu
385                 390                 395                 400

Ser Asp Glu Arg Arg Thr Arg Leu Gly Arg Arg Arg Val Gly Ala Gly
                405                 410                 415

Arg Leu Lys Ser Arg Arg
        420

<210> 22
<211> 9001
<212> DNA
<213> Beta vulgaris

<220>
<221> misc_feature
<222> (2702)..(2702)
<223> n is a, c, g, or t

<400> 22
ttttccaaca acataccgag tagttttctc caagtttaag ttatttatgt tattgtaatt 60

gtaatgtaag agctagtaaa gctcagttta gtttctttgt ttttagtatg aatgtaccat 120

ttttagtatg taattatgct tttaattaat gcaaaatttt ccgttgtaaa aaaaagtcta 180

catgtccctc ttgcagagat aaatgtaagg gacattattg cttagttctc gactaattta 240

caataagttt ataattggtt atcatggttc atgagtactc ctgttcatag aaggacaata 300

atttataaag gtttcttagt agcttccaat gacggagctc aatgaccctg ttaattagct 360

aagtaataag ttggccatta gccaataaca actgtattat tagctagtaa gttcattaga 420

tcgatataac ttataagtct aatgaatata aatatgaata agatggaact gaaatgagtt 480

ttatatttga aaaattagtg gcatacgtgc ttaaacgttt ttttgattat ggctagaaga 540

cttagaacga agttggagga attcttcatt catataaatt tatgctagat aaactaatag 600

ccagtcaagt caagtttatg tttttaaaaa aaatcttatc tcatgtagta cgagaatctt 660

tattttataa tttcgttata ataaatatgc atttcactcc tttcaaatcg ttagctacat 720

cacttctgca accaagcaag caagccataa tatttaatca caggttcttt aatactatag 780

tctatatata catgcaatcc acttacatta attatcatct tattctccca tcatcaagaa 840

aacacattac aatccaaaga aataaaacat ggagaattct agatctctgg gtaaccctgt 900

tagccctaga ccccctaatc atggtaatct cataacaatt ttaagtattg atgggggagg 960

cattagggga ataatccctg cagttatgct tgaattcctt gaatcacaac tccaggtatg 1020

tacataatta agaaatgatg atatacgatc atctttagta ctcttgttaa atgatttgac 1080

gtaagaaatc atgtatatat tatgcatgcc ttactataat attgcgtgca tgttcataat 1140

cacaacagaa gttagatggt gaggaagcaa gattggctga ttattttgat gttattgccg 1200

gaacgagcac cggtggcctc ataactgcca tgttaaccgc ccctaacgac aaaactcggc 1260

cactctacgc cgccaaggag attactcctt tttatcttga gcattgtccg aagatcttcc 1320

gccaaccaag gtaaataaat caaatttatc atctatgtat atcaggattg gtaaattaga 1380

gaggatgaaa aacccttaaa tgcttggaac tattaacgct cgagttccaa ttatgggcca 1440

ttgtctctta taggtctcaa ctattgattt ttttctatag caggcgcgga tgtaagattt 1500

tgaatttgga agggtcctaa ctaaaccaaa gaaataaatt acaatgacca tattaacata 1560
taagaagttt agttttaaca ataagaaaac caacagttgg tcttgtaaga gaccgtctac 1620
ttttaattta aaagtaggcg cggcccaagc ccaatattaa gaagagaata gatcatattt 1680
cttaatatag tgactacttt aatctattta gtgaccattt taatctattt agtgaccagt 1740
ttaatctatt cattaataac attctttaat tcatttagtg actgatttaa tccatttagt 1800
gactagctta atccattttg taagactgac ggactatttt taaaaattgt ttgttctaat 1860
tcaaaattta gaagggaaat aaaagaagga gaagagaatg gacgactagg atggtgcaag 1920
atacctaaaa aaagtgttag gttagagaga aattgaatgg gcttcttagg agaatgtagg 1980
tttgcattgt ctaattttat attaaaatta gaccgtctat tataataatt tgtggaaaac 2040
aaaactttat attatttgaa tcatgtatta gatatacctt aattatattg atatcttaat 2100
gttatcttca tttttagtaa attcaacaga tttgatttgt acatccgtta acatatgcga 2160
cactaacatt tgatatcttc agttatatgt tcataatttt tttttttaaa aagttggaca 2220
taaatttggt aaacgaaaat agagacaaat tcacaatttt acatagtaat attattttca 2280
agtatttgga aacaagcttg gtcaaactta atatagcaat accgtagaaa gtcaaattat 2340
tgaatttatt tgaaacaaaa ggagaataat cgttatatca caaaaaaaaa agtattaaaa 2400
ttagagctaa attaagagag attggtagca ttcaagataa tgaaatgatg attagatata 2460
aaaaagaaaa gatgataaaa gagaaaagtt aaagaacagg aagggcatgt tctcttcagc 2520
ataaaacagc tgaactgaac taacctgaat tgaactgatt ggagctgaac tgaactgaac 2580
tgaactgaac tgaattgaat ttaatttaat ttaattgtat tgaattaaat taaatttaat 2640
ttaatttaat tgaattgaat taaattgaat gtaataaata tataaataat atttaaacaa 2700
tnagtacagt tattataata ataattgtgc ccttctgaaa ttgttaaggc acgtcgtttg 2760
caagatttgt ccttttgcat taaaatttgg gtgcaaatcc tgcacaaatt gaaatatata 2820
gtattatctc tccactcatt tttatcttct tgtatcattt taggctcgac aataatgact 2880
ctgccagagg ggtagttgaa ccttcttata tcatcatcat aattcataat aataatttta 2940
ttatatatac tccctccgtt ccaaaatata gttctcgttt ctttttttta cgatgatttg 3000
tgcaagtaga atctaagtgg aataataaaa tttttttgtt tatttaaata aatattgtat 3060
ggaaaaggat gattttagga gagaaaaatg gaaaataatt ggtgaaagag tattaattgt 3120
aatattttag ttgaataaat aacagaaaaa ccaaaatata agaagcaaat aaatgatggg 3180
acacgatctt tgtagacaaa ttacagaaaa atttggaaac gaatatggaa atgggaacta 3240
tattttggga cacccaaata gaaatgggaa ctatattttg ggacggaggg agtaatatta 3300
ataatcataa attattaata ataatagtaa taattaatga tactaatgat aattaataac 3360
cgaagatcgc gtgattattt gtgttggagt tgtagtggga tatttggttc aatagggact 3420

ctaatgaagc tgctaagcgg gccgaaatac gatggaaagt accttcacaa tctagtaaag 3480

gaattgctcg gacaaagaag gttgcatcaa gcgttgacta atgtggttat cccaactttt 3540

gatatcaaga atcttcagcc tgttctcttt tctacttata tggtatgttg ttgaatttcc 3600

aatttattga agtacattat tattgtaccc ttggttatct tattcattag caaatactag 3660

cattcaaata gcatttatag tgatttgtca agaaaaaaaa taagtagagg aatcatagtt 3720

atcacttatt agaactacct cttttagaag aatatacttc ctctattttt ttagatggaa 3780

catttacctt tttggtgagt agattaaaat aatcatatgt tgtatttaaa aaaacagaat 3840

tagtatatat cttcttttgt gtcatataat acacttgatc aatttaaaag ataacatgat 3900

tccaatattg acttttttat acacataatt tcacatgtca aataatagaa aaaaagtcaa 3960

ctttgctgaa tttggagatg ataatacata gtttatgtgt tggttaagta atttcatact 4020

actacattga tagattatga aattgatcaa gaaaactaat caataattaa tgattataat 4080

gatctaggtc ccaattgcta aagaattgga cgtgttgctg tcagacatat gcataggcac 4140

gtctgcagct ccaacgttcc taccagcgca tcattttgaa aacaaagatg atcagggcaa 4200

tgtcaaagaa ttcaacctca ttgatggtgg tattgctgcc aataatcctg taagattatt 4260

tcttcatcca tgtttggatt ttctctccat ccctaaattt aatttgatta ggaattagtc 4320

attcttaata aaaattatga atcatcaatt aaattatcta ataaaaagta attcaaacat 4380

ttgttcgata taatgtactt atttttttta ttaaatttta attttcagac atattccaaa 4440

tgtcgtccta caaaagaaat tgcaaatcca tctaaaaact tcgtttgata catctaataa 4500

tgtatgttcg agtcaatcat taaattttca tatcaagata aatatcaaag taagatgttt 4560

caaactttta cttagtcgat taattctatt aattccagtc aatttaccat caacttcttc 4620

ggtcttccca aaacaaatgg aaaaatatat attcactgca tacgtgtctt tgtgttatat 4680

tcctatctct aaagtattta agtttttatg atattaggac atatatattc tctagtattt 4740

aatgagaaag atcgaaaagt tttcgtatat tcataaaacc ttaacaactg ctattcaaaa 4800

tatacgataa gtttcatcaa cattacccac taattaaatg taatttcact gtataagtac 4860

agtcattagt ggcaataagc gaagtgacaa aacaaatagt gaagagcaac ccgaatttct 4920

tcccgataaa ggccacggat cgtgaacggt tgctggtgat atcgttaggg actggatccg 4980

acaaggtaga gcagctctat aatgctaaaa gcgcagcaaa gtggggaatt atttcatggt 5040

tatttgacaa tggtaatact ccactacttg atgcttttaa tcaatcaaag gctgatatgg 5100

tcgatttcca caattctgtg gcctttcaag catatggttc tcttgacaat tatcttcgca 5160

tccaagtaag ttatttttat tatacggatt tggatgttta tgtacgtata tatacttctg 5220

ttaaaggttg ataagatgag agttacgcgc tcattttacc aaggatcttt ggtttcaatg 5280

gtaatttggg accggtcaga cccgtggctg cgggcctatc acggtcaaga atttttattt 5340

caggctcgca ccgtcacgag cctgcctaga tggctcgcga gccgaacact catttccttt 5400

gctttttagc ctaaaactgt caaaagtcca aaaaacgaca taaaacacgg gccagctcgc 5460

aggagccacg agcccagtcc gtggataaaa tcttgaaatt tcaggcccga cccaccattt 5520

ccccaaaccc gcgggccgag cccgtgaaca agctcatgcc ctgatgccca ggtctaggtc 5580

tgcctcgcac ggagccttca tcccgaagcc cattacaagc cagacaccca actccctaca 5640

acaccacatc aacccttcca atccactcgc cttccctctt cactgctgcc ggctgccctc 5700

taggtttatc attttttcta agctcttttg tctaagttaa atccactggt ttctttcatt 5760

ttctttttga tttaagttcg attaccaatg tttctttaac tttgcaactt ttggggagat 5820

accttattgt aatttgctct aatgaaagtt tcttatgctt ataataatgc tcaattgtct 5880

ttcttgatct agtaatctga gagttatcca gttttaaata gttagagcta gtttcactaa 5940

tggtggcaat aagggtgtgg cagggggcga tgcttggttt tggactttgg taatggtggt 6000

aatgaaggga ggttacacag tgaggatggt ggttggtgta attggtgggg aaactcgggg 6060

tagagagata acccctaaag gcgagattat gcttagtcaa tcgaaaggtg tgcctatttt 6120

aagcggacca cctaaaggtc ggattattat aatcaatttt tcctaaataa attgcaattt 6180

gtggttggat ttctagtttg aaactaaatc tcaattgcaa gtctgcaacc gtatgaacta 6240

tgaaccattt tgaggttaat atatctatag atttttattt caaaaaaata taaaaatata 6300

ttatttcaaa aaggtccaag aatacgtctt ttaagagtgt ccaagaacat ctaaccacga 6360

gttatatatt tgtctattat gtggtgatca ctttttcaag atttgaggtc tttaattagg 6420

ccttgatgat atagatttgt tagagaaaaa catttgcttg attaaaaaag gaaataaatg 6480

atatgacaaa tgactaattg actcatgtct aaccttttaa atgtggtgtg atttaactcg 6540

acatttaata tgtggttggt ttgataaatt ttagcgagca cgctttatat aagtgtaact 6600

tatatatctt gtgcataagt ttagttattt attttttact ttattaaaca aaattgataa 6660

atatgatata gttaattaaa tatattactc tcataaatca agctacccta aatctgtaaa 6720

tgcaaattat attgcaggat gatacactaa agggagtatc agcatcagtc gatgtggcaa 6780

ccacagaaaa tttggcaaat cttgtcacaa ttggaaaggc actcttgaaa aagccagtat 6840

ccaggattaa tttcgatacg ggtagatatc aacctatccc taatggaggg accaatgagg 6900

aagccttaat taggtacttg atttaaataa atcattcata aagaatcacg ctcattgctt 6960

gtcaataaaa tatttatgtt tatgtttaat actaatgctt gttttctaat acttttaaca 7020

ctttactttc ttacatgaga tgccgagacg agtgtagagt aatttattgt gtaggtaact 7080

ttttgctcta tatgagtcac ttatataaaa gtgacatata tatggtaata agttacctgt 7140

aagacgaggt taactatttg ccataaatag gtcactttac ttatatagaa gtgactcatt 7200

taagattaaa attaagttac cttttattta aaattgaccc atattgattt gaaaagtgac 7260

ccatattgat ttgaaaagtg acccatttaa gctttaagtg aactgatgtg acaataaatt 7320

tattatacac ccggtgcaca taatacaaat tgtttaaatt atcaagggtt tgttggacaa 7380

aagtaattgt tagttgtaag cttctagcta tgagtttgtg agcttttaga tgcctaggta 7440

gtttctaact tttagttatg aaactagttg tctaaagtgt ttggtaataa taattattga 7500

cgaaatttta acatgtaaat aatatttaaa tttggcaact tttgataaaa gctaccgaaa 7560

agcttaatca tcttagattt tgaccaagac tactcaaaag ctaaaagtta tctaaaagtt 7620

acatccaaaa gctactttga ggaatattta aacaattttt tcaaagacta aaagctatct 7680

taactacata aaaagtatgt caaaagttac accaaacatg tcctacattg accacgttat 7740

tctaacctac aaaactgact gggcaaacct gctactttgc aagaggtatc agtttattag 7800

ggtgtttcat cttacctacc taaacagtaa aagtaaatat ataaataaaa caaatattaa 7860

ttttatataa atattttatg agattagtat taatgtatat cctataatat caacattcaa 7920

aaaactttta cgaatagata aaaagatata ttaagaatta aaattatgca aattcaaaag 7980

tcaattcaaa ttactcctat taaaaattgg cggtatatga tttaaacttt caaaaataag 8040

aaaattataa aattatgtca ctctttgtta ctactgaata catgtttgtc caattgacag 8100

gtttgcgaaa ctccttaccg aggaaaagag cagacgcgaa caatatcgga cgaaccgaag 8160

tgactaatat cacatttttt aaacccatgt ttcaatggat actatgatta tatagtatat 8220

atatatgtat acaatacatc atgatctgga aaaaaactta cgctcgattt atgataaata 8280

aatttgtcaa accatgtagc tctcacaaaa tctgtaatat ttaatgaatt ttagtatgcg 8340

tagatatggc ctatgggcgt ggaatattta tccatgtaac tcatttattt ttacagctaa 8400

gtcattgcat tttaatctac gaaatttctc ttttaatcaa taacctaaaa ataaatagtt 8460

tgtgcatcaa tttctctcta tttactctgt aagtatttgt tgtttttctt tattgaacga 8520

taactacagt gccactccta tatatacaag agatcacttt ctaaacctag ggataactat 8580

acaagaagag aaactaaccg ttaaaatctt tctacgatca ataaaaggag gaatatataa 8640

tcacatgaaa gtgcgaaata atcaatacaa gtgagtttaa ttccctccgc cactctaact 8700

catttcccta ctctacactt aattctccca cgctatcaat cccattcatt tactccagta 8760

tcagatgcta gtaattcttc atagaatatg aaatatacta ggattatatt ttgcgtttgg 8820

atatagtttt ggtagaataa caattctact ctagttacgt tttctactat tattgggata 8880

tcctttagaa tgtaacctat aaaatatgat caataaaaac actcaaatca aggaagttta 8940

cgcaaatata gcctggtatc aaagctatac taatcttaaa gattattttt tccttcgatc 9000

c 9001

<210> 23
<211> 1224
<212> DNA
<213> Artificial Sequence

<220>
<223> cDNA of Beta vulgaris phospholipase 1

<400> 23
atggagaatt ctagatctct gggtaaccct gttagcccta gaccccctaa tcatggtaat 60

ctcataacaa ttttaagtat tgatggggga ggcattaggg gaataatccc tgcagttatg 120

cttgaattcc ttgaatcaca actccagaag ttagatggtg aggaagcaag attggctgat 180

tattttgatg ttattgccgg aacgagcacc ggtggcctca taactgccat gttaaccgcc 240

cctaacgaca aaactcggcc actctacgcc gccaaggaga ttactccttt ttatcttgag 300

cattgtccga agatcttccg ccaaccaagt gggatatttg gttcaatagg gactctaatg 360

aagctgctaa gcgggccgaa atacgatgga aagtaccttc acaatctagt aaaggaattg 420

ctcggacaaa gaaggttgca tcaagcgttg actaatgtgg ttatcccaac ttttgatatc 480

aagaatcttc agcctgttct cttttctact tatatggtcc caattgctaa agaattggac 540

gtgttgctgt cagacatatg cataggcacg tctgcagctc caacgttcct accagcgcat 600

cattttgaaa acaaagatga tcagggcaat gtcaaagaat tcaacctcat tgatggtggt 660

attgctgcca ataatccttc attagtggca ataagcgaag tgacaaaaca aatagtgaag 720

agcaacccga atttcttccc gataaaggcc acggatcgtg aacggttgct ggtgatatcg 780

ttagggactg gatccgacaa ggtagagcag ctctataatg ctaaaagcgc agcaaagtgg 840

ggaattattt catggttatt tgacaatggt aatactccac tacttgatgc ttttaatcaa 900

tcaaaggctg atatggtcga tttccacaat tctgtggcct ttcaagcata tggttctctt 960

gacaattatc ttcgcatcca agatgataca ctaaagggag tatcagcatc agtcgatgtg 1020

gcaaccacag aaaatttggc aaatcttgtc acaattggaa aggcactctt gaaaaagcca 1080

gtatccagga ttaatttcga tacgggtaga tatcaaccta tccctaatgg agggaccaat 1140

gaggaagcct taattaggtt tgcgaaactc cttaccgagg aaaagagcag acgcgaacaa 1200

tatcggacga accgaagtga ctaa 1224

<210> 24
<211> 407
<212> PRT
<213> Beta vulgaris

<400> 24

Met Glu Asn Ser Arg Ser Leu Gly Asn Pro Val Ser Pro Arg Pro Pro
1               5                   10                  15

Asn His Gly Asn Leu Ile Thr Ile Leu Ser Ile Asp Gly Gly Gly Ile
            20                  25                  30

Arg Gly Ile Ile Pro Ala Val Met Leu Glu Phe Leu Glu Ser Gln Leu
        35                  40                  45

Gln Lys Leu Asp Gly Glu Glu Ala Arg Leu Ala Asp Tyr Phe Asp Val
    50                  55                  60

Ile Ala Gly Thr Ser Thr Gly Gly Leu Ile Thr Ala Met Leu Thr Ala
65                  70                  75                  80

Pro Asn Asp Lys Thr Arg Pro Leu Tyr Ala Ala Lys Glu Ile Thr Pro
                85                  90                  95

Phe Tyr Leu Glu His Cys Pro Lys Ile Phe Arg Gln Pro Ser Gly Ile
            100                 105                 110

Phe Gly Ser Ile Gly Thr Leu Met Lys Leu Leu Ser Gly Pro Lys Tyr
        115                 120                 125

Asp Gly Lys Tyr Leu His Asn Leu Val Lys Glu Leu Leu Gly Gln Arg
    130                 135                 140

Arg Leu His Gln Ala Leu Thr Asn Val Val Ile Pro Thr Phe Asp Ile
145                 150                 155                 160

Lys Asn Leu Gln Pro Val Leu Phe Ser Thr Tyr Met Val Pro Ile Ala
                165                 170                 175

Lys Glu Leu Asp Val Leu Leu Ser Asp Ile Cys Ile Gly Thr Ser Ala
            180                 185                 190

Ala Pro Thr Phe Leu Pro Ala His His Phe Glu Asn Lys Asp Asp Gln
        195                 200                 205

Gly Asn Val Lys Glu Phe Asn Leu Ile Asp Gly Gly Ile Ala Ala Asn
    210                 215                 220

Asn Pro Ser Leu Val Ala Ile Ser Glu Val Thr Lys Gln Ile Val Lys
225                 230                 235                 240

Ser Asn Pro Asn Phe Phe Pro Ile Lys Ala Thr Asp Arg Glu Arg Leu
                245                 250                 255

Leu Val Ile Ser Leu Gly Thr Gly Ser Asp Lys Val Glu Gln Leu Tyr
            260                 265                 270

Asn Ala Lys Ser Ala Ala Lys Trp Gly Ile Ile Ser Trp Leu Phe Asp
        275                 280                 285

Asn Gly Asn Thr Pro Leu Leu Asp Ala Phe Asn Gln Ser Lys Ala Asp
    290                 295                 300

Met Val Asp Phe His Asn Ser Val Ala Phe Gln Ala Tyr Gly Ser Leu
305                 310                 315                 320

Asp Asn Tyr Leu Arg Ile Gln Asp Asp Thr Leu Lys Gly Val Ser Ala
                325                 330                 335

Ser Val Asp Val Ala Thr Thr Glu Asn Leu Ala Asn Leu Val Thr Ile
            340                 345                 350

Gly Lys Ala Leu Leu Lys Lys Pro Val Ser Arg Ile Asn Phe Asp Thr
        355                 360                 365

Gly Arg Tyr Gln Pro Ile Pro Asn Gly Gly Thr Asn Glu Glu Ala Leu
    370                 375                 380

Ile Arg Phe Ala Lys Leu Leu Thr Glu Glu Lys Ser Arg Arg Glu Gln
385                 390                 395                 400

Tyr Arg Thr Asn Arg Ser Asp
                405

<210>  25
<211>  14701
<212>  DNA
<213>  Beta vulgaris

<220>
<221>  misc_feature
<222>  (10783)..(10802)
<223>  n is a, c, g, or t

<400>  25
gagtgtggag tagttgcttt gtgcgagcgt gttttgtttg tgcgaacttc tgctgtcacg      60

ttttgtttgt ggaattgctg tcacgtttaa ttaccattca agtgtgatct ccttctagta     120

gattgtgatc ttgcttgtgg tatattggat ggtgtgagcc ccagacgtga ttttgttcgg     180

ttgatgcggt tgtgcatcgt ttctccaaaa ccctagttgg tttggtgcgg ttacgcatcg     240

ttattttgat gggttatggt ggcccatcca agctattgtg ttggtgcggt tgtacatagt     300

tgatgcccta agtgacgaat tgaatcatga aggttggttg gttgtccata attcaaaaag     360

ggtatttctt ggttgtccat aattcaaaaa aggtatttct tggttgtccc taatccaaaa     420

tggtgtgtat tggttgtccc taatcttaaa agctggttat tgaaggcgtt gatttcacgt 480
cttattggtc tcctagtaat aattctttta tatatttttt caattggagt gttggtgtta 540
ctatcctatg attgaggggg gagtattaga gttatggtaa gtatattata ttagagtatt 600
atgtttggag tattatattt attaggagtc ttgtattata tacctacgag ctttcatatg 660
cacgcgatgc gtgcgttaat ttcaaaattt gttattctat tagatactcc attcgtactt 720
tatttatgat taaacttaaa tgtgaaaaat tcaacagtta gatgaattta gatatattaa 780
ttcgctaatg taaaaaaata aaaaggttgt gagtgattgt taacattaat tttttgtgaa 840
tgaatattga acagaaagaa caaaaataat gagttggttt aactaaatta aatagtatca 900
aaagtagtga atgttcagac ttttcatgtg cacgttatgt atgccatctt ttttttataa 960
gttataaata ttttgtccat ttaatttttg attgaagtat ttgatgcata taccatttta 1020
aacttgtttt aaatggcaag ttggaatgag aattttttaa atgatttgct tttgtaattt 1080
tttatattct attgattaga tcattaaggg tattttggtc actttaaaag aggacaccac 1140
acctcctccc ttggcttata taatagagat ctgttaagtt gttagtacgt atatctagtt 1200
cagtaaggat ttctgatatt aggttgtata tatatattcc tcaaaagttt gataagtttc 1260
ccatattcta aaccttaaca ctttgagtga gtgaaatgtg gcagaagcta atttctttta 1320
tgtatattga tgatatacac agaatgcatg catgtgttta tagaagactt accattcaaa 1380
atcaacaata tacatggatt agaacaattt ttaaaatgta cttcaatttt ctctcactgg 1440
acttggacac gattggggag aagaaggtgg tggacaccgt gtaggaggag aagactgaag 1500
agaagacata caccatacct ttcaagaaga aggatagagt atgtgtgtat gtattagcta 1560
ggcagactgt tggagtctag aggttgagat gtggaataag aaggagaata atacgggtga 1620
gaggttttag ttaattgtca aatgttgcgc tctagcatat gtccttattg atagatgact 1680
gtcaaagttt ttgatcacta gctccttatt tcactactaa atactaatta cactaatcga 1740
ctgtacatac acccacacac atatatatac atatatgaga atgagggata acctagtggt 1800
taatgcttct ctcccggttc caggagtcct atgatcgatt ctcacccttg acaagttagg 1860
ttctccttta ttccccaagc ttatcacacg tacacacgtc gctgtcatag acatgtcatc 1920
tttaagttta agttaaaaat ttataataaa ttaattaagt gtatggcatg catgtatagt 1980
tgcttccagc aattaattcc tcctaaaatt aaatagttag cttaccagtc cacaatgtag 2040
gaataacaaa gaataaatgg gggaagtaga gtgaatatat tatataattc atataatagt 2100
ctcgcggcta ttaagaagac gatttttgct aagctggtta tggtgtgttg tttcttcttt 2160
tttttctctc ttgctcaggt aattaggttg gtgtttgaga aataatactg aaaatgacga 2220
acttacaaag ctctcctcca tccttcaatt actacaacag gaagaaattg gtcacaatcc 2280

ttagcatcga cggtggtggc attcgaggca ttattcctgc tgtcattctt gcttttcttg 2340

agcgacttct tcaggtacgt acataaaaca taatcatact acatatacat atctcctata 2400

tatccattca tctctcgcac tcatttgtac tcatttagcg tgtgaatcgt gcatatcaca 2460

tcgaacaatg ttattttgat atgaataatg ataattaggt tcctagtaga gttgggcatg 2520

atagccgggc tagtccaaca cgttccacca tgactttatc cggttatccc cacacgatac 2580

aattttgttc gaaagtgaaa gacactgtta tgggtcatat atagacatca tttttaaaat 2640

ttggcataaa tatgagaact atgtaatttc tatcttgtta tcattgatac cttgtaaaac 2700

tatgcaatcg tgcatgcacc taatgaataa taacaataaa caataaagta actaattaag 2760

tataatgagt agtgtacaat tgtacatgtg cacaataatt tcagatgcga aatgaaaata 2820

ttatttacta cgttcataat atgttaaaaa aagcactcct gagacataat gtgatgcata 2880

gttaccccgc aaaaaacaat taattaatat gatgtcaggt taattggtta taattatgta 2940

ggagttggat ggagaggaag taagactagc agattatttt gatgtgatag caggaacaag 3000

cacgggaggg ttgattacag ccatgttaac tgcaccagat gactataatc gtcctctata 3060

tgctgcgaaa gatatcacaa cattttactt gaagcatggt cctcaaatat ttcctcagga 3120

aaggtaaaaa atttaaatgt cagaatattc cataccacaa tataattttt aataagtagc 3180

ctataaatgg tggtcacacg ctaagcatga caattttatt aaaagatcaa tcgaattttt 3240

attaaatctt ataggattaa aaattagaat tcattgggtg gaaaaaaaaa tgaaaaaggt 3300

tgtgattttt cgtccatcgt taaatcgaaa caaaaagaaa agtcaaagta aaggcttatt 3360

atttctcatt tataaatatc caaattttag tacccaatac ccataaatag ttcgaaccat 3420

atagacacaa taatcaattt tcgtgtgaat ggtttgtagg gaaaccctat cctttcttat 3480

ccataatggt gtataaataa attttaccca caaaaatata caagtttctt catatactat 3540

atcatgaaaa taactattat gacatatatc tacttagtta cccgtcgagt aggtttacta 3600

aacttatcca tacatcaaat atgtcaaaaa gaatccagtt aattgtttga ggatgtgagt 3660

catgtgattg gtgagctatc aattgttcat cactttctaa attcaaactc gaggaagtaa 3720

tcaattggtc ttggcatagt ggttgtccac tttgtccctt aatcaattat cgaggattcg 3780

attctcaatt tttgggaatg gaaagatatt tttgaccagt cctttaccct taagtgtagc 3840

actcgaagcg agaggattaa tcattgctgt cggcggtgaa tacttagatt gtttttaaaa 3900

aaaacttgag ggagtgatac tagcagatca tgtatcctca tgtttgtcaa tgaaggtgtg 3960

caaagtatat gctagtatgc tactaccgcc catgaccctg aaattcaatt tttcattgtt 4020

tgttttagtt gcattgtcct atttggctat ttcaatactt tagtaacttt aaaaaaaaaa 4080

tacttcctcc atctcttttt actcgctaca ctttttatta tgggaagttt cacattactt 4140

gctacacttc ttttttgggc aataaactat ctctatctct ctcctcccac atgagtcccg 4200

tgttgagatc ctctagagtt ccaaaaaaac tctacacggt agagttcgtt aaatatcagt 4260

cgttgattaa gaaatcaaag gcttgtattt tgttttccaa aaatcccccc tattttcccc 4320

cgaatatcct gccaatatct tttccttttc caccaatcat gagcctttga ttttatgata 4380

tatggtcagg attcaactct actttgtaga gttttcataa aactctagag gatccggacc 4440

ccatgagtcc cttttctttt tattctctac ttctctcttc tccaaaaatt gcctaggagt 4500

ccgtgaaatg agaagtgtag cgagtaaaaa gaaacggatg aagtatattg ttaatatttt 4560

ttaaaatata tatattttat tgtaggatta atcttaataa ttactaaggg atttccaaca 4620

tgtttgatgt cgcctgtatt ttgctgacca agtttagaaa aattgatagg ttgtttctaa 4680

catctttttg ccggccacca agctggagat gtagacatat agaagtagtt tatgattttc 4740

ccactttgaa ctggttatac atacattaat tagttattac aagtcttttt tagatgatta 4800

ctccatcgat tccaccattg ttcatgtcat cttgtcatat acagcgactt tcgtaactat 4860

tgtgtattat tattatatac tccctcttta ccaatctaga tgcaattatt ttccttttat 4920

ggtttctccg gacacacttt ttacatctat gattatatat tagtagaatt tagttataga 4980

tgatatattg taaatttata cattattgaa acaaatctaa tatgatattt atatgataat 5040

tttatttgct tatatcagta aaaaaatatg cttagttaac attgatgatc tttaacccta 5100

gaaaaacaaa tgttgcaagt tatatggaat taacaaagga ggttatattt ttcttggaaa 5160

agaaccatag ttgtctcttt ccttgaagta caattatgat gtcagtgggt aatggaatct 5220

acccggatcc aaatcatttt ttagcgattt gtatcctcaa attttggacc caacggatat 5280

ggatcatgtc ttaaatcatt acatggagtc gtatccattt acccattttt taaaaattat 5340

tttaaaatat ataattaaaa gtgaaaaact taaaggattt tttaactgga ataatacaaa 5400

ctttgggccg ttctctcgca ataatacaaa cttttgatta ttttttaata atacaaactt 5460

taggccaaaa ttcctccagt aggttaatac cggttggtaa cttgttgtag gaggtaacca 5520

agctgacgtg gcacctttag gacacgtgtc tttatttgat tggctgtttt tttaaattta 5580

gggaaaaaaa aaaccaaaat ttttcttttt tttttctttc ttctcctgct ccccccttca 5640

ttctctttct cactactcca ccccaccgac ccccacccct ggcccctgcc cccagcccct 5700

ccctgcctgc acaccgcatc cctccgacga ccaacccccc atctctggcc cttcccccac 5760

cccgaaacca taccgcaccc ctccgacaac cacccctggc ccctccccca cccgaaaacc 5820

acaccgcacc tcttcgacaa ccaaccccca tttctggccc ctcccccacc ccgaaaccac 5880

actgcacccc tccaacggcc atcacctgac gccattctcg atcgctcgaa cttagatcct 5940

ttcccgcaca acaacaagtc gtcgtcgtca ccgcgcgaaa tcaatacgat attcgaaaat 6000

ggcggtcatt ttattatcga cgaattggag gaggaggaag agacgtcgtt gttgacgatt 6060

tcgcatagtt acgcgatctg gggaagtggt ttcgtgtggt ggtgagtttg aggtggtttc 6120

gtggtggggg agtttgaggc ggtttcatgg ttggggacga ggtggtgatg gtggtttcat 6180

ccgtgggggt ggagctgtgg gagggggtgg aactgtggag gtgagggtgg ggggaggggg 6240

tgggggtgga gctgtggagg tgggggaagg gggtgggagt ggagccgtgg aggtggagga 6300

gggggtggga gtgggctttc acattttcag ttggttggct aggggaagga ggaagagatt 6360

ttggagttta aaaaaatcga cctattctac atgttaatgg tcaacttgac ctactggagg 6420

aattttggcc taaagtttgt attattaaaa aataatcaaa agtttgtatt attgcgagag 6480

aacggcccaa agtttgtatt attcgagtta aaaaatccaa acttaaaacc attttatgat 6540

tttatctttc aaattactta tatgtttatt gtgatgaata tttatgttgt cattattatt 6600

ctttgtaatg tacaaatatg attttgcaat gaacaacata ctatcatata cttcctccat 6660

ttctaaataa gtgcaacatt tcttgttttc acatttgcca atgcattatt ttaaccagta 6720

atatcttcaa ttatcaataa gtaaaaatta taaaaagttg atattaaaaa aattcacatt 6780

tagatgaatc taacaagatc ccacatgact atgtttttcc taaaatgcaa atcactaaag 6840

ctaattgatg tttaaactgt gaatagtaaa cactatgcaa atgttacact tatttagaaa 6900

cggaggaagt aataaataac ccaattgaat agttatataa ttatcaaaaa ataagtaatt 6960

acaaaagatg gttaattcat aatgtagtat gtacccaagt gtagaaatat aaccaaaata 7020

atataatata tttctaatta aacactattg agttcattaa gaagatattc ttgcttttga 7080

gcttccttcc atttccgatt gatttgaata gtgagatctc gacaccataa cgtatcttag 7140

ttgtttgaac tagtcgaatt gaactgatac aagaaaggaa atagcagtag ttaaaaaaaa 7200

ttattcaagt ttataataca atgaacataa tttttcaaac atacaattaa cacattctaa 7260

taatacaata aacatgctcc cgcacatagt cccgactatt aggctgtgca aacaacccaa 7320

tagcacagga ccccgaattt cttagggtcc ccaaaatctg gaaacaaaaa aatatagttg 7380

attacggcgc taaaacaatt caactacata tattagcaga gttgttatat gagattaact 7440

ctacaacaag aatacgcaag ttcgactcat gttccgggaa tatctttaca tactttttgc 7500

aagtgtttat gatatttctt tctagtttga gcatttcact catctcccca ccacttcata 7560

cttactttct aactattttc cttacttttt tttcaatact tttttttgat aaattgtttt 7620

tctcaattct ttagggtact tttttgataa attcttttat caattcctta gggtacttat 7680

tttttttgga agtgtatatt tgtccaataa gaattgaata atttcctgcc acaagtttac 7740

tagtttaccg gtttagtcac ctcatcatat atataaattt tttttttttt gttgtacctt 7800

ttagagtgag atagaaaaat tattatcgtt atctttataa gtagaacatc agtcataatg 7860

tggttttata ttgagttgtt acacacaatt tcaaatgagt ttgaggtttt agtttttcga 7920

tttacaagat aaatttatat aattgtaata ttataaaaga ttttttttat catttgagtt 7980

gcaaaaaaat gagattaggg gtccaatcct aaaattggaa caagacctcc aaaatgttta 8040

agacggccct gcccgcacac aatgagcatg tgattaaaat gagcatgtta gtaatacaat 8100

gaacatcaac ctaaaaatgt gcaccttgta tttgtaaatg gtacaataaa tatcacataa 8160

tcatacaaag aacatttgaa caaatatcac acatttatag acaaatatat atgataatca 8220

acccatcatt aattcatatc atttcattga atatattaca aacacataac gaattatgac 8280

tgaaatgcaa taaacatctg accaacatgt gtttcagtga acatattctg attggacaat 8340

gaacatatgc cataattcat gttattaatg caattaatat gtgtcaaaga cataataaat 8400

atatgaagaa ctgtacataa catctgaaca aacacataat aaacatatgg ctaaatgtaa 8460

taaacatatg accaacacaa attcaattag tacattacaa taatacaacg aacatatatt 8520

tacagtacaa cattacctgt ttttttctag atgtcgaact cgttcatctc ctttattcat 8580

tgggctctct catttgttca ttgggacatc aattgcaagg ttgttttcat ttttcacaaa 8640

tatcaagatc ttcactagaa aagatggaaa aaaaaataag aaatgaaagt gcaatatgta 8700

aaaaaaaata ttcttttttc ttatcaaatg catagatcta ataacttatg ttctttgcaa 8760

ctcgttgtat tcatcttcat gtacgaaaat tgtttaaaaa aatgaagaat ttaaaaaggt 8820

tgatgagaaa ggttagagag aagttaaaga gatgttggag gatttgggga gaagagataa 8880

aaagtctttg aaaatttctg gggaaaaatg aaagtaatga attcaaattt ttttatgttt 8940

tttggtgttc attggagatc aacaaatgtt catttcgtgc atttttggtt taatgaatac 9000

gcgttttatg ttttgttgac atccgttttg acccatgcac accttcctca acatacatcc 9060

gttcgcacgg ttgatctact gatcattcgc ttgaaaaggg aataaggtca tgacagcgtt 9120

aagaatgaaa caagaaagat cgtatatttg ttggtaactt ggtactacaa ttagggttgt 9180

tcaccggtcc aggatcggat cgaacctgac ctgcctggac cgttctttat gggattcttc 9240

ggaccaggtg tggatcggaa aaatcaggtc tggtcttcag tccggacctg ttgggccgga 9300

aaaattaact tgtatctctg attcttcgga cctgttacga cccaccatca ataaatatca 9360

gaattccata gatttatcag tttacagagg accaactaca acaagcaaac aaaaaaacaa 9420

ttctagtact tcctccgttt ttttttttac ttgcaacact tttttttcat caactccaat 9480

gtaaaatttg ggtatgagat aggcatacag gaaaaaaaga gagatcccca tgtgattgga 9540

gagagataga gagaaaggaa aagaaaaagt agaaagtgtt gcaagtaatt aattattttc 9600

tttttaggaa agtgttgcaa gtaaaaagac ggaggaagta tttagttttt attacaaatt 9660

gtttgggggg ggggggcagg agacatggag tcacactaga ctaggacact caaagaggca 9720

ccaatgctac tcactaagtg attaaagtta aagatagaaa agggtgaaaa agactcgcga 9780

actgaaaaac aaacctgatc ggtggtgggc ggtagctgcg gtcgcctgag cggcgtcgtg 9840

gcgtcggtgc gactggtttg gcgaggcgtg accgcgtgag gactgaggtg tttgagttct 9900

cttagggttt cttttagaat gactaaatga gagagggaga agtaggaata aagggctggc 9960

atttttttaa aaaaaaaaaa tactaaaaag cttccgagtt ttcggtctgg accggatttt 10020

ttcgagtttg aaccagaccg aaccggaaac ccggaataga cacattccac ggaccgggga 10080

ccggattgga atcgccggtc cggttcgatc cgatccaaaa tccggtccga ttagatccga 10140

cccacctatg tgaacagccc tagctacaat actacatgac tacctgcctt attaactctc 10200

ccttttatta gatttttcgt tagctaataa ctcccttagg atattgaatc tactttgttg 10260

gttataactc ttataagtta ctattatagt aggagaacaa ctattattat gtgcaaaatt 10320

acacagaatt tgtttttctt ccatttttag gggaccactt gctcagctga ttagcttcat 10380

caaagcaatg acagggccaa aatatgatgg caagtattta cgccatatac taagggaaaa 10440

attgaaagag acaaggttgc atcacacact aacaaatgtt gtcattccaa cttttgacat 10500

taagaccttc caaccagtta tattctcttc ctacaaggta aataaattat aatcgtgtgc 10560

tcatctccaa taattttacc ttaaatgaat tttcatcaac taatcatttg ctttgctgat 10620

gaccatcctt gatttgaatg aagagaatat atatatatat atatatatat atatatatat 10680

atatatatat atatatatat attatatata ctatatatat agtacgtagt acgtacgtac 10740

gttacgtacg ttacgtagta cgtacgggta cttaacgtcg tannnnnnnn nnnnnnnnnn 10800

nnagtatagt agtatatata tatatatata tatatatata tatatatata tatatatata 10860

tatatatata tatatatata tatatatata tatataggat gaggaactag ctcatgtggt 10920

agagcactcc cctctgatcc ttgaggtcag gggttcgatc cttaccccca acattatgtt 10980

ggggcatttc cttagcatgc tttgcatgct ttaccagcca atgtgctggt agttcctaca 11040

aggctagtag aatgaatttt acgtgttata actcatatga ataagtgatg taactccaaa 11100

aaaaaaaata ttagaaaaaa gaaattcaag aaaaaaaaaa ttgggatcaa aattttttaa 11160

ggggtataac tctaataaag tattgtaact ctaatccaat catgtggtcg aaattcattc 11220

tcatttctca tttaaagagc attctcatat gatattactc ctatatatat tcgtgcttgt 11280

tttttctttt attagatgat cctagcttga tatcgacgca aactaagaac ttcaaaagga 11340

aataatgaaa ttactatttt aatgttttct ttttgtttta ttccatatat atgtaaacac 11400

agtattatcg atctgatagg cataaatgtt atacttaatg tgattatcca aatagtttaa 11460

ttgagagttt tactcttatc aagttatcat gtcgtctagt gttaataatt ctttagtttt 11520

atgtctattt tgtcgtagct atagtccgac tggtcaagta cttgatcaaa cagtttaagc 11580

ggtgaaccta ttgcatgact tttacctagg caactagtat atgtctggaa aagtgaatga 11640

cgatctctca ttttcatagt gaaatttaca atattctcta agcttttatg atattaatga 11700

taggtgatat atgatgcgag gttattgaag tgtgtggatt ttcctagtgc cctttagtta 11760

cattgttaat ttgttagggt tgattcaagt agatttacag acctatatga gggcataact 11820

aagagaccat attttattga tggaagaaaa gtagagaggg tcactttagc agctactcct 11880

actacgagta gctaagtatt acggatttaa aaaatattcc tcccaatgat agaaattaag 11940

gtcgaaataa aacaacattt taagataaca catatttata aaaacaaaac tagtgaaaaa 12000

taacctagac aaattcgaat cttatttgca tcgacaaact ccaacatttt catcattatt 12060

ttcaaaaaca tatcaaaaca ttgaaaatgc cagatcttct gtccgccccc tccctttgac 12120

caaaatatga atttatacgt aagtagtttt accttcgtaa ttttacattt gtgattttgt 12180

agacttcaat taacattttc atttgtgttc aattaaactt tgcgacttag tcaacggatt 12240

aattacatcc ttctttatgc ataaaaaagt ccatttatca atcaacccaa gaagactcac 12300

aatgttttta tcatttaatt tttgcaaatt ctaagttatc atttaatttt tacagattct 12360

aagttatcct gatctggatg cgaaattatc agatatatgt ataggcacat cggccgctcc 12420

taccattctg ccttctttct atttccaaaa tgcatttgaa aacgaaaaga caagggaatt 12480

caacctcatt gatggtggaa ttgtgagtac aaatccggta agtcaagctc aatcaatcat 12540

attattattc ttatgcttag aggatgcaca atagcatggt ttcacttggg gcaaaggtga 12600

accaatgggt gaggttggtc ttaccattgg ggtgagtcaa ggcaaacatg gcttaatgag 12660

gttgggataa tttgctcccc ctctctccca acccatgcat cggaccaaga gcccaagcta 12720

gtaacaaaag gcttgtgtac tttcacagag aatgagcttc atgtctttga gtgtagagct 12780

tttttccttc ccaaacgtcc tactcttaga attgttattt acataaccta ctttaattat 12840

ttttttttat taatttataa aatagttgtt atgttattac atagtaagtt gtcgtatcag 12900

agttcttttc acgaaaactc aaagctgaaa cataaaaaca gacgagttta ttcatcatac 12960

cacctagata taccacaatg ttagtacatt ttgttaattt ggaactatcc aaatgaacaa 13020

taagcagaaa aatagtcttg agtgtaatgt ctacaatgtt cacaatacat tcaagtgtat 13080

gttgaatttt ttgtataaga gcctaaaaaa cttttatcca acaatacttt aaaccagcag 13140

atagaatatt atttgatgta tcaataattt cattctatct ttttgttttg tcgtctgggc 13200

agacttatct ggcaataaat gaagttacca aacaaatgat gaaggagaat ccagattacg 13260

ggaccatcca taataagttg ctggttttat cgataggaac aggatcagga aaaattgagc 13320

agaaatacaa tgctaaaact gcaggaaagt gggggcttgt ctcttggctt tttcaaaatg 13380

gatcatcacc aattataagt gctttttatg aagccggggc tgacttagtt gattaccaaa 13440

ataatatcct cttccaatcg tttcgttcag aggacaagta ccttcgtgtg caagtaagtc 13500

gcatgatacc cttcaaattt aactttaatg tagtaatccg tttacgatta atcggcacat 13560

attgttggaa ataaaacagg atgacagctt aacaggaaca actgcttcaa cagatgtagc 13620

aacagagaag aacttggaaa atctagtgaa aattggtcaa gaattgctta acaaaccagc 13680

cagcagggtg gacccagaaa ctgggcatct gaaagcaatt ccacaccttg gtaccaatgc 13740

agatgctctt cgaaggttgg ttcataaatt ttataatatt gccaatataa attgcacatt 13800

attagttatt actactagat agattaacaa gtcattaaca aataggttcg caaaacaact 13860

ctcggatgaa agaaaatacc gcaggggcaa agactccaat cagatgcaag agtacagctg 13920

agctgacgat gtgcaggcat ttctgagtac tgagctgtct ttgattgtat actatatgat 13980

aaatgtgtgc aatgtaattg acctcaaatc ttctatctca taatttttgt actattctgt 14040

tggttgatta taaatttgta acgttctaaa gattatctta aaaggtacta gaccgtagta 14100

caatacctgc taataatata tagtagcata cattcttttt ttggctccct tccgggaatc 14160

cgtggtaaga tgaatttcct ttagactgtc ctcacacagg gccactcacc cagaagacca 14220

acaacccccc tccccccaac tatattgaaa tttttgcttg caccaagatt tgaaccttgg 14280

cttcccatat gtgagacacg aaaccatacc attggaccca agggtccttg gtatataaag 14340

tagcatacat gtgacacaca aagagagaca gtagatgttg cttcaggtaa tgcagaaacc 14400

ccctatatga tcaacatgat caacagaact atgtatatct atacttcttt ccctagataa 14460

gcaagaattt atggaggcta actatccttg tttccctgta atgcttatac aagaagatgc 14520

aaaatagaaa gctcagagcc agatactctc ttaatagacc caggagtaac ctgaacttgc 14580

taccatattc aacacattta agccagaagg taaaacacag aagttcgaaa gtaaagacag 14640

caagttcaca aagctaggga tctttttgaa gtgcaaccat cggacattat gggattgcta 14700

a 14701

<210> 26
<211> 1215
<212> DNA
<213> Artificial Sequence

<220>
<223> cDNA of Beta vulgaris phospholipase 2

<400> 26
atgacgaact tacaaagctc tcctccatcc ttcaattact acaacaggaa gaaattggtc 60

acaatcctta gcatcgacgg tggtggcatt cgaggcatta ttcctgctgt cattcttgct 120

tttcttgagc gacttcttca ggagttggat ggagaggaag taagactagc agattatttt 180

gatgtgatag caggaacaag cacgggaggg ttgattacag ccatgttaac tgcaccagat 240

gactataatc gtcctctata tgctgcgaaa gatatcacaa cattttactt gaagcatggt 300

cctcaaatat ttcctcagga aaggggacca cttgctcagc tgattagctt catcaaagca 360

atgacagggc caaaatatga tggcaagtat ttacgccata tactaaggga aaaattgaaa 420

gagacaaggt tgcatcacac actaacaaat gttgtcattc caacttttga cattaagacc 480

ttccaaccag ttatattctc ttcctacaag attctaagtt atcctgatct ggatgcgaaa 540

ttatcagata tatgtatagg cacatcggcc gctcctacca ttctgccttc tttctatttc 600

caaaatgcat ttgaaaacga aaagacaagg gaattcaacc tcattgatgg tggaattgtg 660

agtacaaatc cgacttatct ggcaataaat gaagttacca aacaaatgat gaaggagaat 720

ccagattacg ggaccatcca taataagttg ctggttttat cgataggaac aggatcagga 780

aaaattgagc agaaatacaa tgctaaaact gcaggaaagt gggggcttgt ctcttggctt 840

tttcaaaatg gatcatcacc aattataagt gctttttatg aagccggggc tgacttagtt 900

gattaccaaa ataatatcct cttccaatcg tttcgttcag aggacaagta ccttcgtgtg 960

caagatgaca gcttaacagg aacaactgct tcaacagatg tagcaacaga gaagaacttg 1020

gaaaatctag tgaaaattgg tcaagaattg cttaacaaac cagccagcag ggtggaccca 1080

gaaactgggc atctgaaagc aattccacac cttggtacca atgcagatgc tcttcgaagg 1140

ttcgcaaaac aactctcgga tgaaagaaaa taccgcaggg gcaaagactc caatcagatg 1200

caagagtaca gctga 1215

<210> 27
<211> 404
<212> PRT
<213> Beta vulgaris

<400> 27

Met Thr Asn Leu Gln Ser Ser Pro Pro Ser Phe Asn Tyr Tyr Asn Arg
1               5                   10                  15

Lys Lys Leu Val Thr Ile Leu Ser Ile Asp Gly Gly Gly Ile Arg Gly
            20                  25                  30

Ile Ile Pro Ala Val Ile Leu Ala Phe Leu Glu Arg Leu Leu Gln Glu
        35                  40                  45

Leu Asp Gly Glu Glu Val Arg Leu Ala Asp Tyr Phe Asp Val Ile Ala
    50                  55                  60

Gly Thr Ser Thr Gly Gly Leu Ile Thr Ala Met Leu Thr Ala Pro Asp
65                  70                  75                  80

Asp Tyr Asn Arg Pro Leu Tyr Ala Ala Lys Asp Ile Thr Thr Phe Tyr
                85                  90                  95

Leu Lys His Gly Pro Gln Ile Phe Pro Gln Glu Arg Gly Pro Leu Ala
            100                 105                 110

Gln Leu Ile Ser Phe Ile Lys Ala Met Thr Gly Pro Lys Tyr Asp Gly
        115                 120                 125

Lys Tyr Leu Arg His Ile Leu Arg Glu Lys Leu Lys Glu Thr Arg Leu
    130                 135                 140

His His Thr Leu Thr Asn Val Val Ile Pro Thr Phe Asp Ile Lys Thr
145                 150                 155                 160

Phe Gln Pro Val Ile Phe Ser Ser Tyr Lys Ile Leu Ser Tyr Pro Asp
                165                 170                 175

Leu Asp Ala Lys Leu Ser Asp Ile Cys Ile Gly Thr Ser Ala Ala Pro
            180                 185                 190

Thr Ile Leu Pro Ser Phe Tyr Phe Gln Asn Ala Phe Glu Asn Glu Lys
        195                 200                 205

Thr Arg Glu Phe Asn Leu Ile Asp Gly Gly Ile Val Ser Thr Asn Pro
    210                 215                 220

Thr Tyr Leu Ala Ile Asn Glu Val Thr Lys Gln Met Met Lys Glu Asn
225                 230                 235                 240

Pro Asp Tyr Gly Thr Ile His Asn Lys Leu Leu Val Leu Ser Ile Gly
                245                 250                 255

Thr Gly Ser Gly Lys Ile Glu Gln Lys Tyr Asn Ala Lys Thr Ala Gly
            260                 265                 270

Lys Trp Gly Leu Val Ser Trp Leu Phe Gln Asn Gly Ser Ser Pro Ile
        275                 280                 285

Ile Ser Ala Phe Tyr Glu Ala Gly Ala Asp Leu Val Asp Tyr Gln Asn
    290                 295                 300

Asn Ile Leu Phe Gln Ser Phe Arg Ser Glu Asp Lys Tyr Leu Arg Val
305                 310                 315                 320

Gln Asp Asp Ser Leu Thr Gly Thr Thr Ala Ser Thr Asp Val Ala Thr
                325                 330                 335

Glu Lys Asn Leu Glu Asn Leu Val Lys Ile Gly Gln Glu Leu Leu Asn
            340                 345                 350

Lys Pro Ala Ser Arg Val Asp Pro Glu Thr Gly His Leu Lys Ala Ile
        355                 360                 365

Pro His Leu Gly Thr Asn Ala Asp Ala Leu Arg Arg Phe Ala Lys Gln
    370                 375                 380

Leu Ser Asp Glu Arg Lys Tyr Arg Arg Gly Lys Asp Ser Asn Gln Met
385                 390                 395                 400

Gln Glu Tyr Ser

<210>  28
<211>  40
<212>  DNA
<213>  Artificial Sequence

<220>
<223>  R59Q mutant - primer AlleleX

<400>  28
gaaggtgacc aagttcatgc tccgtccagc tcctgcagct                              40

<210>  29
<211>  39
<212>  DNA
<213>  Artificial Sequence

<220>
<223>  R59Q mutant - primer alleleY

<400>  29
gaaggtcgga gtcaacggat tcgtccagct cctgcagcc                               39

<210>  30
<211>  22
<212>  DNA
<213>  Artificial Sequence

<220>
<223>  R59Q mutant - primer common

<400>  30
tccccggaac catccttgcc tt                                                 22

<210>  31
<211>  201
<212>  DNA
<213>  Artificial Sequence

<220>
<223>  partial sequence indicating detectable single nucleotide
       polymorphism that causes amino acid substitution R59Q in Sorghum
       bicolor phospholipase

<400>  31
gagcccgtcg tgctggggca gagggtgacg gtgctgacgg tggacggcgg cggcatccgt        60

ggtctcatcc ccggaaccat ccttgccttc ctcgaggccc rgctgcagga gctggacggg       120

ccggaggtta ggctcgcgga ctacttcgac tacatcgccg ggacgagcac cggcgggctc 180

atcaccgcca tgctcaccgc g 201

<210> 32
<211> 41
<212> DNA
<213> Artificial Sequence

<220>
<223> V162I mutant - primer AlleleX

<400> 32
gaaggtgacc aagttcatgc tgagcgacac gctcaccaac a 41

<210> 33
<211> 41
<212> DNA
<213> Artificial Sequence

<220>
<223> V162I mutant - primer AlleleY

<400> 33
gaaggtcgga gtcaacggat tgagcgacac gctcaccaac g 41

<210> 34
<211> 21
<212> DNA
<213> Artificial Sequence

<220>
<223> V162I mutant - primer common

<400> 34
ctgcagcagc ctgacgtcga a 21

<210> 35
<211> 201
<212> DNA
<213> Artificial Sequence

<220>
<223> partial sequence indicating detectable single nucleotide
      polymorphism that causes amino acid substitution V162I in Sorghum
      bicolor phospholipase

<400> 35
catgtccgcg ctgaggaagc caaggtacaa cggcaagtgc ctccgtaacc tgatcatgag 60

catgctcggc gagacgaggg tgagcgacac gctcaccaac rtcatcatcc ctaccttcga 120

cgtcaggctg ctgcagccca tcatcttctc cacctacgac gtacgtacgc cgccggccgc 180

cgtcgtcatg aataatcaat c 201

<210> 36

<211>  39
<212>  DNA
<213>  Artificial Sequence

<220>
<223>  S291L mutant - primer AlleleX

<400>  36
gaaggtgacc aagttcatgc tggcagatgc cccaccgcg                              39


<210>  37
<211>  39
<212>  DNA
<213>  Artificial Sequence

<220>
<223>  S291L mutant - primer AlleleY

<400>  37
gaaggtcgga gtcaacggat tggcagatgc cccaccgca                              39


<210>  38
<211>  19
<212>  DNA
<213>  Artificial Sequence

<220>
<223>  S291L mutant - primer common

<400>  38
gcctgtacac ggcgcggca                                                    19


<210>  39
<211>  201
<212>  DNA
<213>  Artificial Sequence

<220>
<223>  partial sequence indicating detectable single nucleotide
       polymorphism that causes amino acid substitution S291L in Sorghum
       bicolor phospholipase

<400>  39
ctgtacccag tgaagccgtg gaactgccgc aagttcctgg tgctgtccat cgggacgggg      60

tcgacgtcgg agcagggcct gtacacggcg cggcagtgct ygcggtgggg catctgccgg     120

tggatccgga acaacggcat ggcccccatc atcgacatct tcatggcggc gagctcggac     180

ctggtggaca tccacgtcgc c                                               201


<210>  40
<211>  39
<212>  DNA
<213>  Artificial Sequence

<220>
<223>  Q372stop mutant - primer AlleleX

<400>  40
gaaggtgacc aagttcatgc tgaccctgga cacccgctg                              39


<210>  41
<211>  42
<212>  DNA
<213>  Artificial Sequence

<220>
<223>  Q372stop mutant - primer AlleleY

<400>  41
gaaggtcgga gtcaacggat tgttgaccct ggacacccgc ta                          42


<210>  42
<211>  20
<212>  DNA
<213>  Artificial Sequence

<220>
<223>  Q372stop mutant - primer common

<400>  42
gtcgggatcg gggagcggat                                                   20


<210>  43
<211>  197
<212>  DNA
<213>  Artificial Sequence

<220>
<223>  partial sequence indicating detectable single nucleotide
       polymorphism that causes amino acid substitution Q372stop in
       Sorghum bicolor phospholipase

<400>  43
gcatccagga caactcgctg cacggcgccg cggccaccgt ggacgcggcg acgccggaga       60

acatgcggac gctcgtcggg atcggggagc ggatgctggc gyagcgggtg tccagggtca      120

acgtggagac agggaggtac gaaccggtgc ctggggaagg aagcaacgct gatgcgctcg      180

ctgggatcgc aaggcag                                                     197


<210>  44
<211>  19
<212>  DNA
<213>  Artificial Sequence

<220>
<223>  Forward Primer

<400>  44
ggcccccatc atcgacatc                                                    19


<210>  45
<211>  24
<212>  DNA

<213>  Artificial Sequence

<220>
<223>  Reverse Primer

<400>  45
aatcaggcaa caaattcact tctc                                              24

## Beschreibung

### GEBIET DER ERFINDUNG

Die vorliegende Erfindung betrifft das Gebiet der Vereinfachung arbeitsaufwendiger Züchtungsprogramme mittels molekularbiologischer Methoden, Markertechnologie sowie der Gentechnik. Insbesondere werden Sorghumhirsepflanzen zur Verfügung gestellt, die durch Modifikationen im Genom, die eine vorzugsweise pollenspezifisch exprimierte Patatin-Phospholipase betreffen, in der Lage sind Haploidie zu induzieren, wodurch haploide Nachkommen erzeugt und in kurzer Zeit durch Chromosomenverdopplung Inzuchtlinien für die Hybridzüchtung hergestellt werden können. Im Speziellen werden Sorghumhirsepflanzen zur Verfügung gestellt, die Mutationen in der Patatin-Phospholipase aufweisen, Verfahren zur Herstellung und Identifizierung dieser Mutationen bzw. der mutierten Pflanze sowie das entsprechende Nukleinsäuremolekül, welches die mutierte Patatin-Phospholipase kodiert sowie Vektoren und Wirtszellen insbesondere Pflanzenzellen enthaltend das Nukleinsäuremolekül, und aus solchen Pflanzenzellen generierte Pflanzen, die in der Lage sind Haploidie zu erzeugen sowie deren Nachkommen, Kreuzungsprodukte, Inzuchtlinien und jeweils deren Pflanzenteile und -produkte.

Basierend auf den Erkenntnissen aus Sorghum stellt die vorliegende Erfindung Verfahren zur Herstellung und Identifizierung von transgenen und nicht-transgenen pflanzlichen Haploiden-Induktoren bereit sowie die entsprechenden Pflanzen, die die Eigenschaft der Haploidie-Induktion erhalten haben oder dessen Induktionsleistung verbessert wurde. Ferner sind von der Erfindung auch Samen oder Nachkommen, Organe, Pflanzenteile, Gewebe oder Zellen der erfindungsgemäßen Pflanze sowie deren Verwendung miterfasst.

### HINTERGRUND DER ERFINDUNG

Sorghum ist eine in Deutschland recht neue Kultur, gerät jedoch aufgrund ihrer Massenwüchsigkeit, Trockentoleranz sowie gute Wasser- und Nährstoffeffizienz in den Fokus der Wissenschaft und Praxis bei der Suche nach weiteren hochertragsreichen Kulturen zur Substratproduktion, insbesondere im Hinblick auf die Verwendung in der Biogasproduktion, aber auch zur Verwendung als Futtermittel, Nahrungsmittel und zur Ethanol-Produktion.

Allgemeine Zuchtziele für Sorghum sind die Anpassung an das Klima Mitteleuropas, d.h. eine verbesserte Kältetoleranz, die Verbesserung des Ertrags, der Standfestigkeit gepaart mit guter Jugendentwicklung sowie Entwicklung von Krankheits- und Schädlingsresistenzen.

Sorghum kann wie eine selbstbefruchtende Kultur züchterisch verbessert werden. Die Verbesserung der Population kann durch die aufwendige Selektionszüchtung durch eine gezielte Auswahl der besten Pflanzen zur Saatgutproduktion für das nächste Anbaujahr erreicht werden. Daneben wurden mittels der Hybridzüchtung neue Möglichkeiten zur Verbesserung der Sorten geschaffen. Allerdings beruht der Prozess der Hybridzüchtung auf eine mehrere Generationen dauernde Erzeugung von reinerbigen Vater- und Mutterlinien, wodurch auch der Prozess der Hybridzüchtung trotz guter Erfolge zeit- und kostenintensiv ist.

Aufgabe der vorliegenden Erfindung war es daher ein effizientes System zur Züchtung von Sorghum bereitzustellen.

### ZUSAMMENFASSUNG DER ERFINDUNG

Die vorliegende Erfindung betrifft das Gebiet der Vereinfachung von arbeitsaufwendigen Züchtungsprogrammen, der Markertechnologie sowie der Gentechnik. Die Erfindung stellt Sorghumhirsepflanzen zur Verfügung, die durch Modifikationen im Genom, die eine pollenspezifisch exprimierte Patatin-Phospholipase betreffen, in der Lage sind Haploidie zu induzieren, wodurch haploide Nachkommen erzeugt und in kurzer Zeit durch Chromosomenverdopplung Inzuchtlinien, d.h. reinerbige Vater- und Mutterlinien, für die Hybridzüchtung hergestellt werden können. Zusätzlich können die Erkenntnisse dazu verwendet werden, transgene sowie nicht-transgene pflanzliche Haploiden-Induktoren herzustellen oder die Induktionsleistung von Pflanzen zu verbessern.

Die vorliegende Erfindung betrifft daher die Ausführungsformen, die in den folgenden Punkten [1] bis [29] aufgeführt und in den Beispielen illustriert sind.
[1] Sorghumhirsepflanze, welche in der Lage ist Haploidie zu induzieren, dadurch gekennzeichnet, dass die Pflanze eine oder mehrere Modifikationen aufweist, die eine endogene Patatin-Phospholipase betreffen, welche vorzugsweise pollenspezifisch exprimiert wird.
[2] Pflanze nach [1], dadurch gekennzeichnet, dass die Patatin-Phospholipase durch die Nukleotidsequenz gemäß SEQ ID Nr.: 1 oder 2 oder durch eine Nukleotidsequenz, die zu mindestens 80% identisch ist zu SEQ ID Nr.: 1 oder 2 kodiert wird, oder durch eine Nukleotidsequenz kodiert wird, die mit der komplementären Sequenz zu der Nukleotidsequenz gemäß SEQ ID Nr.: 1 oder 2 unter stringenten Bedingungen hybridisiert, oder die die in SEQ ID Nr.: 3 gezeigte Aminosäuresequenz oder eine homologe Aminosäuresequenz umfasst.
[3] Pflanze nach [1] oder [2], wobei die Modifikation der Patatin-Phospholipase ursächlich für die Eignung als Haploideninduktor ist.
[4] Pflanze nach einem von [1] bis [3], dadurch gekennzeichnet, dass die eine oder mehrere Modifikationen
   a) eine oder mehrere Mutationen in dem endogenen Gen kodierend die Patatin-Phospholipase definiert in [2], vorzugsweise Substitutionen sind, welche zu einem oder mehreren Aminosäureaustauschen oder Erzeugen eines Stopp-Codons führen;
   b) eine oder mehrere Insertionen einer Expressionskassette sind, welche einen Promotor operativ verknüpft mit
      (i) einem Nukleinsäuremolekül kodierend dsRNA umfasst, wobei die dsRNA mindestens 19 Nukleotide umfasst, die komplementär zu einer Teilsequenz der Nukleotidsequenz definiert in [2] ist; oder
      (ii) ein Nukleinsäuremolekül kodierend die Patatin-Phospholipase definiert in [2] oder kodierend einen funktionellen Teil der Patatin-Phospholipase umfasst, wobei die kodierte Patatin-Phospholipase oder der funktionellen Teil davon eine oder mehrere Mutationen aufweist, welche zu einem oder mehreren Aminosäureaustauschen oder Erzeugen eines Stopp-Codons führen; oder
   c) einen Knock-out der Patatin-Phospholipase bewirkt.
[5] Pflanze nach [4], dadurch gekennzeichnet, dass die eine oder mehrere Mutationen zu einem Aminosäureaustausch
   a) in dem Bereich der Aminosäurepositionen 37 bis 240 gemäß SEQ ID Nr.: 3 führen, vorzugsweise wobei dieser Bereich der funktionellen Domäne der Patatin-Phospholipase entspricht; und/oder
   b) in dem Bereich der Aminosäurepositionen 241 bis 385 gemäß SEQ ID Nr.: 3 führen, oder
   c) zu einem Erzeugen eines Stopp-Codons in dem Bereich der Aminosäurepositionen 241 bis 385 gemäß SEQ ID Nr.: 3 führen.
[6] Pflanze nach [4] oder [5], dadurch gekennzeichnet, dass die eine oder mehrere Mutationen zu einem Aminosäureaustausch in dem Bereich der Aminosäurepositionen 40-93, 135-204 und/oder 270-320 gemäß SEQ ID Nr.: 3, bevorzugt in dem Bereich der Aminosäurepositionen 53-85, 150-192 und/oder 285-311 gemäß SEQ ID Nr.: 3, oder besonders bevorzugt in dem Bereich der Aminosäurepositionen 55-75, 157-167 und/oder 285-298 gemäß SEQ ID Nr.: 3, oder Erzeugen eines Stopp-Codons in dem Bereich der Aminosäurepositionen 322-402 gemäß SEQ ID Nr.: 3, bevorzugt in dem Bereich der Aminosäurepositionen 342-392 gemäß SEQ ID Nr.: 3, oder besonders bevorzugt in dem Bereich der Aminosäurepositionen 362-382 gemäß SEQ ID Nr.: 3, führen.
[7] Pflanze nach einem von [4] bis [6], dadurch gekennzeichnet, dass die eine oder mehrere Mutationen zu einem Aminosäureaustausch an der Aminosäureposition 59, 162 und/oder 291 gemäß SEQ ID Nr.: 3, und/oder zu einem Stopp-Codon an der Aminosäureposition 372 gemäß SEQ ID Nr.: 3 führen.
[8] Pflanze nach einem von [1] bis [7], dadurch gekennzeichnet, dass die modifizierte Patatin-Phospholipase
   (i) eine Aminosäuresequenz gemäß SEQ ID Nr.: 3 oder eine homologe Aminosäuresequenz umfasst, in der mindestens ein Aminosäureaustausch vorhanden ist, wobei an der Position 59 Arginin (R), an der Position 162 Valin (V), und/oder an der Position 291 Serin (S) gemäß SEQ ID Nr.: 3 durch eine andere Aminosäure ausgetauscht ist, vorzugsweise durch Glutamin (Q) an Position 59, Isoleucin (I) an Position 162 und/oder Leucin (L) an Position 291;
   (ii) durch eine Nukleotidsequenz kodiert wird, die die kodierende Sequenz der DNA-Sequenz gemäß SEQ ID Nr.: 1 (ableitbar von der zugehörigen cDNA gemäß SEQ ID Nr.: 2) oder einer DNA-Sequenz, die zu mindestens 80% identisch ist zu SEQ ID Nr.: 1, umfasst, in der mindestens ein Nukleotidaustausch vorhanden ist, der zu einem Aminosäureaustauch führt, wobei ein oder mehrere Nukleotide an den Nukleotid-Positionen 421-423, 815-817, 1420-1422 und/oder 1663-1665 gemäß SEQ ID Nr.: 1 (entsprechend der Nukleotid-Positionen 175-177, 484-486, 871-873 und/oder 1114-1116 der SEQ ID Nr.: 2) ausgetauscht sind;
   (iii) eine Aminosäuresequenz gemäß SEQ ID Nr.: 6, 9 oder 12 umfasst; oder
   (iv) durch eine Nukleotidsequenz kodiert wird, die die kodierende Sequenz der DNA-Sequenz gemäß SEQ ID Nr.: 4 (ableitbar von der zugehörigen cDNA gemäß SEQ ID Nr.: 5), SEQ ID Nr.: 7 (ableitbar von der zugehörigen cDNA gemäß SEQ ID Nr.: 8), SEQ ID Nr.: 10 (ableitbar von der zugehörigen cDNA gemäß SEQ ID Nr.: 11) oder SEQ ID Nr.: 13 (ableitbar von der zugehörigen cDNA gemäß SEQ ID Nr.: 14) umfasst.
[9] Pflanze nach einem von [1] bis [8], dadurch gekennzeichnet, dass die Pflanze homozygot oder heterozygot für die eine oder mehrere Mutationen ist, vorzugsweise dadurch gekennzeichnet, dass die Pflanze homozygot für die eine oder mehrere Mutationen ist.
[10] Nukleinsäuremolekül, welches eine der in von [4] bis [8] gekennzeichnete mutierte Patatin-Phospholipase kodiert.
[11] Nukleinsäuremolekül nach [10], dadurch gekennzeichnet, dass dessen Vorhandensein in einer Pflanze, vorzugsweise in Abwesenheit einer Wild-Typ Patatin-Phospholipase, dazu führt, dass die Pflanze in der Lage ist Haploidie zu induzieren.
[12] Nukleinsäuremolekül von mindestens 15, 16, 17, 18, 19 oder 20, bevorzugt mindestens 21, 22, 23, 24 oder 25, besonders bevorzugt mindestens 30, 35, 40, 45 oder 50, und insbesondere bevorzugt mindestens 100, 200, 300, 500 oder 1000 Nukleotiden Länge, das spezifisch an eine wie in eine von [5] bis [8] definierte Nukleotidsequenz hybridisiert, und eine der Mutationen umfasst oder ein Paar von Nukleinsäuremolekülen, das geeignet ist einen Bereich, der mindestens eine der Mutationen enthält, in einer Polymerase-Kettenreaktion (PCR) zu amplifizieren, vorzugsweise in Form eines Oligonukleotids, vorzugsweise mit einer Länge von maximal 50 Nukleotiden, das vorzugsweise eine der Nukleotidsequenzen gemäß SEQ ID Nr.: 28-30, 32-34, 36-38 oder 40-42 aufweist.
[13] Vektor, vorzugsweise Pflanzenvektor umfassend ein Nukleinsäuremolekül nach einem von [10] bis [12], oder eine Expressionskassette definiert in [4].
[14] Wirtszelle, vorzugsweise Pflanzenzelle enthaltend ein Nukleinsäuremolekül nach einem von [10] bis [12], eine Expressionskassette definiert in [4], einen Vektor nach [13] oder das Nukleinsäuremolekül nach einem von [10] bis [12] als Transgen, optional unter der Kontrolle eines heterologen Promotors, vorzugsweise eines Pollen-spezifischen Promotors.
[15] Verfahren zum Erhalt einer Pflanze welche in der Lage ist Haploidie zu induzieren bzw. mit einer erhöhten Induktionsrate gegenüber dem Wild-Typ, umfassend die folgenden Schritte:
   (a)
      (i) Mutagenisieren von Pflanzenzellen und anschließendem Regenerieren von Pflanzen aus den mutagenisierten Pflanzenzellen oder Mutagenisieren von Pflanzen;
      (ii) Identifizieren einer Pflanze aus (i), die eine oder mehrere Mutationen in einer endogenen DNA-Sequenz aufweist, welche der einen oder mehreren der in [4] bis [8] gekennzeichneten Mutationen entsprechen und/oder welche dazu führen, dass an der Position 75 Asparaginsäure (D), an der Position 79 Glycin (G), und/oder an der Position 203 Prolin (P) der Aminosäuresequenz gemäß SEQ ID Nr. 3 durch eine andere Aminosäure ausgetauscht wird, vorzugsweise durch Asparagin (N) an Position 75, Arginin (R) an Position 79 und/oder Leucin (L) an Position 203 oder welche diesen entsprechen, und in der Lage ist, den Erhalt haploider Nachkommen mit einer erhöhten Rate im Vergleich mit einer nicht-mutagenisierten Pflanze zu induzieren; oder
   (b)
      (i) Einbringen des Nukleinsäuremoleküls, das eine oder mehrere Mutationen aufweist, welche der einen oder mehreren der in [4] bis [8] gekennzeichneten Mutationen entsprechen und/oder welche dazu führen, dass an der Position 75 Asparaginsäure (D), an der Position 79 Glycin (G), und/oder an der Position 203 Prolin (P) der Aminosäuresequenz gemäß SEQ ID Nr. 3 durch eine andere Aminosäure ausgetauscht wird, vorzugsweise durch Asparagin (N) an Position 75, Arginin (R) an Position 79 und/oder Leucin (L) an Position 203 oder welche diesen entsprechen, in Pflanzenzellen, oder Einbringen der in [4] definierten Expressionskassette in Pflanzenzellen; und
      (ii) Regenerieren transgener Pflanzen aus den Pflanzenzellen aus (i).
[16] Verfahren nach [15], wobei es sich bei der endogenen DNA-Sequenz bzw. dem Nukleinsäuremolekül um eine kodierende Nukleotidsequenz handelt, welche die in SEQ ID Nr.: 3 für Sorghumhirse (Sorghum bicolor), SEQ ID Nr.: 18 für Sonnenblume (Helianthus annuus), SEQ ID Nr.: 21 für Gerste (Hordeum vulgare) oder in SEQ ID Nr.: 24 oder 27 für Beta vulgaris (z.B. Zuckerrübe) gezeigten Aminosäuresequenzen umfasst.
[17] Verfahren nach [16], wobei das Einbringen des Nukleinsäuremoleküls beispielweise durch Agrobacterium-Transformation, homologe Rekombination beispielsweise mittels CRISPR/Cas bzw. CRISPR/Cpf1 und Repairtemplate durchgeführt werden kann und das Mutagenisieren chemische und physikalische Mutagenese, TILLING, gezielte Mutagenese beispielweise durch Einsatz von Zinkfinger-Nukleasen, von TALE- (*Transcription Activator-like Effector*) Nukleasen, Meganukleasen und des CRISPR/Cas bzw. CRISPR/Cpf1 Systems umfasst.
[18] Pflanze enthaltend Pflanzenzelle nach [14] und/oder erhältlich durch ein Verfahren nach einem von [15] bis [17], vorzugsweise wobei es sich bei der Pflanze um Sorghumhirse, Sonnenblume, Roggen, Weizen, Kartoffel, Gerste oder Zuckerrübe handelt.
[19] Organ, Pflanzenteil, Gewebe oder Zelle der Pflanze nach einem von [1] bis [9] oder [18] oder Samen oder Nachkommen der Pflanze nach einem von [1] bis [9] oder [18], wobei der Samen oder die Nachkommen die in einem von [4] bis [8] definierte Mutation aufweist und/oder ein Nukleinsäuremolekül nach einem von [10] bis [12], eine Expressionskassette nach [4] oder einen Vektor nach [13].
[20] Verfahren zum Erhalt einer haploiden Pflanze, umfassend die folgenden Schritte:
   (a) Kreuzen einer Pflanze gemäß einem von [1] bis [9] oder [18] mit einer Pflanze derselben Gattung, vorzugsweise derselben Spezies,
   (b) Selektieren eines befruchteten haploiden Samens oder Embryos, und
   (c) Erzeugen einer haploiden Pflanze aus dem Samen oder Embryo aus (b).
[21] Haploide Pflanze, haploider befruchteter Samen oder Embryo erhältlich durch das Verfahren nach [20].
[22] Organ, Pflanzenteil, Gewebe, Zelle, Samen oder Nachkommen der Pflanze nach [21].
[23] Verfahren zum Erhalt diploider Pflanzen, umfassend die folgenden Schritte:
   (a) Herstellen einer haploiden Pflanze gemäß eines Verfahrens nach [20];
   (b) Verdoppeln des haploiden Chromosomensatzes in mindestens einer Zelle der haploiden Pflanze, und
   (c) Regenerieren der diploiden Pflanze aus der Zelle aus (b).
[24] Diploide Pflanze erhältlich durch das Verfahren nach [23].
[25] Verfahren zur Herstellung von hybriden Pflanzen umfassend die folgenden Schritte:
   (a) Kreuzen einer Pflanze gemäß [24] mit einer zweiten Pflanze derselben Gattung, vorzugsweise derselben Spezies,
   (b) Selektieren der hybriden Pflanzen hinsichtlich des gewünschten Merkmals.
[26] Hybride Pflanze erhältlich durch das Verfahren nach [25].
[27] Verfahren zur Identifizierung einer Pflanze nach einem von [1] bis [9] oder [18] durch Nachweis einer Mutation in dem Patatin-Phospholipase Gens, beispielsweise eine Mutation definiert in [4] bis [9], oder eines Markerallels, das mit der Mutation gekoppelt ist, vorzugsweise unter Verwendung von Nukleinsäure nach [12] als molekulare Marker.
[28] Verwendung der Nukleinsäure nach [10] oder [11], der Expressionskassette definiert in [4] oder des Vektors nach [13] in einer Pflanze zur Vermittlung der Eigenschaft eines Haploideninduktors oder zur Erhöhung der Induktionsrate zur Herstellung einer Pflanze oder transgenen Pflanze, welche in der Lage ist Haploidie zu induzieren.
[29] Verwendung der Pflanze nach einem [1] bis [9] oder [18] zur Herstellung eines haploiden befruchteten Samens oder Embryos oder einer haploiden Pflanze.
[30] Verwendung der Nukleinsäure nach [12] als molekularer Marker zum Nachweis einer Mutation in dem Patatin-Phospholipase Gens.

Zunächst werden einige der in dieser Anmeldung verwendeten Begriffe nachfolgend näher erläutert:

"Die Eigenschaft eines Haploideninduktors vermitteln" oder das "Vermitteln der Eigenschaft eines Haploideninduktors" oder "in der Lage sein Haploidie zu induzieren" ein vergleichbarer Ausdruck bedeutet, dass eine Pflanze durch Verwendung einer erfindungsgemäßen Nukleinsäure bzw. durch Modifizierung des Genoms, im Speziellen durch Mutation einer Patatin-Phospholipase, in die Lage versetzt wird, befruchtete Samen oder Embryos, welche einen einfachen (haploiden) Chromosomensatz aufweisen, aus einer Kreuzung mit einer Pflanze derselben Gattung, vorzugsweise derselben Spezies, welche nicht die Eigenschaft eines Haploideninduktors aufweist, hervorzubringen. Die Eigenschaft eines Haploideninduktors, angegeben als absolute Haploiden-Induktionsrate, meint, dass mindestens 0,1%, 0,2%, 0,3%, 0,4%, 0,5%, 0,6%, 0,7%, 0,8%, 0,9% oder 1%, bevorzugt mindestens 1,5%, 2%, 2,5%, 3%, 3,5%, 4%, 4,5% oder 5%, besonders bevorzugt mindestens 6%, 7%, 8%, 9%, 10%, 11 %, 12%, 13%, 14% oder 15%, oder ganz besonders bevorzugt mindestens 20%, 25%, 30%, 35%, 40%, 45% oder 50% der befruchteten Samen oder Embryos einen haploiden Chromosomensatz aufweisen.

Ein "funktionelles Fragment" einer Nukleotidsequenz meint einen Abschnitt einer Nukleotidsequenz, welcher die identische oder eine vergleichbare Funktionalität wie die Gesamtnukleotidsequenz, aus welcher das funktionelle Fragment stammt, aufweist. Als solches kann das funktionelle Fragment eine Nukleotidsequenz besitzen, welche über eine Länge von mindestens 50%, 55%, 60%, 65%, 70%, 75%, 80%, 85%, 90%, 92%, 94% 96%, 97%, 98% oder 99% identisch oder homolog mit der Gesamtnukleotidsequenz ist. Weiterhin kann ein "funktionelles Fragment" einer Nukleotidsequenz auch einen Abschnitt einer Nukleotidsequenz meinen, welcher die Funktionalität der Gesamtnukleotidsequenz beispielsweise im Zuge von posttranskriptionalem oder transkriptionellem Gene Silencing verändert. Als solches kann das funktionelle Fragment einer Nukleotidsequenz mindestens 14, 15, 16, 17, 18, 19, 20, 21, 22, 23, 24 oder 25, bevorzugt mindestens 30, 35, 40, 45, 50, 60, 70, 80, 90, 100, 120 oder 140, besonders bevorzugt mindestens 160, 180, 200, 250, 300, 350, 400, 450, 500, 600, 700, 800, 900 oder 1000 aufeinanderfolgende Nukleotide der Gesamtnukleotidsequenz umfassen.

Ein "funktioneller Teil" eines Proteins meint einen Abschnitt eines Proteins bzw. eine Anschnitt der Aminosäuresequenz, die das Protein kodiert, wobei der Abschnitt die identische oder eine vergleichbare Funktionalität wie das Gesamtprotein in einer pflanzlichen Zelle ausüben kann. Ein funktioneller Teil eines Proteins weist über eine Länge von mindestens 50%, 55%, 60%, 65%, 70%, 75%, 80%, 85%, 90%, 92%, 94% 96%, 97%, 98% oder 99% eine identische oder unter Berücksichtigung von konservativen und semikonservativen Aminosäureaustauschen ähnliche Aminosäuresequenz auf wie das Protein, aus welchem der funktionelle Teil stammt.

"Haploideninduktor" meint auch einen *in vivo* Haploideninduktor.

Der Begriff "heterolog" bedeutet, dass das eingebrachte Polynukleotid beispielsweise von einer Zelle oder einem Organismus mit einem anderen genetischen Hintergrund derselben Spezies oder einer anderen Spezies stammt, oder homolog ist zu der prokaryotischen oder eukaryotischen Wirtszelle, dann aber in einer unterschiedlichen genetischen Umgebung lokalisiert ist und sich so von einem eventuell natürlicherweise vorhandenen korrespondierenden Polynukleotid unterscheidet. Ein heterologes Polynukleotid kann zusätzlich zu einem entsprechenden endogenen Gen vorhanden sein.

Unter "Hybridisieren" oder "Hybridisierung" wird ein Vorgang verstanden, bei dem sich ein einzelsträngiges Nukleinsäuremolekül an einen weitestgehend komplementären Nukleinsäurestrang anlagert, d. h. Basenpaarungen mit diesem eingeht. Standardverfahren zur Hybridisierung sind beispielsweise in Sambrook et al., Molecular Cloning: A Laboratory Manual 3rd Ed. Cold Spring Harbor Laboratory Press, Cold Spring Harbor, NY, 2001 beschrieben. Vorzugsweise wird darunter verstanden, dass mindestens 80% oder 85%, bevorzugt mindestens 90%, 91%, 92%, 93%, 94% oder 95%, besonders bevorzugt mindestens 96%, 97%, 98% oder 99% der Basen des Nukleinsäuremoleküls eine Basenpaarung mit dem weitestgehend komplementären Nukleinsäurestrang eingehen. Die Möglichkeit einer solchen Anlagerung hängt von der Stringenz der Hybridisierungsbedingungen ab. Der Begriff "Stringenz" bezieht sich auf die Hybridisierungsbedingungen. Hohe Stringenz ist dann gegeben, wenn eine Basenpaarung erschwert ist, niedrige Stringenz, wenn eine Basenpaarung erleichtert ist. Die Stringenz der Hybridisierungsbedingungen hängt beispielsweise von der Salzkonzentration bzw. Ionenstärke und der Temperatur ab. Generell kann die Stringenz durch eine Erhöhung der Temperatur und/oder eine Erniedrigung des Salzgehaltes erhöht werden. Unter "stringenten Hybridisierungsbedingungen" sind solche Bedingungen zu verstehen, bei denen eine Hybridisierung überwiegend nur zwischen homologen Nukleinsäuremolekülen und Homologen stattfindet. Der Begriff "Hybridisierungsbedingungen" bezieht sich dabei nicht nur auf die bei der eigentlichen Anlagerung der Nukleinsäuren herrschenden Bedingungen, sondern auch auf die bei den anschließenden Waschschritten herrschenden Bedingungen. Stringente Hybridisierungsbedingungen sind beispielsweise Bedingungen, unter denen überwiegend nur solche Nukleinsäuremoleküle hybridisieren, die mindestens 80%, mindestens 85%, mindestens 90% oder mindestens 95% Sequenzidentität aufweisen. Stringente Hybridisierungsbedingungen sind beispielsweise: Hybridisieren in 4 x SSC bei 65°C und anschließendes mehrfaches Waschen in 0,1 x SSC bei 65°C für insgesamt etwa 1 Stunde. Der hier verwendete Begriff "stringente Hybridisierungsbedingungen" kann auch bedeuten: Hybridisieren bei 68°C in 0,25 M Natriumphosphat, pH 7,2, 7% SDS, 1 mM EDTA und 1% BSA für 16 Stunden und anschließendes zweimaliges Waschen mit 2 x SSC und 0,1% SDS bei 68°C. Bevorzugt findet eine Hybridisierung unter stringenten Bedingungen statt.

"Die Induktionsleistung eines Haploideninduktors erhöhen" oder "die Erhöhung der Induktionsleistung eines Haploideninduktors" oder vergleichbare Ausdrücke meinen, dass die Haploiden-Induktionsrate einer Pflanze, welche die Eigenschaft eines Haploideninduktors aufweist, erhöht wird. Somit kann die Zahl der befruchteten Samen, welche einen haploiden Chromosomensatz aufweisen und aus einer Kreuzung des Haploideninduktors mit einer Pflanze derselben Gattung, vorzugsweise derselben Spezies, welche nicht die Eigenschaft eines Haploideninduktors aufweist, hervorgegangen sind, um mindestens 0,1 %, 0,2%, 0,3%, 0,4%, 0,5%, 0,6%, 0,7%, 0,8%, 0,9% oder 1%, bevorzugt mindestens 1,5%, 2%, 2,5%, 3%, 3,5%, 4%, 4,5% oder 5%, und besonders bevorzugt mindestens 6%, 7%, 8%, 9%, 10%, 15%, 20%, 30% oder 50% höher sind als die Zahl der haploiden befruchteten Samen, welche ohne die Verwendung der Nukleinsäure bzw. ohne Modifizierung des Genoms, im Speziellen ohne Mutation einer Patatin-Phospholipase im Sinne der vorliegenden Erfindung erzielt wird, d.h. die Haploiden-Induktionsrate kann um mindestens 0,1 %, 0,2%, 0,3%, 0,4%, 0,5%, 0,6%, 0,7%, 0,8%, 0,9% oder 1 %, bevorzugt mindestens 1,5%, 2%, 2,5%, 3%, 3,5%, 4%, 4,5% oder 5%, und besonders bevorzugt mindestens 6%, 7%, 8%, 9%, 10%, 15%, 20%, 30% oder 50% relativ zu der zuvor erreichten Haploiden-Induktionsrate gesteigert werden.

"Komplementäre" Nukleotidsequenz bedeutet bezogen auf eine Nukleinsäure in Form einer doppelsträngigen DNA, dass der zum ersten DNA Strang komplementäre zweite DNA Strang entsprechend den Basenpaarungsregeln die Nukleotide aufweist, die zu den Basen des ersten Stranges entsprechend den Watson-Crick-Regeln korrespondieren.

Ein "molekularer Marker" ist eine Nukleinsäure, die polymorph in einer pflanzlichen Population ist und als Bezugs- oder Orientierungspunkt verwendet wird. Ein Marker zum Erkennen eines Rekombinationsereignisses sollte geeignet sein, Unterschiede oder Polymorphismen innerhalb einer pflanzlichen Population zu überwachen. Somit ist ein solcher Marker in der Lage verschiedene allelische Zustände (Allele) zu detektieren und zu unterscheiden. Der Begriff "molekularer Marker" bezieht sich auch auf Nukleotidsequenzen, welche komplementär oder zumindest weitestgehend komplementär oder homolog sind zu genomischen Sequenzen, zum Beispiel Nukleinsäuren, welche als Sonden oder Primer eingesetzt werden. Für Marker finden sich diese Unterschiede auf DNA-Ebene und sind beispielsweise Polynukleotidsequenzunterschiede wie zum Beispiel SSRs (*simple sequence repeats*), RFLPs (*restriction fragment length polymorphisms*), FLPs (*fragment length polymorphisms*) oder SNPs (*single nucleotide polymorphisms*)*.* Die Marker können von genomischen oder exprimierten Nukleinsäuren wie beispielsweise gespleißter RNA, cDNA oder ESTs abgeleitet sein und können sich auch auf Nukleinsäuren beziehen, die als Sonden oder Primer-Paare eingesetzt werden und als solche geeignet sind, ein Sequenzfragment unter Verwendung PCR-basierter Verfahren zu amplifizieren. Marker, die genetische Polymorphismen (zwischen Teilen einer Population) beschreiben, können unter Verwendung gut etablierter Verfahren aus dem Stand der Technik nachgewiesen werden (An Introduction to Genetic Analysis. 7th Edition, Griffiths, Miller, Suzuki et al., 2000). Dazu gehören z.B. DNA-Sequenzierung, PCR-basierte sequenzspezifische Amplifikation, Nachweis von RFLPs, Nachweis von Polynukleotid-Polymorphismen mittels Allel-spezifischer Hybridisierung (ASH), Detektion von amplifizierten variablen Sequenzen des Pflanzengenoms, Detektion einer 3SR (*self sustained sequence replication*), Detektion von SSRs, SNPs, RFLPs oder AFLPs (*amplified fragment length polymorphisms*)*.* Ferner sind auch die Methoden zur Detektion von ESTs (*expressed sequence tags*) und SSR-Marker abgeleitet von EST-Sequenzen und RAPD (*randomly amplified polymorphic DNA*) bekannt. Je nach Kontext kann der Begriff Marker in der Beschreibung auch eine spezifische Chromosom-Position in dem Genom einer Spezies, wo ein spezifischer Marker (z.B. SNP) gefunden werden kann, meinen.

"Operativ verknüpft" meint verbunden in einem gemeinsamen Nukleinsäuremolekül, in einer Weise, dass die verbundenen Elemente derart zueinander positioniert und orientiert sind, dass eine Transkription des Nukleinsäuremoleküls stattfinden kann. Eine DNA, welche operativ mit einem Promotor verknüpft ist, steht unter der transkriptionellen Kontrolle dieses Promotors

Eine "Pflanze" im Sinne der Erfindung kann, sofern nicht anders angegeben, von jeder Spezies aus den dikotyledonen und monokotyledonen Pflanzen sein. Bevorzugt sind Pflanzen in Agrikultur oder Hortikultur oder zur Erzeugung von Bioenergie (Bioethanol, Biogas etc.). Hierzu zählen beispielhaft *Solanum tuberosum*, *Triticum aestivum*, *Triticum durum, Triticum spelta*, *Helianthus annuus*, *Secale cereale*, *Hordeum vulgare, Hordeum bulbosum, Brassica napus*, *Brassica oleracea*, *Brassica rapa*, *Brassica juncacea*, *Brassica nigra*, *Glycine max*, *Gossypium sp.*, *Sorghum bicolor, Sorghum sudanense, Sorghum bicolor* x *Sorghum sudanense,* Triticale, *Saccharum officinarium*, *Setaria italica, Oryza sativa*, *Oryza minuta*, *Oryza australiensis*, *Oryza alta*, *Brachypodium distachyon*, *Hordeum marinum*, *Aegilops tauschii*, *Daucus glochidiatus, Daucus pusillus*, *Daucus muricatus, Daucus carota, Eucalyptus grandis, Erythranthe guttata*, *Genlisea aurea*, *Musa sp.*, *Avena sp.*, *Nicotiana sylvestris, Nicotiana tabacum*, *Nicotiana tomentosiformis, Solanum lycopersicum*, *Coffea canephora*, *Vitis vinifera*, *Cucumis sativus, Morus notabilis*, *Crucihimalaya himalaica, Crucihimalaya wallichii*, *Cardamine flexuosa*, *Lepidium virginicum, Capsella bursa-pastoris*, *Olmarabidopsis pumila*, *Arabis hirsuta*, *Raphanus sativus, Eruca vesicaria sativa*, *Citrus sinensis, Jatropha curcas, Populus trichocarpa* oder *Beta vulgaris.*

Eine erfindungsgemäße Sorghumhirsepflanze ist eine Pflanze der Gattung *Sorghum,* insbesondere der Spezies *Sorghum bicolor, Sorghum sudanense, Sorghum bicolor* × *Sorghum sudanense, Sorghum* × *almum* (*Sorghum bicolor* × *Sorghum halepense*), Sorghum arundinaceum, Sorghum × drummondii, *Sorghum halepense* und/oder Sorghum propinquum bzw. deren Hybriden sowie sämtliche sich davon ableitenden Sorten.

Pflanzliche "Organe" meinen beispielsweise Blätter, Sprossachse, Stamm, Wurzeln, vegetative Knospen, Meristeme, Embryos, Antheren, Ovula, Samen oder Früchte, insbesondere Samenkörner. Der Begriff "Pflanzenteil" bzw. "Pflanzenteile" beinhaltet, ist jedoch nicht beschränkt auf, die Sprossachse bzw. den Halm, Blätter, Blüten, Blütenstände, Wurzeln, Früchte und Samen sowie die Pollen. Pflanzliche "Teile" meinen ferner einen Zusammenschluss mehrerer Organe, z.B. eine Blüte oder ein Samen, oder einen Teil eines Organs, z.B. einen Querschnitt aus der Sprossachse. Pflanzliche "Gewebe" sind zum Beispiel Kallusgewebe, Speichergewebe, meristematische Gewebe, Blattgewebe, Sprossgewebe, Wurzelgewebe, Pflanzentumorgewebe oder reproduktives Gewebe sowie das Bildungsgewebe, Grundgewebe (das sogenannte Parenchym), Leitgewebe, Festigungsgewebe und das Deckgewebe (die sogenannte Epidermis). Das Gewebe wird durch diese Auflistung jedoch nicht beschränkt. Unter pflanzlichen "Zellen" sind beispielsweise isolierte Zellen mit einer Zellwand oder Aggregate davon oder Protoplasten zu verstehen.

Ein "Promotor" ist ein nicht-translatierter DNA-Abschnitt, typischerweise stromaufwärts einer kodierenden Region, welche die Bindestelle für die RNA-Polymerase beinhaltet und die Transkription der DNA initiiert. Ein Promotor enthält zudem andere Elemente, die als Regulatorgen der Genexpression fungieren (z.B. cis-regulatorischen Elemente). Ein "Kern-oder Minimalpromotor" ist ein Promoter, der zumindest die Grundelemente, welche für die Transkriptionsinitiation gebraucht werden, aufweist (z.B. TATA-Box und/oder Initiator).

Im Zusammenhang mit der vorliegenden Erfindung betrifft der Begriff "Modifikationen" eine Nukleotidsequenz, welche die Spezifität und/oder die Expressionsstarke beeinflusst, zum Beispiel indem die regulatorische Sequenz eine bestimmte Gewebespezifität vermittelt. Eine solche regulatorische Sequenz kann stromaufwärts des Transkriptionsinitiationspunkts eines Minimalpromotors, aber auch stromabwärts davon wie beispielsweise in einer transkribierten aber nicht translatierten Leader-Sequenz oder innerhalb eines Introns lokalisiert sein.

Eine "Transgene Pflanze" bezieht sich auf eine Pflanze, in deren Genom mindestens ein Polynukleotid, vorzugsweise ein heterologes Polynukleotid, integriert ist. Bevorzugt ist das Polynukleotid stabil integriert, was bedeutet, dass das integrierte Polynukleotid in der Pflanze stabil erhalten bleibt, exprimiert wird und auch stabil an die Nachkommen vererbt werden kann. Das stabile Einbringen eines Polynukleotids in das Genom einer Pflanze schließt auch die Integration in das Genom einer Pflanze der vorhergehenden Parentalgeneration mit ein, wobei das Polynukleotid stabil weitervererbt werden kann. Der Begriff "heterolog" bedeutet, dass das eingebrachte Polynukleotid beispielsweise von einer Zelle oder einem Organismus mit einem anderen genetischen Hintergrund derselben Spezies oder einer anderen Spezies stammt, oder homolog ist zu der prokaryotischen oder eukaryotischen Wirtszelle, dann aber in einer unterschiedlichen genetischen Umgebung lokalisiert ist und sich so von einem eventuell natürlicherweise vorhandenen korrespondierenden Polynukleotid unterscheidet. Ein heterologes Polynukleotid kann zusätzlich zu einem entsprechenden endogenen Gen vorhanden sein.

"Zur Verwendung als Haploideninduktor geeignet" oder "ist in der Lage Haploidie zu induzieren" meint, dass eine Pflanze in die Lage ist, befruchtete Samen, welche einen einfachen (haploiden) Chromosomensatz aufweisen, aus einer Kreuzung mit einer Pflanze derselben Gattung, vorzugsweise derselben Spezies, welche nicht die Eigenschaft eines Haploideninduktors aufweist, hervorzubringen. Die Verwendung als Haploideninduktor, angegeben als absolute Haploiden-Induktionsrate, meint, dass mindestens 0,1 %, 0,2%, 0,3%, 0,4%, 0,5%, 0,6%, 0,7%, 0,8%, 0,9% oder 1 %, bevorzugt mindestens 1,5%, 2%, 2,5%, 3%, 3,5%, 4%, 4,5% oder 5%, besonders bevorzugt mindestens 6%, 7%, 8%, 9%, 10%, 11 %, 12%, 13%, 14% oder 15%, oder ganz besonders bevorzugt mindestens 20%, 25%, 30%, 35%, 40%, 45% oder 50% der befruchteten Samen einen haploiden Chromosomensatz aufweisen.

Ausgestaltungen und Ausführungsformen der vorliegenden Erfindung werden in exemplarischer Weise mit Bezug auf die angehängten Figuren und Sequenzen beschrieben:

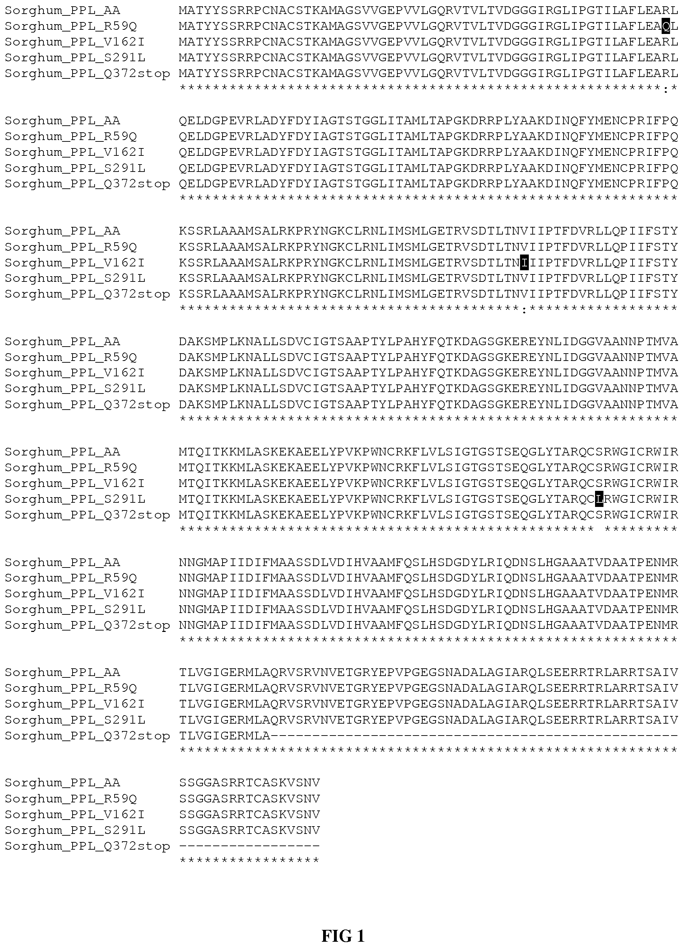

- **Figur 1:**: Sequenzalignment (CLUSTAL O 1.2.4) der Aminosäuresequenzen der Wild-Typ Patatin-Phospholipase aus Sorghumhirse (Sorghum_PPL_AA, SEQ ID Nr.: 3), der Patatin-Phospholipase aus Sorghumhirse mit einem Austausch der Aminosäure Arginin (R) durch Glutamin (Q) an der Aminosäureposition 59 (Sorghum_PPL_R59Q, SEQ ID Nr.: 6), der Patatin-Phospholipase aus Sorghumhirse mit einem Austausch der Aminosäure Valin (V) durch Isoleucin (I) an der Aminosäureposition 162 (Sorghum_PPL_V162I, SEQ ID Nr.: 9), der Patatin-Phospholipase aus Sorghumhirse mit einem Austausch der Aminosäure Serin (S) durch Leucin (L) an der Aminosäureposition 291 (Sorghum_PPL_S291L, SEQ ID Nr.: 12) und der Patatin-Phospholipase aus Sorghumhirse mit einem Austausch der Aminosäure Glutamin (Q) durch ein Stopp-Codon an der Aminosäureposition 372 (Sorghum_PPL_Q372Stopp, SEQ ID Nr.: 15). Die Aminosäurepositionen 59, 162 und 291 sind schwarz unterlegt.

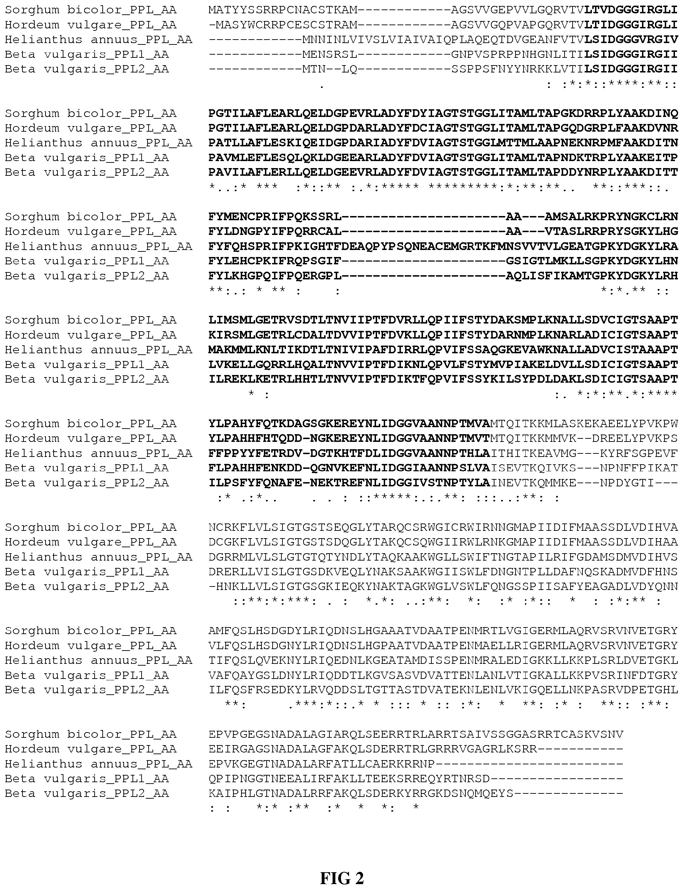

- **Figur 2:**: Sequenzalignment (CLUSTAL O 1.2.4) der Aminosäuresequenzen der Wild-Typ Patatin-Phospholipase aus Sorghumhirse (Sorghum bicolor_PPL_AA, SEQ ID Nr.: 3), der Patatin-Phospholipase aus Gerste (Hordeum vulgare_PPL_AA, SEQ ID Nr.: 21), der Patatin-Phospholipase aus Sonnenblume (Helianthus annuus_PPL_AA, SEQ ID Nr.: 18), der Patatin-Phospholipase 1 aus Zuckerrübe (Beta vulgaris_PPLl_AA, SEQ ID Nr.: 24) und der Patatin-Phospholipase 2 aus Zuckerrübe (Beta vulgaris _Phospholipase2_AA, SEQ ID Nr.: 27). Die Aminosäurepositionen, welche die funktionelle Domäne der Phospholipase darstellen, sind fett gedruckt.

### AUSFÜHRLICHE BESCHREIBUNG DER ERFINDUNG

Die vorliegende Erfindung stellt eine Pflanze der Gattung Sorghum, im Folgenden auch als Sorghumhirsepflanze bezeichnet, zur Verfügung, die in der Lage ist Haploidie zu induzieren. Durch diese Eigenschaft der Haploideninduktion ist die Pflanze in der Lage, befruchtete Samen oder Embryos, welche einen einfachen (haploiden) Chromosomensatz aufweisen, aus einer Kreuzung mit einer Pflanze derselben Gattung, vorzugsweise derselben Spezies, welche nicht die Eigenschaft eines Haploideninduktors aufweist, hervorzubringen. Durch dieses System war es den Erfindern möglich, ein effizientes System zur Züchtung von Sorghumhirsepflanzen bereitzustellen, denn durch Chromosomenverdopplung in den haploiden Nachkommen können Inzuchtlinien, d.h. reinerbige Vater- und Mutterlinien für die Hybridzüchtung generiert werden.

Die erfindungsgemäßen Sorghumhirsepflanzen sind dadurch gekennzeichnet, dass sie eine oder mehrere Modifikationen aufweisen, die die Patatin-Phospholipase betreffen, wodurch entweder die Eigenschaft zur Haploideninduktion vermittelt wird oder auch eine natürlich vorhandenen Fähigkeit zur Haploideninduktion verbessert bzw. die Induktionsleistung erhöht wird. Somit ist die Modifikation betreffend die Patatin-Phospholipase ursächlich für die Eignung der erfindungsgemäßen Sorghumhirsepflanze als Haploideninduktor in Züchtungsprogrammen.

Erfindungsgemäße Pflanzen zeichnen sich dadurch aus, dass sie eine Induktionsrate von vorzugsweise mindestens 0,5% aufweisen und sich somit von Wildtyp-Pflanzen bzw. von Nicht-Haploideninduktoren unterscheiden.

In den im Rahmen der vorliegenden Erfindung durchgeführten Experimenten wurde festgestellt, dass die Patatin-Phospholipase in Sorghumhirse pollenspezifisch exprimiert wird und daher vermutlich einen Einfluss auf das Pollenschlauch-Wachstum oder die Interaktion zwischen den Gametophyten hat. Somit ist die erfindungsgemäße Sorghumhirsepflanze vorzugsweise dadurch gekennzeichnet, dass die Patatin-Phospholipase pollenspezifisch exprimiert wird und/oder Einfluss auf das Pollenschlauch-Wachstum oder die Interaktion zwischen den Gametophyten hat.

In einer bevorzugten Ausführungsform wird die Patatin-Phospholipase durch die Nukleotidsequenz gemäß SEQ ID Nr.: 1 oder 2 kodiert ist oder durch eine Nukleotidsequenz, die zu mindestens 80%, vorzugsweise 85%, weiter bevorzugt 90%, noch weiter bevorzugt 95% und am bevorzugtesten 99% identisch zu SEQ ID Nr.: 1 oder 2 ist, kodiert ist oder durch eine Nukleotidsequenz kodiert wird, die mit der komplementären Sequenz zu der Nukleotidsequenz gemäß SEQ ID Nr.: 1 oder 2 unter stringenten Bedingungen hybridisiert, beziehungsweise die in SEQ ID Nr.: 3 gezeigte Aminosäuresequenz oder eine homologe Aminosäuresequenz umfasst. Wie oben bereits erwähnt, umfasst die Sorghumhirsepflanze in Sinne der vorliegenden Erfindung Pflanzen jeglicher Art der Gattung Sorghum, insbesondere der Arten *Sorghum bicolor, Sorghum sudanense* und *Sorghum bicolor* x *Sorghum sudanense* bzw. deren Hybriden sowie sämtliche sich davon ableitenden Sorten. Dementsprechend ist vernünftigerweise anzunehmen, dass sich im Laufe der Artbildung sowie der Sortenzüchtung die Nukleotidsequenz und entsprechend auch die Aminosäuresequenz der Patatin-Phospholipase verändert haben. In diesem Zusammenhang bedeutet der Begriff homolog, dass die betreffenden Gene (aus zwei verschiedenen Pflanzenarten bzw. Sorten) im Wesentlichen die gleiche Funktion und einen gemeinsamen Vorläufer haben, und sich daher typischerweise eine signifikante Identität in deren Nukleinsäure- bzw. kodierten Aminosäuresequenz zeigt, welche vorzugsweise bei mindestens 80% liegt.

Im Sinne der Erfindung wird unter einem "Homolog" ein Protein gleichen phylogenetischen Ursprungs verstanden, unter einem "Analog" ein Protein verstanden, welches die gleiche Funktion ausübt, jedoch einen anderen phylogenetischen Ursprung hat, unter einem "Ortholog" ein Protein aus einer anderen Spezies verstanden, das die gleiche Funktion ausübt und unter einem "Paralog" ein Protein verstanden, das durch Duplikation innerhalb einer Spezies entstanden ist, wobei diese Kopie entweder die gleiche Proteinfunktion behält, ihr Expressionsmuster verändert, jedoch nicht die Funktion, ihre Proteinfunktion ändert oder sich die ursprüngliche Genfunktion zwischen beiden Kopien aufteilt.

Ein kodiertes Protein (bzw. Aminosäuresequenz) ist grundsätzlich dann ein Homolog im Sinne der vorliegenden Erfindung, wenn es die gleiche Funktion ausübt, unabhängig davon ob es denselben oder einen anderen phylogenetischen Ursprung hat oder aus derselben oder einer anderen Spezies stammt. Eine Homolog ist ferner in der Lage ist, die Eigenschaft der Haploideninduktion zu komplementieren, d.h. durch eine Modifizierung des durch das Homolog kodierenden Genprodukts der Pflanze, aus der das Gen stammt, die Eigenschaft eines Haploideninduktors zu vermitteln oder die Induktionsleistung eines Haploideninduktors zu erhöhen. Dementsprechend kann das betreffende Homolog zur Patatin-Phospholipase kodiert durch die Nukleotidsequenz gemäß SEQ ID Nr.: 1 oder 2 bzw. umfassend die Aminosäuresequenz gemäß SEQ ID Nr.: 3 vorzugsweise dadurch gekennzeichnet werden, dass es in der Lage ist die Eigenschaft eines Haploideninduktors, die in der erfindungsgemäßen Sorghumhirsepflanze beobachtet wird, zu komplementieren. Zusätzlich oder alternativ kann das Patatin-Phospholipase-Homolog vorzugsweise dadurch gekennzeichnet werden, dass durch die Modifizierung des durch das Homolog kodierten Genprodukts die Eigenschaft eines Haploideninduktors vermittelt oder die Induktionsleistung eines Haploideninduktors erhöht wird.

Entsprechende Techniken und Verfahren zu Komplementationsgenetik in Sorghum sind dem Fachmann aus dem Stand der Technik bekannt, beispielsweise aus der Publikation von Li et al., J Genet. 94 (2015), 445-452, in der der Sorghum-Phänotyp, der sich durch eine braune Mittelrippe auszeichnet und durch eine Punktmutation in dem *bmr-6* Gen verursacht wird, durch Einbringen des Wildtpy-bmr-6 Gens komplementiert wurde.

Wie in Beispiel 2 gezeigt, führt ein Aminosäureaustausch in der Aminosäuresequenz der Patatin-Phospholipase aus Sorghumhirse (SEQ ID Nr.: 3) in Sorghumhirse zu einer Haploideninduktionsrate von teilweise mehr als 1,5%. Es ist davon auszugehen, dass durch weitere Aminosäureaustausche, was zu einer weiteren Modifizierung der kodierenden Sequenz der Patatin-Phospholipase führt, es zu einer weiteren Erhöhung der Induktionsrate kommen kann. Somit sind von der vorliegenden Erfindung Sorghumhirsepflanzen umfasst, die eine oder mehrere Modifikationen bzw. Mutationen aufweisen, die die Patatin-Phospholipase betreffen.

Die oben erwähnten Modifikationen, die die Patatin-Phospholipase betreffen, sind im Sinne der vorliegenden Erfindung bevorzugt dadurch gekennzeichnet, dass es Mutationen sind, die in der endogenen DNA-Sequenz kodierend die Patatin-Phospholipase, zu einen oder mehreren Aminosäureaustauschen führen oder zu der Erzeugung eines Stopp-Codons.

Eine Mutation meint eine Modifikation auf DNA-Ebene, also eine Veränderung der Genetik und/oder der Epigenetik. Beispielsweise kann eine Veränderung der Genetik der Austausch von mindestens einer Nukleobase in der endogenen DNA-Sequenz oder in einer regulatorischen Sequenz der endogenen DNA-Sequenz sein. Findet ein solcher Nukleobasen-Austausch z.B. in einem Promotor statt, kann dies zu einer veränderten Aktivität des Promotors führen, da beispielsweise *cis*-regulatorische Elemente derart modifiziert werden, dass die Affinität eines Transkriptionsfaktors zu dem mutierten *cis*-regulatorischen Element im Vergleich zum Wildtyp-Promotor verändert ist, sodass die Aktivität des Promotors mit dem mutierten *cis*-regulatorischen Element erhöht oder erniedrigt ist, je nachdem ob es sich bei dem Transkriptionsfaktor um einen Repressor oder Induktor handelt oder ob die Affinität des Transkriptionsfaktors zu dem mutierten *cis*-regulatorischen Element verstärkt oder abgeschwächt wird. Findet ein solcher Nukleobasen-Austausch z.B. in einer kodierenden Region der endogenen DNA-Sequenz statt, kann dies zu einem Aminosäureaustausch im kodierten Protein führen, was eine Veränderung der Aktivität oder Stabilität des Proteins im Vergleich zum Wildtyp-Protein bewirken kann. Ein weiteres Beispiel für eine Veränderung der Genetik ist die Deletion von Nukleotiden in der regulatorische Sequenz und/oder der endogenen DNA-Sequenz sowie die Addition von Nukleotiden in der regulatorischen Sequenz und/oder der endogenen DNA-Sequenz. Eine Veränderung der Epigenetik kann beispielsweise durch ein verändertes Methylierungsmuster der DNA erfolgen.

Verfahren zum Mutagenisieren von DNA-Sequenzen werden im Zusammenhang mit dem Verfahren zum Erhalt eines pflanzlichen Haploideninduktors näher beschrieben.

Da die beschriebenen Mutationen zu einer Änderung oder Verkürzung der Aminosäuresequenz der Patatin-Phospholipase führen, kann davon ausgegangen werden, dass die Mutationen die Aktivität oder Stabilität der durch die endogene DNA-Sequenz kodierten Patatin-Phospholipase in der Sorghumhirsepflanze im Vergleich zu einer Wildtyp-Pflanze verändern, d.h. erhöhen oder reduzieren.

Die Erzeugung von Stopp-Codons in den funktionellen Domänen eines Proteins führt in der Regel zu einem Verlust der Funktion des Proteins, wohingegen die Erzeugung eines Stopp-Codons nach der funktionellen Domäne auch zu einer Erhöhung der Aktivität oder Stabilisierung des Proteins führen könnte. Im Falle der Mutationen, die vorzugsweise Aminosäuresubstitutionen sind, kann es zu einer Erhöhung der Aktivität des Proteins kommen, zum Beispiel durch Optimierung der Sequenz oder ebenso gut zu einer Inhibierung oder zum Verlust der Aktivität. Auch kann es durch Mutationen in dem Promotorbereich zu einer Änderung der Expression des Gens kommen.

Bezogen auf die vorliegende Erfindung kann demensprechend durch die beschriebene Mutation die biologische Aktivität der Patatin-Phospholipase dahingehend geändert werden, dass ihre ursprüngliche Funktion in Pollen nicht mehr in dem Maße ausgeübt wird, wie es in der Wildtyp-Sorghumhirsepflanze der Fall ist.

Dies kann einerseits durch Überexpression des Gens verbunden mit einer erhöhten Proteinmenge und Aktivität geschehen, beispielsweise durch Mutationen in dem Promotorbereich, durch die Erhöhung der Stabilität des Proteins, beispielsweise durch eine Verkürzung des Proteins durch die Erzeugung eines Stopp-Codons, oder durch die Bildung einer aktiveren Form der Patatin-Phospholipase, beispielsweise durch Aminosäuresubstitutionen in der funktionellen Domäne, was beispielsweise zu einem schnelleren Wachstum des Pollenschlauchs, einer Entkopplung des Transports der generativen Zellen im Pollenschlauch mit dessen Wachstum oder einer gestörten Interaktion der Gametophyten gefolgt von einer unvollständigen Befruchtung mit anschließender Chromosomeneliminierung führen könnte. Andererseits könnte auch durch die Überexpression des Gens die korrekte Bildung, Faltung und/oder Stabilität der Patatin-Phospholipase inhibiert, verhindert oder herabgesetzt werden. Es könnte jedoch auch zu einer verringerten Bildung oder keiner Bildung einer funktionalen Patatin-Phospholipase kommen, beispielsweise durch Mutationen in dem Promotorbereich, zu der Bildung einer weniger aktiven, inaktiven oder instabilen Form der Patatin-Phospholipase, beispielsweise durch die Erzeugung eines Stopp-Codons oder durch Aminosäuresubstitutionen in der funktionellen Domäne, was auch wiederum zu einer fehlerhaften Befruchtung führen könnte. Ebenso könnte durch die beschriebenen Mutationen die Lokalisation der Patatin-Phospholipase verändert werden, sodass sie beispielsweise nicht mehr pollenspezifisch exprimiert wird.

Infolgedessen sind in der vorliegenden Erfindung auch Sorghumhirsepflanzen umfasst, die eine oder mehrere Insertionen einer Expressionskassette aufweisen, die ein Nukleinsäuremolekül kodierend die Patatin-Phospholipase, die durch die Nukleotidsequenz gemäß SEQ ID Nr.: 1 oder 2 oder durch eine Nukleotidsequenz, die zu mindestens 80%, vorzugsweise 85%, weiter bevorzugt 90%, noch weiter bevorzugt 95% und am bevorzugtesten 99% identisch ist zu SEQ ID Nr.: 1 oder 2 kodiert wird, oder kodierend einen funktionellen Teil dieser Patatin-Phospholipase, oder die ein Nukleinsäuremolekül aufweisend eine Nukleotidsequenz, die mit der komplementären Sequenz zu der Nukleotidsequenz gemäß SEQ ID Nr.: 1 oder 2 unter stringenten Bedingungen hybridisiert, oder die ein Nukleinsäuremolekül kodierend die Patatin-Phospholipase mit einer Aminosäuresequenz gemäß der SEQ ID Nr.: 3 oder einer homologe Aminosäuresequenz oder einen funktionellen Teil davon umfasst, und operativ verknüpft mit einem Promotor, vorzugsweise einem Pollen-spezifischen Promotor, ist, wobei die kodierte Patatin-Phospholipase oder der funktionellen Teil davon eine oder mehrere Mutationen aufweist, welche zu einem oder mehreren Aminosäureaustauschen oder Erzeugen eines Stopp-Codons führen und somit für eine Überexpression des Gens kodierend die Patatin-Phospholipase in einer Pflanze oder einem Teil davon im Vergleich zu einer Wildtyp-Pflanze oder entsprechenden Teil davon sorgt.

Ebenso sind von der vorliegenden Erfindung auch Sorghumhirsepflanzen umfasst, in denen die Expression der Patatin-Phospholipase teilweise oder vollständig inhibiert ist bzw. eine verringerte Menge des Patatin-Phospholipase Proteins vorliegen oder keine funktionale Patatin-Phospholipase gebildet wird. Somit umfasst die vorliegende Erfindung auch Sorghumhirsepflanzen, in denen durch einen RNAi-Ansatz (Fire et al., Nature 391 (1998), 806-811) die Expression der genannten Patatin-Phospholipase teilweise oder vollständig inhibiert ist. Demensprechend ist die erfindungsgemäße Pflanze weiterhin dadurch gekennzeichnet, dass sie eine oder mehrere Insertionen einer Expressionskassette aufweist, welche einen Promotor und ggf. einen Terminator umfasst der operativ mit einem Nukleinsäuremolekül verknüpft ist, das eine dsRNA kodiert, die mindestens 19 oder 20, bevorzugt mindestens 21, 22, 23, 24 oder 25, besonders bevorzugt mindestens 30, 35, 40, 45 oder 50, und insbesondere bevorzugt mindestens 100, 200, 300 oder 500 Nukleotide, die komplementär zu einer Teilsequenz der Nukleotidsequenz gemäß SEQ ID Nr.: 1 oder 2 oder einer Teilsequenz der Nukleotidsequenz die zu mindestens 80%, vorzugsweise 85%, weiter bevorzugt 90%, noch weiter bevorzugt 95% und am bevorzugtesten 99% identisch zu einer Teilsequenz der Nukleotidsequenz gemäß SEQ ID Nr.: 1 oder 2 aufweist, umfasst.

Zudem umfasst die vorliegende Erfindung auch Sorghumhirsepflanzen in denen es durch Mutation zu einem Knock-out der Patatin-Phospholipase kommt.

Geeignete Promotoren, verwendbar in den Expressionskassetten, können Promotoren sein, welche konstitutiv induziert werden (Bsp.: 35S-Promoter aus dem "*Cauliflower mosaic virus*" (Odell et al., Nature 313 (1985), 810-812), besonders geeignet sind solche Promotoren, welche entwicklungsspezifisch (Bsp.: blühspezifische Promotoren) oder gewebespezifisch sind, insbesondere solche, welche in Pollen spezifisch aktiv sind (Beispiele: Chen et al., Molecular Biology Reports 37 (2010), 737-744, Zhao et al., Planta 224 (2006), 405-412 oder Twell et al. Genes & Development 5(1991), 496-507). Geeignete Promotoren können auch synthetische bzw. chimäre Promotoren sein, welche in der Natur nicht vorkommen, aus mehreren Elementen zusammengesetzt sind und einen Minimalpromotor beinhalten sowie stromaufwärts des Minimalpromotors mindestens ein *cis*-regulatorisches Element aufweisen, welches als Bindungsstelle für spezielle Transkriptionsfaktoren dient. Chimäre Promotoren können den gewünschten Spezifitäten nach konzipiert und werden durch unterschiedliche Faktoren induziert oder reprimiert. Beispiele für solche Promotoren finden sich in Gurr and Rushton (TRENDS in Biotechnology 23 (2005), 275-282) oder Venter (Trends in Plant Science 12 (2007), 118-1249. Ein geeigneter Terminator ist beispielsweise der *nos-*Terminator (Depicker et al., Journal of Molecular and Applied Genetics 126 (1982), 561-573). Promotoren und andere Transkriptionsregulationselemente sind allgemein bekannt und dem Fachmann im Stand der Technik zugänglich; siehe beispielsweise WO 00/75359 auf Seite 23, Zeile 5 bis Seite 24, Zeile 17.

Wie in Beispiel 2 gezeigt, führt ein Austausch der Aminosäure Arginin gegen Glutamin an der Aminosäureposition 59 gemäß der Aminosäuresequenz der Patatin-Phospholipase gezeigt in SEQ ID Nr.: 6 oder ein Austausch der Aminosäure Valin durch Isoleucin an der Aminosäureposition 162 gemäß der Aminosäuresequenz der Patatin-Phospholipase gezeigt in SEQ ID Nr.: 9 in Sorghumhirsepflanzen zu einer Haploideninduktionsrate von mehr als 0,5%.

Anhand von Fig. 2 ist zu erkennen, dass diese Aminosäurepositionen in der funktionellen Domäne der Patatin-Phospholipase liegen, die dem Bereich der Aminosäurepositionen 37 bis 240 entspricht.

Somit stellt die vorliegende Erfindung Sorghumhirsepflanzen bereit, die eine oder mehrere Mutationen aufweisen, die in dem Bereich der Aminosäurepositionen 37 bis 240 gemäß SEQ ID Nr.: 3 zu einem Aminosäureaustausch führen, was vorzugsweise der funktionellen Domäne der Patatin-Phospholipase entspricht. In einer bevorzugten Ausführungsform führen die eine oder mehrere Mutationen in dem Bereich der Aminosäurepositionen 40 bis 93 oder 135 bis 204, bevorzugt in dem Bereich der Aminosäurepositionen 53-85 oder 150-192, besonders bevorzugt in dem Bereich der Aminosäurepositionen 55-75 oder 157-167 zu einem Aminosäureaustausch.

Des Weiteren führt ein Austausch der Aminosäure Serin gegen Leucin an der Aminosäureposition 291 gemäß der Aminosäuresequenz der Patatin-Phospholipase gezeigt in SEQ ID Nr.: 12 in Sorghumhirsepflanzen zu einer Haploideninduktionsrate von mehr als 1,5%. Auch konnte durch eine Mutation in der Nukleotidsequenz gemäß SEQ ID Nr.: 1, die zu einem Stopp-Codon an der Aminosäureposition 372 gemäß SEQ ID Nr.: 3 führte, ein Sorghumhirse-Haploideninduktor generiert werden. Durch diese Mutation wurde die Aminosäure Glutamin (Q) an der Position 372 durch ein Stopp-Codon ausgetauscht.

Anhand von Fig. 2 ist zu erkennen, dass diese Positionen außerhalb der funktionellen Domäne der Patatin-Phospholipase liegen.

Somit stellt die vorliegende Erfindung auch Sorghumhirsepflanzen bereit, die eine oder mehrere Mutationen aufweisen, die in dem Bereich der Aminosäurepositionen 241 bis 385 gemäß SEQ ID Nr.: 3 zu einem Aminosäureaustausch führen, was vorzugsweise einer Region außerhalb der funktionellen Domäne der Patatin-Phospholipase entspricht, wobei die Mutation auch zu einem Stopp-Codon und somit zu einer Verkürzung der Patatin-Phospholipase führen kann. In einer bevorzugten Ausführungsform führen die eine oder mehrere Mutationen in dem Bereich der Aminosäurepositionen 270 bis 320, bevorzugt in dem Bereich der Aminosäurepositionen 285 bis 311, besonders bevorzugt in dem Bereich der Aminosäurepositionen 285 bis 298 zu einem Aminosäureaustausch und/oder in dem Bereich der Aminosäurepositionen 322-402, bevorzugt in dem Bereich der Aminosäurepositionen 342-392, besonders bevorzugt in dem Bereich der Aminosäurepositionen 362-382 zu einem Stopp-Codon.

In einer besonders bevorzugten Ausführungsform der vorliegenden Erfindung ist die erfindungsgemäße Pflanze dadurch gekennzeichnet, dass die eine oder mehrere Mutationen zu einem Aminosäureaustausch an der Aminosäureposition 59, 162 und/oder 291 führen und/oder zu einem Stopp-Codon an der Aminosäureposition 372 gemäß SEQ ID Nr.: 3.

In diesem Zusammenhang umfasst in einer Ausführungsform der vorliegenden Erfindung die modifizierte Patatin-Phospholipase eine Aminosäuresequenz gemäß SEQ ID Nr.: 3, in der mindestens ein Aminosäureaustausch vorhanden ist, wobei an der Position 59 Arginin (R), an der Position 162 Valin (V), und/oder an der Position 291 Serin (S) durch eine andere Aminosäure ausgetauscht ist, vorzugsweise durch Glutamin (Q) an der Position 59, Isoleucin (I) an der Position 162 und/oder Leucin (L) an der Position 291 beziehungsweise wird durch eine Nukleotidsequenz gemäß SEQ ID Nr.: 1 kodiert, in der mindestens ein Nukleotidaustausch vorhanden ist, was zu einem Aminosäureaustauch und/oder zu einem Stopp-Codon führt, wobei an den Positionen 421-423, 815-817, 1420-1422 und/oder 1663-1665 gemäß SEQ ID Nr.: 1 (entsprechend der Nukleotid-Positionen 175-177, 484-486, 871-873 und/oder 1114-1116 der SEQ ID Nr.: 2) ein oder mehrere Nukleotide ausgetauscht sind.

Pflanzen besitzen als Eukaryoten zwei oder mehr Kopien ihrer genetischen Information pro Zelle. Jedes Gen wird in der Regel durch zwei Allele, die im homozygoten Zustand identisch bzw. im heterozygoten Zustand unterschiedlich sein können, repräsentiert. Der Phänotyp der erfindungsgemäßen Pflanze wird durch eine oder mehrere Mutationen in der Patatin-Phospholipase verursacht, wobei die erfindungsgemäße Pflanze homozygot oder heterozygot, vorzugsweise homozygot für die mutierte Patatin-Phospholipase ist.

In einer weiteren Ausführungsform der vorliegenden Erfindung wird ein Nukleinsäuremolekül beansprucht, welches die zuvor definierten spezifischen Mutationen umfasst, die zu den Aminosäureaustauschen R59Q, V162I, S291L und/oder Q372Stopp basierend auf der Aminosäuresequenz von SEQ ID Nr.: 3 führen. Dabei ist das erfindungsgemäße Nukleinsäuremolekül dadurch gekennzeichnet, dass dessen Vorhandensein in einer Pflanze dazu führt, dass die Pflanze in der Lage ist Haploidie zu induzieren oder dass die Induktionsleistung einer Pflanze, die bereits zur Haploideninduktion fähig ist, verbessert wird. Vorzugsweise führt das Vorhandensein des erfindungsgemäßen Nukleinsäuremoleküls in Abwesenheit einer Wildtyp-Patatin-Phospholipase in einer Pflanze dazu, dass die Pflanze in der Lage ist Haploidie zu induzieren oder dass die Induktionsleistung einer Pflanze, die bereits zur Haploideninduktion fähig ist, verbessert wird.

Das erfindungsgemäße Nukleinsäuremolekül kann als Transgen eingesetzt werden, um in einer Pflanze die Eigenschaft eines Haploideninduktors zu vermitteln oder die Induktionsleistung eines Haploideninduktors zu erhöhen. Vorzugsweise handelt es sich bei dem erfindungsgemäßen Nukleinsäuremolekül um ein isoliertes Nukleinsäuremolekül, welches aus seiner natürlichen bzw. ursprünglichen Umgebung, d.h. dem genetischen Kontext, herausgelöst ist. Ein Nukleinsäuremolekül kann doppelsträngig oder einzelsträngig, linear oder zirkulär sein. Es kann sich hierbei um genomische DNA, synthetische DNA, cDNA oder einen RNA-Typ, beispielsweise siRNA oder miRNA, handeln, wobei in RNA statt der Nukleobase Thymin die Nukleobase Uracil vorkommt.

In einer bevorzugten Ausgestaltung der vorliegenden Erfindung hat die erfindungsgemäße Nukleinsäure oder eine von der Nukleinsäure kodierte RNA oder ein von der Nukleinsäure kodiertes Protein oder Polypeptid Einfluss auf das Pollenschlauch-Wachstum in einer Pflanze, auf die Interaktion zwischen die Gametophyten oder auf die Befruchtung als solches.

DNA-Hybridisierungssonden, die von der modifizierten Sequenz der Patatin-Phospholipase abgeleitet sind, d.h. eine der oben beschriebenen Mutationen umfassen, können zum Identifizieren von erfindungsgemäßen Pflanzen verwendet werden, d.h. zur Detektion der Mutationen im Gen der Patatin-Phospholipase eingesetzt werden. Um eine spezifische Hybridisierung zu erzielen, sollten solche Sonden spezifisch sein und mindestens eine Länge von 15 Nukleotiden, bevorzugt mindestens 20 Nukleotide aufweisen. Eine ausführliche Anleitung zu der Hybridisierung von Nukleinsäuren kann man in Tijssen, Laboratory Techniques in Biochemistry and Molecular Biology - Hybridization with Nucleic Acid Probes, Teil 1, Kapitel 2, "Overview of principles of hybridization and the strategy of nucleic acid probe assays," Elsevier, New York (1993); und in Current Protocols in Molecular Biology, Kapitel 2, Ausubel, et al., eds, Greene Publishing and Wiley Interscience, New York (1995) finden. Die Sonden können auch zum Amplifizieren eines Bereiches der modifizierten Sequenz der Patatin-Phospholipase, der mindestens eine der zuvor beschriebenen Mutationen erhält, durch den bekannten Prozess der Polymerase Kettenreaktion (PCR) eingesetzt werden.

Daher ist ein Nukleinsäuremolekül von mindestens 15, 16, 17, 18, 19 oder 20, bevorzugt mindestens 21, 22, 23, 24 oder 25, besonders bevorzugt mindestens 30, 35, 40, 45 oder 50, und insbesondere bevorzugt mindestens 100, 200, 300, 500 oder 1000 Nukleotiden Länge Gegenstand der vorliegenden Erfindung, wobei dieses Nukleinsäuremolekül spezifisch an eine zuvor beschriebene Nukleotidsequenz, die das modifizierte Patatin-Phospholipase Gen umfasst, hybridisiert, und eine der Mutationen umfasst oder ein Paar von Nukleinsäuremolekülen, das geeignet ist einen Bereich, der mindestens eine der Mutationen enthält, in einer Polymerase-Kettenreaktion (PCR) zu amplifizieren, vorzugsweise in Form eines Oligonukleotids, vorzugsweise mit einer Länge von maximal 50 Nukleotiden. Vorzugsweise weist das Nukleinsäuremolekül die unter Punkt [12] beschriebene Ausführungsform auf.

Ein weiterer Gegenstand der Erfindung sind Vektoren, die das erfindungsgemäße Nukleinsäuremolekül oder die zuvor erwähnte Expressionskassette umfassen. Ein erfindungsgemäßer Vektor kann das mutierte Gen der Patatin-Phospholipase mit den zuvor erwähnten Eigenschaften der Nukleotidsequenz umfassen, das operativ mit einem heterologen Promotor verknüpft ist oder es kann das mutierte Gen der Patatin-Phospholipase zusammen mit seinem natürlichen Promotor umfassen.

Ein anderer Vektor kann das Wildtyp-Gen der Patatin-Phospholipase umfassen, das operativ mit einem heterologen Promotor verknüpft ist. Des Weiteren kann ein Vektor ein rekombinantes DNA-Molekül enthalten, das eine Nukleotidsequenz aufweist, die für eine doppelsträngige RNA kodiert und somit nach Expression in einer Pflanzenzelle zur Inhibierung der Expression des Patatin-Phospholipase Gens führt.

Weiterhin kann ein Vektor das zuvor beschriebene Nukleinsäuremolekül enthalten, das spezifisch an die mutierte Nukleotidsequenz der Patatin-Phospholipase bindet.

Bei dem beschriebenen Vektor kann es sich um ein Plasmid, ein Cosmid, eine Phage oder einen Expressionsvektor, einen Transformationsvektor, Shuttle-Vektor oder Klonierungsvektor handeln, er kann doppel- oder einzelsträngig, linear oder zirkulär sein oder kann einen prokaryotischen oder eukaryotischen Wirt entweder durch Integration in dessen Genom oder extrachromosomal transformieren. Bevorzugt ist das erfindungsgemäße Nukleinsäuremolekül in einem Expressionsvektor mit einer oder mehreren regulatorischen Sequenzen, welche die Transkription und optional die Expression in einer prokaryotischen oder eukaryotischen Wirtszelle erlauben, operativ verknüpft; siehe beispielsweise Sambrook et al., Molecular Cloning: A Laboratory Manual 3rd Ed. Cold Spring Harbor Laboratory Press, Cold Spring Harbor, NY, 2001 und die internationale Anmeldung WO 00/75359 auf Seite 21, Zeile 20 bis Seite 22, Zeile 32. Eine regulatorische Sequenz, vorzugsweise DNA, kann homolog oder heterolog zu der erfindungsgemäßen Nukleinsäure sein. Vorzugsweise sind diese regulatorischen Sequenzen Promotoren oder Terminatoren insbesondere ein Transkriptions-Initiations-Startpunkt, eine Ribosomen-Bindestelle, ein RNA-prozessierendes Signal, eine Transkriptions-Terminations Stelle und/oder ein Polyadenylierungssignal. Die Vektoren enthalten zusätzlich für gewöhnlich Indikator-/Reportergene oder Resistenzgene zum Nachweisen der Übertragung des gewünschten Vektors bzw. DNA-Moleküls/Nukleinsäuremoleküls und zum Selektieren der Individuen die diese enthalten, da ein direkter Nachweis über die Expression des Gens meinst eher schwierig ist. In einer bevorzugten Ausführungsform ist der Vektor ein Pflanzenvektor.

Zusätzlich zu den oben beschriebenen Vektoren, stellt die vorliegende Erfindung auch ein Verfahren bereit, das die Einbringung eines beschriebenen Vektors in eine Wirtszelle umfasst. Der Vektor kann beispielsweise durch Konjugation, Mobilisation, biolistische Transformation, Agrobakterium-vermittelte Transformation, Transfektion, Transduktion, Vakuuminfiltration oder Elektroporation eingebracht werden. Solche Verfahren ebenso wie Verfahren zur Präparation von beschriebenen Vektoren sind dem Fachmann geläufig (Sambrook *et al.* 2001).

In einem weiteren Aspekt betrifft die vorliegende Erfindung Wirtszellen, die die beschriebenen Vektoren, Nukleinsäuremoleküle oder Expressionskassetten enthalten. Eine Wirtszelle im Sinne der Erfindung kann eine prokaryotische (z.B. bakteriell) oder eukaryotische Zelle (z.B. eine pflanzliche Zelle oder eine Hefezelle) sein. Vorzugsweise ist die Wirtszelle ein Agrobakterium wie *Agrobacterium tumefaciens* oder *Agrobacterium rhizogenes* oder eine Pflanzenzelle, welche die erfindungsgemäße Nukleinsäure, den Vektor oder die beschriebene Expressionskassette umfasst. Dem Fachmann sind sowohl zahlreiche Methoden wie Konjugation oder Elektroporation bekannt, mit denen er die erfindungsgemäße Nukleinsäure, den Vektor der vorliegenden Erfindung oder die Expressionskassette in ein Agrobakterium einbringen kann, als auch Methoden wie diverse Transformationsverfahren (biolistische Transformation, Agrobakterium-vermittelte Transformation), mit denen er die erfindungsgemäße Nukleinsäure, den Vektor oder die Expressionskassette der vorliegenden Erfindung in eine Pflanzenzelle einbringen kann (Sambrook *et al.* 2001).

Weiter bevorzugt betrifft die vorliegende Erfindung eine transgene Pflanzenzelle, die das erfindungsgemäße Nukleinsäuremolekül als Transgen, den Vektor der vorliegenden Erfindung, oder die Expressionskassette umfasst sowie eine transgene Pflanze oder einen Teil davon, welche die transgene Pflanzenzelle umfasst. Eine solche transgene Pflanzenzelle oder Pflanze ist beispielsweise eine Pflanzenzelle oder Pflanze, welche mit dem erfindungsgemäßen Nukleinsäuremolekül, mit dem Vektor der vorliegenden Erfindung, oder der Expressionskassette vorzugsweise stabil, transformiert ist. Eine transgene Pflanze der vorliegenden Erfindung ist vorzugsweise zur Verwendung als Haploideninduktor geeignet. In einer bevorzugten Ausgestaltung der transgenen Pflanzenzelle ist das Nukleinsäuremolekül mit einer oder mehreren regulatorischen Sequenzen, welche die Transkription und optional die Expression in der Pflanzenzelle erlauben, operativ verknüpft. Eine regulatorische Sequenz, vorzugsweise DNA, kann homolog oder heterolog zu der erfindungsgemäßen Nukleinsäure sein. Das Gesamtkonstrukt aus dem erfindungsgemäßen Nukleinsäuremolekül und der/den regulatorischen Sequenzen stellt dann das Transgen dar. Ein Teil einer Pflanze kann ein befruchteter oder unbefruchteter Samen, ein Embryo, ein Pollen, ein Gewebe, ein Organ oder eine pflanzliche Zelle sein, wobei der befruchtete oder unbefruchtete Samen, der Embryo oder der Pollen auf der transgenen Pflanze erzeugt werden und in deren Genom die erfindungsgemäße Nukleinsäure als Transgen, der Vektor oder die Expressionskassette integriert ist. Ebenso schließt die vorliegende Erfindung auch einen Nachkommen der transgenen Pflanze ein, in dessen Genom die erfindungsgemäße Nukleinsäure als Transgen, der Vektor oder die Expressionskassette integriert ist und der zur Verwendung als Haploideninduktor geeignet ist.

In einem weiteren Aspekt betrifft die vorliegende Erfindung ein Verfahren zur Herstellung einer Pflanze, welche zur Verwendung als Haploideninduktor geeignet ist. Das Verfahren kann die folgenden Schritte umfassen:
(a) Mutagenisieren von Pflanzenzellen und anschließendem Regenerieren von Pflanzen aus den mutagenisierten Pflanzenzellen oder Mutagenisieren von Pflanzen; und
(b) Identifizieren einer Pflanze aus (a), die eine oder mehrere Mutationen in einer endogenen DNA-Sequenz kodierend die oben beschriebene Patatin-Phospholipase aufweist, die zu einem oder mehreren Aminosäureaustauschen oder Erzeugen eines Stopp-Codons führen, vorzugweise zu einem oder mehreren der beschriebenen Aminosäureaustauschen S291L, R59Q, V162I und Q372Stopp gemäß der Aminosäuresequenz SEQ ID Nr. 3 führen oder die zu einem oder mehreren Aminosäureaustauschen in Sequenzen von pflanzlichen Patatin-Phospholipasen führen, die den Aminosäureaustauschen S291L, R59Q, V162I und Q372Stopp gemäß der Aminosäuresequenz SEQ ID Nr. 3 entsprechen und die in der Lage ist, den Erhalt haploider Nachkommen mit einer erhöhten Rate im Vergleich mit einer nicht-mutagenisierten Pflanze zu induzieren. Dabei verursacht mindestens eine Mutation, dass in der identifizierten Pflanze die Eigenschaft eines Haploideninduktors vermittelt wird oder die Induktionsleistung eines Haploideninduktors erhöht wird.

Zusätzlich ergeben sich aus weiteren Untersuchungen erste Hinweise, dass weitere potentielle Mutationsorte existieren, die geeignet sind den Pflanzen die Eigenschaft der Haploideninduktion zu vermitteln oder deren Induktionsleistung zu erhöhen. Diese Mutationen führen dazu, dass an der Position 75 Asparaginsäure (D), an der Position 79 Glycin (G), und/oder an der Position 203 Prolin (P) der Aminosäuresequenz gemäß SEQ ID Nr.: 3 durch eine andere Aminosäure ausgetauscht wird, vorzugsweise durch Asparagin (N) an Position 75, Arginin (R) an Position 79 und/oder Leucin (L) an Position 203 bzw. dass in pflanzlichen Patatin-Phospholipasen Aminosäureaustausche stattfinden, die diesen Aminosäureaustauschen entsprechen.

Die endogene DNA-Sequenz aus Schritt (b) des vorstehenden Verfahrens kann grundsätzlich für jedwede pflanzliche Patatin-Phospholipase kodieren. Vorzugsweise kodiert die endogene DNA-Sequenz für eine Patatin-Phospholipase aus Sorghumhirse (SEQ ID Nr.: 3), Sonnenblume (SEQ ID Nr.: 18), Gerste (SEQ ID Nr.: 21), oder Zuckerrübe, wobei Zuckerrübe zwei putative Patatin-Phospholipasen aufweist (SEQ ID Nr.: 24 und 27). Die in dem Schritt (b) genannten pflanzlichen Patatin-Phospholipasen sind vorzugsweise solche aus Sonnenblume (SEQ ID Nr.: 18), Gerste (SEQ ID Nr.: 21) oder Zuckerrübe, wobei Zuckerrübe zwei putative Patatin-Phospholipasen aufweist (SEQ ID Nr.: 24 und 27).

Dem Fachmann ist bekannt, wie eine Mutation im Sinne der Erfindung durch den Prozess einer Mutagenisierung im Schritt (a) des Verfahrens zur Herstellung einer Pflanze, welche zur Verwendung als Haploideninduktor geeignet ist, erreicht werden kann. Die Mutagenisierung schließt hierbei sowohl die konventionelle Mutagenese als auch die ortspezifische Mutagenese beziehungsweise das "Genome Editing" ein. Bei der konventionellen Mutagenese wird die Modifikation auf DNA-Ebene nicht gezielt herbeigeführt. Die Pflanzenzelle oder die Pflanze wird mutagenen Bedingungen wie z.B. TILLING durch UV-Licht-Bestrahlung oder den Einsatz chemischer Stoffe ausgesetzt (Till et al., BMC Plant Biology 4 (2004), 12). Eine weitere Methode der Zufallsmutagenese ist die Mutagenese mit Hilfe eines Transposons. Die ortspezifische Mutagenese ermöglicht die Einbringung von Modifikation auf DNA-Ebene zielgerichtet an vordefinierten Stellen der DNA. Hierfür können beispielsweise, TALENS (WO 2010/079430, WO 2011/072246), Meganukleasen (Silva et al., Current Gene Therapy 11 (2011), 11), Homing-Endonukleasen (Chevalier, Molecular Cell 10 (2002), 895-905), Zinkfingernukleasen (Lloyd et al., Proceedings of the National Academy of Sciences of the United States of America 102 (2005), 2232-237 oder ein CRISPR/Cas-System (Gaj et al., Trends in Biotechnology 31 (2013), 397-405) verwendet werden.

Das Identifizieren einer Pflanze im Schritt (b) kann beispielsweise mit Hilfe von molekularen Markern oder Sonden erfolgen. DNA-Sonden sind beispielsweise Primer oder Primerpaare, welche in einer PCR-Reaktion eingesetzt werden können. Beispielsweise können Tillingmutanten durch Sequenzierung des Zielgens in einer Tilling-Population oder weitere Methoden, die Fehlpaarungen in der DNA nachweisen, wie z.B. Schmelzpunktanalysen oder Einsatz fehlpaarungsspezifischer Nukleasen nachgewiesen beziehungsweise identifiziert werden. Vorliegende Erfindung schließt hierfür einsetzbare Primer/Primerpaare ebenfalls mit ein, wie z.B. Primer für die Patatin-Phospholipase.

Ferner können Mutanten, erzeugt mittels Transposons, durch Einsatz von Transposon spezifischen Primern und Zielgen-spezifischem Primern in der PCR über die gesamte Population und anschießende Sequenzierung von PCR-Produkten nachgewiesen werden. Auch solche Primer sind von der vorliegenden Erfindung mit erfasst. Dem Fachmann sind aus dem Stand der Technik weitere Mittel und Verfahren bekannt, welche er zum Identifizieren einer Pflanze im Schritt (b) verwenden kann. Die vorliegende Erfindung betrifft auch molekulare Marker, welche die Anwesenheit oder Abwesenheit einer Mutation in der endogenen DNA-Sequenz oder in einer regulatorischen Sequenz der endogenen DNA-Sequenz nachweisen. Solche Marker basieren beispielsweise auf einem SNP und sind spezifisch für die Mutation (Beispiele: KASPar oder TaqMan Marker).

Das Identifizieren einer Pflanze im Schritt (b) kann auch durch Testen der Induktionsleistung wie in dem anhängigen Beispiel 1 beschrieben durchgeführt werden. Somit betrifft die vorliegende Erfindung auch ein Verfahren zur Identifizierung einer erfindungsgemäßen Pflanze durch Nachweis der Mutation in dem Patatin-Phospholipase Gen bzw. durch Nachweis eines Markerallels, das mit der Mutation gekoppelt ist, vorzugsweise unter Verwendung von oben beschriebenen molekulare Marker.

Ein Beispiel für eine Pflanze, die mittels solch einem Verfahren hergestellt und identifiziert wurde, ist die erfindungsgemäße Sorghumhirsepflanze.

Die vorliegende Erfindung betrifft weiterhin auch eine Pflanze, welche mit dem vorstehenden Verfahren herstellbar ist oder hergestellt ist, oder ein Teil dieser Pflanze, wobei ein Teil einer Pflanze ein befruchteter oder unbefruchteter Samen, ein Embryo, ein Pollen, ein Gewebe, ein Organ oder eine pflanzliche Zelle sein kann, wobei der befruchtete oder unbefruchtete Samen, der Embryo oder der Pollen auf der transgenen Pflanze erzeugt werden und in deren Genom die mindestens eine Mutation vorhanden ist. Ebenso schließt die vorliegende Erfindung auch ein Nachkomme der Pflanze ein, welcher die mindestens eine Mutation aufweist und zur Verwendung als Haploideninduktor geeignet ist. Prinzipiell kann das Verfahren auf jedwede Pflanze enthaltend eine Patatin-Phospholipase angewandt werden und dieser somit die Eigenschaft der Haploideninduktion verliehen werden. Vorzugsweise handelt es sich bei dieser Pflanze um Sorghumhirse, Sonnenblume, Gerste, Zuckerrübe, Roggen, Weizen oder Kartoffel.

In einem anderen Aspekt betrifft die vorliegende Erfindung auch ein Verfahren zur Herstellung einer transgenen Pflanze, welche zur Verwendung als Haploideninduktor geeignet ist. Das Verfahren kann die folgenden Schritte umfassen:
(a) Einbringen des erfindungsgemäßen Nukleinsäuremoleküls, das die oben beschriebene Patatin-Phospholipasen kodiert und darin eine oder mehrere Mutationen aufweist, die zu einem oder mehreren der beschriebenen Aminosäureaustauschen oder zum Erzeugen eines Stopp-Codons führen, vorzugweise zu einem oder mehreren Aminosäureaustauschen ausgewählt aus S291L, R59Q, V162I und Q372Stopp gemäß der Aminosäuresequenz SEQ ID Nr. 3 führen, Einbringen eines Nukleinsäuremoleküls, das eine oder mehrere Mutationen aufweist, die zu einem oder mehreren Aminosäureaustauschen in Sequenzen von pflanzlichen Patatin-Phospholipasen führen, die den Aminosäureaustauschen S291L, R59Q, V162I und Q372Stopp gemäß der Aminosäuresequenz SEQ ID Nr. 3 entsprechen, Einbringen der zuvor beschriebenen Expressionskassette, die entweder ein Nukleinsäuremolekül kodierend die erfindungsgemäße modfizierte Patatin-Phospholipase umfasst und operativ verknüpft mit einem Promotor ist, oder die einen Promotor und ggf. einen Terminator umfasst der operativ mit einem Nukleinsäuremolekül verknüpft ist, das eine dsRNA kodiert, die mindestens 19 Nukleotide der Nukleotidsequenz gemäß SEQ ID Nr.: 1 oder 2 oder einer Nukleotidsequenz die mindestens 80% Identität zu der Nukleotidsequenz gemäß SEQ ID Nr.: 1 oder 2 aufweist, umfasst oder Einbringen des erfindungsgemäßen Vektors, in eine Pflanzenzelle; und
(b) Regenerieren transgener Pflanzen aus den Pflanzenzellen aus (a).

Das Verfahren zur Herstellung einer transgenen Pflanze, welche zur Verwendung als Haploideninduktor geeignet ist, schließt auch die Bereitstellung von zwei oder mehr der oben beschriebenen Nukleinsäure, wahlweise auch unterschiedliche Ausgestaltungen der erfindungsgemäßen Nukleinsäure und optional in einem oder mehreren Vektoren, und das Transformieren von Pflanzenzellen durch Einbringen der zwei oder mehr Nukleinsäuren ein. Alternativ oder ergänzend können neben der erfindungsgemäßen Nukleinsäure auch eine oder mehrere weitere Nukleinsäuren, welche bekanntermaßen einsetzbar sind zur Erzeugung eines Haploideninduktors (z.B. manipuliertes *cenh3-Gen* (Ravi und Chan, Nature 464 (2010), 615-618; EP 2 989 889 A1; EP 3 037 540 A1; WO 2016/138021 A1) bereitgestellt und transformiert oder züchterisch eingebracht werden.

Das Nukleinsäuremolekül aus Schritt (a) des Verfahrens zur Herstellung einer Pflanze, welche zur Verwendung als Haploideninduktor geeignet ist kann für jedwede pflanzliche Patatin-Phospholipase kodieren. Vorzugsweise kodiert die endogene DNA-Sequenz für eine Patatin-Phospholipase aus Sorghumhirse (SEQ ID Nr.: 3), Sonnenblume (SEQ ID Nr.: 18), Gerste (SEQ ID Nr.: 21), oder Zuckerrübe, wobei Zuckerrübe zwei putative Patatin-Phospholipasen aufweist (SEQ ID Nr.: 24 und 27). Die in demselben Schritt (b) genannten pflanzlichen Patatin-Phospholipasen sind vorzugsweise solche aus Sonnenblume (SEQ ID Nr.: 18), Gerste (SEQ ID Nr.: 21) oder Zuckerrübe, wobei Zuckerrübe zwei putative Patatin-Phospholipasen aufweist (SEQ ID Nr.: 24 und 27).

Das Einbringen des Nukleinsäuremoleküls oder des Vektors aus (a) geschieht mittels Transformation, vorzugsweise mittels stabiler Transformation von Pflanzenzellen. Der Vektor kann beispielsweise durch Konjugation, Mobilisation, biolistische Transformation, Agrobakterium-vermittelte Transformation, Transfektion, Transduktion, Vakuuminfiltration oder Elektroporation eingebracht werden. Solche Verfahren sind dem Fachmann geläufig (Sambrook *et al.* 2001). Ein Nukleinsäuremolekül kann zudem auch über homologe Rekombination beispielsweise mittels CRISPR/Cas bzw. CRISPR/Cpf1 und Repairtemplate in das pflanzliche Genom eingebracht werden.

Die vorliegende Erfindung betrifft weiterhin auch eine transgene Pflanze, welche mit diesem Verfahren herstellbar ist oder hergestellt ist, oder ein Teil dieser Pflanze, wobei ein Teil einer Pflanze ein befruchteter oder unbefruchteter Samen, ein Embryo, ein Pollen, ein Gewebe, ein Organ oder eine pflanzliche Zelle sein kann, wobei der befruchtete oder unbefruchtete Samen, der Embryo oder der Pollen auf der transgenen Pflanze erzeugt werden und in deren Genom die eingebrachte Nukleinsäure als Transgen oder der Vektor integriert ist. Ebenso schließt die vorliegende Erfindung auch einen Nachkomme der transgenen Pflanze ein, welcher die eingebrachte Nukleinsäure als Transgen aufweist und zur Verwendung als Haploideninduktor geeignet ist. Prinzipiell kann das Verfahren auf jedwede Pflanze enthaltend eine Patatin-Phospholipase angewandt werden und dieser somit die Eigenschaft der Haploideninduktion verliehen werden. Vorzugsweise handelt es sich bei dieser Pflanze um Sorghumhirse, Sonnenblume, Gerste, Zuckerrübe, Roggen, Weizen oder Kartoffel.

In einem weiteren Aspekt betrifft die vorliegende Erfindung ein Verfahren zur Herstellung einer haploiden Pflanze, welches die folgenden Schritte umfasst:
(a) Kreuzen einer nicht-transgenen oder transgenen Induktor-Pflanze der vorliegenden Erfindung, welche zur Verwendung als Haploideninduktor geeignet ist, mit einer Pflanze derselben Gattung, vorzugsweise derselben Spezies,
(b) Selektieren eines befruchtete haploiden Samens oder Embryos, und
(c) Erzeugen einer haploiden Pflanze aus dem Samen oder Embryo aus (b).

Bevorzugt wird die Pflanze, welche zur Verwendung als Haploideninduktor geeignet ist, als Pollenelter eingesetzt und mit einem Saatelter derselben Gattung, vorzugsweise derselben Spezies gekreuzt. Es kann auch die Pflanze, welche zur Verwendung als Haploideninduktor geeignet ist, als Saatelter eingesetzt werden und mit einem Pollenelter derselben Gattung, vorzugsweise derselben Spezies gekreuzt werden. Beide Kreuzungspartner in Schritt (a), also Saatelter und Pollenelter, können auch dasselbe Individuum sein. Dann stellt der Kreuzungsschritt eine Selbstung dar.

Das Selektieren des haploiden befruchteten Samens oder Embryos kann einen Schritt des Nachweises der Haploidie und des Separierens des haploiden befruchteten Samens oder Embryos von polyploiden befruchteten Samen oder Embryos umfassen. Der Nachweis der Haploidie eines befruchteten Samens oder Embryos kann phänotypisch oder genotypisch erfolgen, indem beispielsweise der Induktor mit einem embryo-spezifischen dominanten Marker versehen wird, der in allen diploiden Nachkommen, aber nicht in den induzierten haploiden Nachkommen sichtbar wird. Des Weiteren kann der Ploidie-Status über Durchflusszytometrie bestimmt werden. Außerdem gibt ein vollständig homozygotes Muster von molekularen Markern einen Hinweis auf haploide Pflanzen. Das Separieren kann automatisiert beispielsweise auf Basis von Daten des Nachweises der Haploidie erfolgen.

Die vorliegende Erfindung betrifft weiterhin auch einen haploiden befruchteten Samen oder Embryo, welcher beim Kreuzen in Schritt (a) des Verfahrens zur Herstellung einer haploiden Pflanze entsteht sowie eine haploide Pflanze, welche mit diesem Verfahren herstellbar ist oder hergestellt ist, oder ein Teil dieser Pflanze, wobei ein Teil einer Pflanze ein Samen, ein Embryo, ein Gewebe, ein Organ oder eine pflanzliche Zelle sein kann. Ebenso schließt die vorliegende Erfindung auch ein Nachkomme der Pflanze ein.

Weiterhin erfasst die vorliegende Erfindung auch eine doppelhaploide (diploide) Pflanze oder einen Teil davon, wobei die doppelhaploide (diploide) Pflanze oder der Teil davon durch Chromosomendoppelung der haploiden Pflanze oder des Teils davon erzeugt wurde. Diese doppelhaploiden (diploiden) Pflanzen können durch folgendes Verfahren hergestellt werden:
(a) Herstellen einer haploiden Pflanze mittels dem erfindungsgemäßen Verfahrens;
(b) Verdoppeln des haploiden Chromosomensatzes in mindestens einer Zelle der haploiden Pflanze, und
(c) Regenerieren der diploiden Pflanze aus der Zelle aus (b).

In dem erfindungsgemäßen Verfahren zur Erzeugung doppelhaploider Pflanzen werden die haploiden Pflanzen aus (a) mit dem Zellteilungshemmstoff Colchizin behandelt. Das führt zur Verdoppelung der Chromosomen. Dem Fachmann ist dieses Verfahren bekannt und es ist beispielsweise in Seguí-Simarro und Nuez, Cytogenetic and Genome Research 120 (2008), 358-369 beschrieben.

Allgemeine Verfahren zur Herstellung von haploiden sowie doppelhaploiden Pflanzen sind dem Fachmann bekannt, beispielsweise aus Dwivedi et al., Biotechnol. Adv. 33 (2015), 812-29 und Murovec & Bohanec, Biochemistry, Genetics and Molecular Biology "Plant Breeding" (2012), eds. Abdurakhmonov, Kapitel 5 und können auf die vorliegende Erfindung angewandt werden.

Eine weitere Ausführungsform der vorliegenden Erfindung umfasst ein Verfahren zur Herstellung von hybriden Pflanzen mit den folgenden Schritten:
(a) Kreuzen der erfindungsgemäßen doppelhaploiden (diploiden) Pflanze mit einer zweiten Pflanze derselben Gattung, vorzugsweise derselben Spezies,
(b) Selektieren der hybriden Pflanzen hinsichtlich des gewünschten Merkmals.

Die doppelhaploiden Pflanzen sind reinerbig und durch Kreuzen zweier reinerbiger Pflanzen tritt der bekannte Heterosis-Effekt ein, der zu einer besonders ausgeprägte Leistungsfähigkeit von Hybridpflanzen führt. Es sind demensprechend auch die hybride Pflanzen, die durch solch ein Verfahren erhalten wird Gegenstand der vorliegenden Erfindung.

In einem weiteren Aspekt betrifft die vorliegende Erfindung die Verwendung der erfindungsgemäßen Nukleinsäure, des erfindungsgemäßen Vektors oder der zuvor beschriebenen Expressionskassette in einer Pflanze zur Vermittlung der Eigenschaft eines Haploideninduktors oder zur Erhöhung der Induktionsleistung eines Haploideninduktors oder die Verwendung der erfindungsgemäßen Nukleinsäure, des erfindungsgemäßen Vektors oder der zuvor beschriebenen Expressionskassette zur Herstellung einer Pflanze oder einer transgenen Pflanze, welche zur Verwendung als Haploideninduktor geeignet ist. Weiterhin schließt die vorliegende Erfindung auch die Verwendung einer oben beschriebenen erfindungsgemäßen Pflanze, welche zur Verwendung als Haploideninduktor geeignet ist, zur Herstellung eines haploiden befruchteten Samens oder Embryos oder einer haploiden Pflanze ein. Vorstehende Erläuterungen zu Gegenstanden und Verfahren der vorliegenden Erfindung sind auch für die genannten Verwendungen anwendbar.

In erfindungsgemäßen Experimenten mit Sorghumhirsepflanzen konnte ein Gen aufgefunden werden, im Speziellen das Gen der Patatin-Phospholipase mit einer oder mehreren Mutationen die geeignet waren, der Pflanze die Eigenschaft eines Haploidie-Induktors zu vermitteln sowie eine Induktionsleistung von mindestens 0,4% bis zu 1,5% oder mehr, sodass erstmal ein effizientes und damit wirtschaftlich anwendbares System zur Herstellung haploider und doppelhaploider Sorghumhirsepflanzen für die Hybridzüchtung bereitgestellt werden konnte. Durch das erfindungsgemäße Verfahren zur Herstellung solcher Haploideninduktoren sowie der Identifizierung von Patatin-Phospholipasen in weiteren Kulturpflanzen, kann dieses System ebenso auf diese übertragen werden.

Die folgenden Beispiele erläutern die Erfindung, ohne jedoch den Gegenstand der Erfindung einzuschränken. Sofern nicht anders angegeben, wurden molekularbiologischen Standardmethoden verwendet, siehe beispielsweise (Sambrook et al., Molecular Cloning: A Laboratory Manual 3rd Ed. Cold Spring Harbor Laboratory Press, Cold Spring Harbor, NY, 2001), Fritsch *et al.*, Cold Spring Harbor Laboratory Press: 1989; Mayer et al., Immunochemical Methods In Cell And Molecular Biology, eds., Academic Press, London, 1987) und Weir et al., Handbook Of Experimental Immunology, Volumes I-IV, Blackwell, eds., 1986).

### BEISPIELE

### 1. Identifizierung der Patatin-Phospholipase als Target zur Vermittlung der Eigenschaft der Haploideninduktion in Sorghumhirse

In einer TILLING Population wurde eine Sorghumhirsepflanze gefunden, welche in der Lage war, Haploidie mit einer Effizienz von etwa 1% zu induzieren. Auf der Suche nach der genetischen Grundlage, welche diese Eigenschaft in der identifizierten Sorghumhirsepflanze vermittelt, fokusierte man sich auf verschiedenen Gene als potentielle Targets. Die Auswahl der Gene erfolgte basierend darauf, dass sie vorzugsweise in Fortpflanzungsorganen der Pflanze exprimiert werden und in irgendeiner Art und Weise eine Rolle in der Befruchtung spielen, denn durch fehlerhafte oder unvollständige Befruchtung, beispielsweise durch einen ausbleibenden oder fehlerhaften Transport der generativen Zellen zu den weiblichen Samenanlagen oder durch Einwirkung auf den Energiestoffwechsel des Pollens, kann es zur Chromosomeneliminierung kommen, was zu einem haploiden Chromosomensatz führt.

Nach ersten vorläufigen Untersuchungen im Rahmen der vorliegenden Erfindung wurde dann ein mutiertes Gen gefunden, das mittels der bioinformatischen Methoden BLASTP und Syntenie Studie (Altschul et al., Nucleid Acid Res. 25 (1997), 3389-3402) als Patatin-Phospholipase in Sorghum identifiziert werden konnte, dessen Nukleotid- und Aminosäuresequenz in SEQ ID Nr.: 1 bzw. SEQ ID Nr.: 3 dargestellt sind, und aufgrund von RNA-Sequenzierung (RNASeq) als eine pollenspezifisch exprimierte Patatin-Phospholipase identifiziert wurde. Das mutierte Gen wies eine Punktmutation in der Nukleotidsequenz der Patatin-Phospholipase auf (vgl. SEQ ID Nr.: 10), welche einen Aminosäureaustausch von Serin zu Leucin an Position 291 bewirkte (vgl. SEQ ID Nr.: 12). Die Punktmutation in der Nukleotidsequenz der Patatin-Phospholipase wurde unter Verwendung der PCR-Methode mit den Primern gemäß SEQ ID Nr.: 44 und 45 identifiziert.

Zur Verifikation des mutierten Gens als Ursache für die beobachtete Haploideninduktion wurde der Locus enthaltend das Gen in den genetischen Hintergrund eines Nicht-Induktors durch züchterische und biotechnologische Methoden eingebracht. Hierdurch gelang es, in den Nicht-Induktor die Eigenschaft eines Haploideninduktors mit einer Effizienz von durchschnittlich 1,5%, wenn die Mutation homozygot vorlag, und 1,2%, wenn die Mutation heterozygot vorlag, einzubringen. Während der Verifikation konnte das mutierte Gen mittels PCR mit den Primern gemäß SEQ ID Nr.: 36 bis 38 nachverfolgt werden. Die Induktionsleistung eines potentiellen Induktors wurde durch Bestäubung von Sorghumhirsepflanzen mit den mutierten Sorghumhirsepflanzen (enthaltend das mutierte Gen) durchgeführt. Dabei unterschieden sich die verwendeten Wildtyp-Sorghumhirsepflanzen genetisch über mehrere Marker von der potentiellen Induktorlinie. Diese Marker wurden dazu verwendet, um homozygote Pflanzen zu identifizieren, welche anschließend mittels Durchflusszytometrie auf Haploidie geprüft wurden.

### 2. Erstellung von weiteren in vivo Sorghumhirse-Haploideninduktoren

Nachdem mit dem zuvor beschriebenen Versuch verifiziert wurde, dass die Patatin-Phospholipase in der Sorghumhirse ein geeignetes Target ist, um in der Sorghumhirse die Eigenschaft der Haploideninduktion zu vermitteln, wurden weitere Mutationen in das endogene Wildtyp-Gen eingebracht. Es wurde eine Mutation eingebracht, die zu einem Austausch der Aminosäure Glutamin (Q) gegen ein Stopp-Codon an der Position 372 führte. Dies führte zu einer Verkürzung des Patatin-Phospholipase Proteins und verlieh der Sorghumhirsepflanze enthaltend diese Mutation die Eigenschaft zur Haploideninduktion mit einer Effizienz von durchschnittlich zwischen 1% und 3%. Wie in Fig. 2 dargestellt, liegen die zuvor beschriebenen Mutationen S291L und Q372Stopp außerhalb der funktionellen Domäne der Patatin-Phospholipase. Daher wurden in einem nächsten Versuch Mutationen in das Gen der Patatin-Phospholipase eingebracht, die einerseits einen R59Q- und andererseits einen V162I-Aminosäureaustausch herbeiführten. Diese beiden Mutationen liegen innerhalb der funktionellen Domäne und bewirken, dass die Sorghumhirsepflanze eine Haploideninduktoreigenschaft erhält, wobei erste Versuche ergaben, dass die Induktionsrate über etwa 0,5% liegt. Ein Sequenzalignment der vier Mutanten zusammen mit dem Wildtyp-Protein ist in Fig. 1 dargestellt.

Es ist davon auszugehen, dass zusätzliche oder andere Mutationen bzw. die Kombination von mehreren Mutationen in der Patatin-Phospholipase zu einer erhöhten bzw. weiter erhöhten Induktionsrate führen, sodass weitere Versuche zum Screening von TILLING-Populationen durchgeführt werden können, um eine Pflanze mit erhöhter Induktionsleistung zu identifizieren.

Des Weiteren könnten auch in den verschiedenen Sorghum Arten und davon abgeleiteten Sorten die Mutationen nicht nur mittels TILLING oder anderen Mutageneseverfahren in die Patatin-Phospholipase eingebracht werden sondern es können beispielsweise weitere Haploideninduktoren durch transgene Expression der Patatin-Phospholipase erzeugt werden. Hierfür sind die entsprechenden Gene inklusive ihrer Promotoren aus den Sorghumhirse-Induktorlinien mit den Mutationen S291L, R59Q, V162I und/oder Q372Stopp in der Patatin-Phospholipase gemäß SEQ ID Nr.: 3 zu klonieren. Diese Gene können in einen geeigneten Transformationsvektor kloniert und in die gewünschte Pflanze transformiert werden.

Die Induktionseigenschaft der oben beschriebenen Sorghumhirsepflanzen lässt sich auf die dargestellten Mutationen in der Patatin-Phospholipase zurückführen, was in der Regel zu einer Änderung der biologischen Aktivität dieser führt. Die biologische Aktivität kann jedoch nicht nur durch das Einbringen der beschriebenen Mutationen sondern auch durch zahlreiche weitere gentechnische Verfahren verändert werden.

Beispielsweise kann das Wildtyp-Gen der Patatin-Phospholipase zusammen mit einem geeigneten Promotor in einen Transformationsvektor kloniert und in die gewünschte Pflanze transformiert werden, sodass es zu einer Überexpression der Patatin-Phospholipase kommt. Weiterhin könnte die Patatin-Phospholipase über RNAi in ihrer Aktivität reduziert werden. Hierfür sind beispielsweise Hairpin-Konstrukte herzustellen, welche dann inklusive eines geeigneten Promotors und Terminators, welche eine Transkription des Hairpin-Konstruktes vor oder zum Zeitpunkt der Pollenausbildung erlauben, in einen geeigneten Transformationsvektor kloniert und in die gewünschte Pflanze transformiert werden. Alternativ könnte nach Knockout-Mutanten gesucht werden, die die Aktivität weiter verringern.

### 3. Erstellung von neuen in vivo Haploideninduktoren

Erste Untersuchungen im Rahmen der vorliegenden Erfindung geben begründeten Anlass zu stipulieren, dass in einigen weiteren Kulturpflanzen die Eigenschaft der Haploideninduktion bzw. eine Verbesserung dieser Eigenschaft über Modifikationen der Patatin-Phospholipase vermittelt werden kann, insbesondere über entsprechende, vorstehend für die Sorghumhirsepflanze beschriebenen Mutationen bzw. gemäß vorstehend beschriebenen erfindungsgemäßen Verfahren. Dabei handelt es sich um Target-Gene, die putative Patatin-Phospholipasen kodieren mit einer der folgenden Aminosäuresequenzen in Sonnenblume (SEQ ID Nr.: 18), Gerste (SEQ ID Nr.: 21) und in Zuckerrübe, wobei Zuckerrübe zwei potentielle Phospholipasen aufweist (SEQ ID Nr.: 24 und 27). Ein Sequenzalignment der Proteinsequenzen aus Sorghum, Sonnenblume, Gerste und Zuckerrübe ist in Fig. 2 dargestellt, in dem zusätzlich die putative funktionelle Domäne gekennzeichnet ist (**fett**). Die Expression dieser Gene in Pollen kann bspw. über RNASeq von Pollen durchgeführt werden.

Gemäß dem vorstehend beschriebenen erfindungsgemäßen Verfahren können nun gezielt Modifikationen betreffend die Patatin-Phospholipase in Sonnenblume, Gerste (und andere Getreide wie Weizen, Roggen und Hafer) und Zuckerrübe eingebracht werden. Beispielsweise können mithilfe des Sequenzalignments in Fig. 2 die Aminosäuren identifiziert werden, die denen an den Positionen 291, 59, 162 und 372 gemäß SEQ ID Nr.: 3 entsprechen und ausgetauscht werden. Dementsprechend sind auch Sonnenblumen-, Gerste- bzw. Getreide- und Zuckerrübenpflanzen Gegenstand der vorliegenden Erfindung, welche in der Lage sind Haploidie zu induzieren, und dadurch gekennzeichnet sind, dass sie eine oder mehrere Modifikationen aufweisen, die eine endogene vorzugsweise pollenspezifisch exprimierte Patatin-Phospholipase betreffen entsprechend eine der der in vorstehenden Items [] oder Ansprüchen insbesondere für Sorghumhirsepflanze gekennzeichneten Ausführungsformen und/oder erhältlich durch das vorstehend beschriebene bzw. in den Ansprüchen gekennzeichnete erfindungsgemäße Verfahren. Es gibt bereits erste Indikationen, dass auch Aminosäureaustausche an Position 75 (Asparaginsäure (D) ist durch Asparagin (N) ersetzt (D75N)), an Position 79 (Glycin (G) ist durch Arginin (R) ersetzt (G79R)) und/oder an Position 203 (Prolin (P) ist durch Leucin (L) ersetzt (P203L)) der Aminosäuresequenz gemäß SEQ ID Nr. 3 sowohl in Sorghum als auch in anderen Kulturarten eine Induktion von Haploiden vermitteln können,

## Patentansprüche

1. Sorghumhirsepflanze, welche in der Lage ist Haploidie zu induzieren, **dadurch gekennzeichnet, dass** die Pflanze eine oder mehrere Modifikationen aufweist, die ein endogenes Gen kodierend eine Patatin-Phospholipase betreffen.

2. Pflanze nach Anspruch 1, **dadurch gekennzeichnet, dass** die Patatin-Phospholipase durch die Nukleotidsequenz gemäß SEQ ID Nr.: 1 oder 2 oder durch eine Nukleotidsequenz, die zu mindestens 80% identisch ist zu SEQ ID Nr.: 1 oder 2 kodiert wird, oder durch eine Nukleotidsequenz kodiert wird, die mit der komplementären Sequenz zu der Nukleotidsequenz gemäß SEQ ID Nr.: 1 oder 2 unter stringenten Bedingungen hybridisiert, oder die die in SEQ ID Nr.: 3 gezeigte Aminosäuresequenz oder eine homologe Aminosäuresequenz umfasst.

3. Pflanze nach Anspruch 1 oder 2, **dadurch gekennzeichnet, dass** die eine oder mehrere Modifikationen eine oder mehrere Mutationen in dem endogenen Gen kodierend die Patatin-Phospholipase, vorzugsweise Substitutionen sind, welche zu einem oder mehreren Aminosäureaustauschen oder Erzeugen eines Stopp-Codons führen, vorzugsweise wobei die eine oder mehrere Mutationen zu einem Aminosäureaustausch
a) in dem Bereich der Aminosäurepositionen 37 bis 240 gemäß SEQ ID Nr.: 3 führen, vorzugsweise wobei dieser Bereich der funktionellen Domäne der Patatin-Phospholipase entspricht; und/oder
b) in dem Bereich der Aminosäurepositionen 241 bis 385 gemäß SEQ ID Nr.: 3 führen, oder
c) zu einem Erzeugen eines Stopp-Codons in dem Bereich der Aminosäurepositionen 241 bis 385 gemäß SEQ ID Nr.: 3 führen.

4. Pflanze nach Anspruch 3, **dadurch gekennzeichnet, dass** die eine oder mehrere Mutationen zu einem Aminosäureaustausch an der Aminosäureposition 59, 162 und/oder 291 gemäß SEQ ID Nr.: 3 und/oder zu einem Stopp-Codon an der Aminosäureposition 372 gemäß SEQ ID Nr.: 3 führen.

5. Pflanze nach einem der Ansprüche 1 bis 4, **dadurch gekennzeichnet, dass** die modifizierte Patatin-Phospholipase
(i) eine Aminosäuresequenz gemäß SEQ ID Nr.: 3 oder eine homologe Aminosäuresequenz umfasst, in der mindestens ein Aminosäureaustausch vorhanden ist, wobei an der Position 59 Arginin (R), an der Position 162 Valin (V), und/oder an der Position 291 Serin (S) durch eine andere Aminosäure ausgetauscht ist, vorzugsweise durch Glutamin (Q) an Position 59, Isoleucin (I) an Position 162 und/oder Leucin (L) an Position 291;
(ii) durch eine Nukleotidsequenz kodiert wird, die die kodierende Sequenz der DNA-Sequenz gemäß SEQ ID Nr.: 1 oder einer DNA-Sequenz, die zu mindestens 80% identisch ist zu SEQ ID Nr.: 1 umfasst, in der mindestens ein Nukleotidaustausch vorhanden ist, der zu einem Aminosäureaustauch führt, wobei an den Positionen 421-423, 815-817, 1420-1422 und/oder 1663-1665 gemäß SEQ ID Nr.: lein oder mehrere Nukleotide ausgetauscht sind;
(iii) eine Aminosäuresequenz gemäß SEQ ID Nr.: 6, 9 oder 12 umfasst; oder (iii) durch eine Nukleotidsequenz kodiert wird, die die kodierende Sequenz der DNA-Sequenz gemäß SEQ ID Nr.: 4, SEQ ID Nr.: 7, SEQ ID Nr.: 10 oder SEQ ID Nr.: 13 umfasst.

6. Nukleinsäuremolekül, welches die in einem der Ansprüche 3 bis 5 gekennzeichnete mutierte Patatin-Phospholipase kodiert.

7. Nukleinsäuremolekül nach Anspruch 6, **dadurch gekennzeichnet, dass** dessen Vorhandensein in einer Pflanze in Abwesenheit einer Wild-Typ Patatin-Phospholipase dazu führt, dass die Pflanze in der Lage ist Haploidie zu induzieren.

8. Nukleinsäuremolekül von mindestens 15, 16, 17, 18, 19 oder 20, bevorzugt mindestens 21, 22, 23, 24 oder 25, besonders bevorzugt mindestens 30, 35, 40, 45 oder 50, und insbesondere bevorzugt mindestens 100, 200, 300, 500 oder 1000 Nukleotiden Länge, das spezifisch an eine wie in Anspruch 4 und 5 definierte Nukleotidsequenz hybridisiert, und eine der Mutationen umfasst oder ein Paar von Nukleinsäuremolekülen, das geeignet ist einen Bereich, der mindestens eine der Mutationen enthält, in einer Polymerase-Kettenreaktion (PCR) zu amplifizieren, vorzugsweise in Form eines Oligonukleotids, vorzugsweise mit einer Länge von maximal 50 Nukleotiden, das vorzugsweise eine der folgenden Nukleotidsequenzen gemäß SEQ ID Nr.: 28-30, 32-34, 36-38 oder 40-42 aufweist.

9. Wirtszelle, vorzugsweise Pflanzenzelle enthaltend ein Nukleinsäuremolekül nach einem der Ansprüche 6 bis 8, einen Vektor, vorzugsweise Pflanzenvektor, umfassend ein Nukleinsäuremolekül nach einem der Ansprüche 6 bis 8 oder das Nukleinsäuremolekül nach einem der Ansprüche 6 bis 8 als Transgen, optional unter der Kontrolle eines heterologen Promotors.

10. Verfahren zum Erhalt einer Pflanze welche in der Lage ist Haploidie zu induzieren bzw. mit einer erhöhten Induktionsrate gegenüber dem Wild-Typ, umfassend die folgenden Schritte:
(a)
(i) Mutagenisieren von Pflanzenzellen und anschließendem Regenerieren von Pflanzen aus den mutagenisierten Pflanzenzellen oder Mutagenisieren von Pflanzen;
(ii) Identifizieren einer Pflanze aus (i), die eine oder mehrere Mutationen in einer endogenen DNA-Sequenz aufweist, welche der einen oder mehreren der in einem der Ansprüche 3 bis 5 gekennzeichneten Mutationen entsprechen und/oder welche dazu führen, dass an der Position 75 Asparaginsäure (D), an der Position 79 Glycin (G), und/oder an der Position 203 Prolin (P) der Aminosäuresequenz gemäß SEQ ID Nr. 3 durch eine andere Aminosäure ausgetauscht wird, vorzugsweise durch Asparagin (N) an Position 75, Arginin (R) an Position 79 und/oder Leucin (L) an Position 203 oder welche diesen entsprechen, und in der Lage ist, den Erhalt haploider Nachkommen mit einer erhöhten Rate im Vergleich mit einer nicht-mutagenisierten Pflanze zu induzieren; oder
(b)
(i) Einbringen des Nukleinsäuremoleküls, das eine oder mehrere Mutationen aufweist, welche der einen oder mehreren der in einem der Ansprüche 3 bis 5 gekennzeichneten Mutationen entsprechen und/oder welche dazu führen, dass an der Position 75 Asparaginsäure (D), an der Position 79 Glycin (G), und/oder an der Position 203 Prolin (P) der Aminosäuresequenz gemäß SEQ ID Nr. 3 durch eine andere Aminosäure ausgetauscht wird, vorzugsweise durch Asparagin (N) an Position 75, Arginin (R) an Position 79 und/oder Leucin (L) an Position 203 oder welche diesen entsprechen, in Pflanzenzellen, und
(ii) Regenerieren transgener Pflanzen aus den Pflanzenzellen aus (i), vorzugsweise wobei es sich bei der endogenen DNA-Sequenz bzw. dem Nukleinsäuremolekül um eine kodierende Nukleotidsequenz handelt welche die in SEQ ID Nr.: 3 für Sorghumhirse (Sorghum bicolor), SEQ ID Nr.: 18 für Sonnenblume (Helianthus annuus), SEQ ID Nr.: 21 für Gerste (Hordeum vulgare) oder in SEQ ID Nr.: 24 oder 27 für Beta vulgaris (z.B. Zuckerrübe) gezeigten Aminosäuresequenzen umfassen.

11. Pflanze enthaltend Pflanzenzelle nach Anspruch 9 und/oder erhältlich durch ein Verfahren nach Anspruch 10, vorzugsweise wobei es sich bei der Pflanze um Sorghumhirse, Sonnenblume, Roggen, Weizen, Kartoffel, Gerste oder Zuckerrübe handelt.

12. Organ, Pflanzenteil, Gewebe oder Zelle der Pflanze nach einem der Ansprüche 1 bis 5 oder 11 oder Samen oder Nachkommen der Pflanze nach einem der Ansprüche 1 bis 5 oder 11, wobei der Samen oder die Nachkommen die in einem der Ansprüche 3 bis 5 definierte Mutation aufweist und/oder ein Nukleinsäuremolekül nach einem der Ansprüche 6 bis 8 oder einen Vektor, vorzugsweise Pflanzenvektor, umfassend ein Nukleinsäuremolekül nach einem der Ansprüche 6 bis 8.

13. Verfahren zum Erhalt einer haploiden Pflanze, umfassend die folgenden Schritte:
(a) Kreuzen einer Pflanze gemäß einem der Ansprüche 1 bis 5 oder 11 mit einer Pflanze derselben Gattung, vorzugsweise derselben Spezies,
(b) Selektieren eines befruchteten haploiden Samens oder Embryos, und
(c) Erzeugen einer haploiden Pflanze aus dem Samen oder Embryo aus (b).

14. Haploide Pflanze, haploider befruchteter Samen oder Embryo erhältlich durch das Verfahren nach Anspruch 13.

15. Organ, Pflanzenteil, Gewebe, Zelle, Samen oder Nachkommen der Pflanze nach Anspruch 14.
